# EUROPEAN PATENT SPECIFICATION

(11) **EP 3 218 483 B1**
(45) Date of publication and mention of the grant of the patent: **14.10.2026**
(21) Application number: 15859580.1
(22) Date of filing: 16.11.2015
(51) Int. Cl.: C12N 15/113, C07H 21/04, C07H 21/00, C12N 15/67, A61K 31/7088

(54) **COMPOUNDS AND METHODS FOR THE MODULATION OF PROTEINS**
VERBINDUNGEN UND VERFAHREN ZUR MODULATION VON PROTEINEN
COMPOSÉS ET PROCÉDÉS DE MODULATION DES PROTÉINES

(30) Priority: 14.11.2014 US 201462080223 P; 27.03.2015 US 201562139626 P; 01.05.2015 US 201562156139 P; 04.05.2015 US 201562156812 P; 25.09.2015 US 201562233183 P
(43) Date of publication of application: 20.09.2017
(60) Divisional application: 26203582.7
(73) Proprietor: Ionis Pharmaceuticals, Inc., Carlsbad, CA 92010 (US)
(72) Inventor: CROOKE, Stanley T., Carlsbad, CA 92010 (US); LIANG, Xue-hai, Carlsbad, CA 92010 (US); SHEN, Wen, Carlsbad, CA 92010 (US)
(74) Representative: Carpmaels & Ransford LLP
(86) International application number: PCT/US2015/060938
(87) International publication number: WO 2016/077837

(56) References cited:
- WO-A1-2014/172698
- US-A1- 2006 166 922
- KLAUS WETHMAR: "The regulatory potential of upstream open reading frames in eukaryotic gene expression : uORF-mediated translational control", WILEY INTERDISCIPLINARY REVIEWS: RNA, vol. 5, no. 6, 1 November 2014 (2014-11-01), United Kingdom, pages 765 - 768, XP055477272, ISSN: 1757-7004, DOI: 10.1002/wrna.1245
- SARAH E CALVO ET AL: "Upstream Open Reading Frames Cause Widespread Reduction Of Protein Expression And Are Polymorphic Among Humans", vol. 106, no. 18, 5 May 2009 (2009-05-05), pages 7507 - 7512, XP003031323, ISSN: 0027-8424, Retrieved from the Internet <URL:http://www.pnas.org/content/106/18/7507> [retrieved on 20090416], DOI: 10.1073/PNAS.0810916106
- CRISTINA BARBOSA ET AL: "Gene Expression Regulation by Upstream Open Reading Frames and Human Disease", PLOS GENETICS, vol. 9, no. 8, 8 August 2013 (2013-08-08), pages e1003529, XP055477051, DOI: 10.1371/journal.pgen.1003529
- Y. ZHANG ET AL: "The 5'-Untranslated Region of Multidrug Resistance Associated Protein 2 (MRP2; ABCC2) Regulates Downstream Open Reading Frame Expression through Translational Regulation", MOLECULAR PHARMACOLOGY, vol. 77, no. 2, 1 February 2010 (2010-02-01), pages 237 - 246, XP055033896, ISSN: 0026-895X, DOI: 10.1124/mol.109.058982
- S. DVIR ET AL: "Deciphering the rules by which 5'-UTR sequences affect protein expression in yeast", PROCEEDINGS OF THE NATIONAL ACADEMY OF SCIENCES, vol. 110, no. 30, 5 July 2013 (2013-07-05), US, pages E2792 - E2801, XP055478067, ISSN: 0027-8424, DOI: 10.1073/pnas.1222534110
- D. R. MORRIS ET AL: "Upstream Open Reading Frames as Regulators of mRNA Translation", MOLECULAR AND CELLULAR BIOLOGY., vol. 20, no. 23, 1 December 2000 (2000-12-01), US, pages 8635 - 8642, XP055477266, ISSN: 0270-7306, DOI: 10.1128/MCB.20.23.8635-8642.2000
- XUE-HAI LIANG ET AL: "Translation efficiency of mRNAs is increased by antisense oligonucleotides targeting upstream open reading frames", NATURE BIOTECHNOLOGY, vol. 34, no. 8, 11 July 2016 (2016-07-11), US, pages 875 - 880, XP055483109, ISSN: 1087-0156, DOI: 10.1038/nbt.3589
- XUE-HAI LIANG ET AL: "Antisense oligonucleotides targeting translation inhibitory elements in 5' UTRs can selectively increase protein levels", NUCLEIC ACIDS RESEARCH, vol. 45, no. 16, 21 July 2017 (2017-07-21), pages 9528 - 9546, XP055477254, ISSN: 0305-1048, DOI: 10.1093/nar/gkx632
- A. G. HINNEBUSCH ET AL: "Translational control by 5'-untranslated regions of eukaryotic mRNAs", SCIENCE, vol. 352, no. 6292, 17 June 2016 (2016-06-17), US, pages 1413 - 1416, XP055478070, ISSN: 0036-8075, DOI: 10.1126/science.aad9868
- XUE-HAI LIANG ET AL: "Translation efficiency of mRNAs is increased by antisense oligonucleotides targeting upstream open reading frames", NATURE BIOTECHNOLOGY, vol. 34, no. 8, 11 July 2016 (2016-07-11), New York, pages 875 - 880, XP055483109, ISSN: 1087-0156, DOI: 10.1038/nbt.3589
- MAJDALANI ET AL.: "Regulation of RpoS by a novel small RNA: the characterization of RprA.", MED MICROBIOL, vol. 39, no. 5, March 2001 (2001-03-01), pages 1382 - 1394, XP055441133

## Description

### SEQUENCE LISTING

The present application is being filed along with a Sequence Listing in electronic format. The Sequence Listing is provided as a file entitled CORE0132WOSEQ_ST25.txt, created November 11, 2015, which is 72 Kb in size.

### BACKGROUND

Translation of a polypeptide or protein encoded by an mRNA typically begins at the start codon of the primary open reading frame (pORF) of the mRNA. Some mRNA transcripts also comprise one or more additional start codons. Such additional start codons may be upstream of the pORF start codon. Such an additional start codon that is upstream of a pORF is referred to as an upstream open reading frame (uORF) start site. The potential role of additional start sites in regulating translation of pORF protein products has been discussed previously *(see* Barbosa et al. PLOS Genetics. 9, e1003529 (2013)). Mutations that introduce or eliminate an additional start codon (a uORF start codon) in a transcript can disrupt regulation of its translation and can lead to disease *(see* Calvo et al. Proc. Natl. Acad. Sci. 106, 7507 (2009)).

Antisense technology is an effective means for modulating the expression of one or more specific gene products and can therefore prove to be uniquely useful in a number of therapeutic, diagnostic, and research applications. Chemically modified nucleosides may be incorporated into antisense compounds to enhance one or more properties, such as nuclease resistance, pharmacokinetics or affinity for a target nucleic acid. In 1998, the antisense compound, Vitravene^{®} (fomivirsen; developed by Isis Pharmaceuticals Inc., Carlsbad, CA) was the first antisense drug to achieve marketing clearance from the U.S. Food and Drug Administration (FDA), and is currently a treatment of cytomegalovirus (CMV)-induced retinitis in AIDS patients. For another example, an antisense compound targeting ApoB, KYNAMRO^{™}, has been approved by the U.S. Food and Drug Administration (FDA) as an adjunct treatment to lipid-lowering medications and diet to reduce low density lipoprotein-cholesterol (LDL-C), ApoB, total cholesterol (TC), and non-high density lipoprotein-cholesterol (non HDL-C) in patients with homozygous familial hypercholesterolemia (HoFH).

US 2006/0166922 A1 describes "Polynucleotides targeted against the extended 5'-UTR region of argininosuccinate synthase and uses thereof". Majdalani, N et al. (Molecular Microbiology (2001) 39(5), 1382-1394) describes "Regulation of RpoS by a novel small RNA: the characterization of RprA". Wethmar, K (WIREs RNA (2014) 5:765-778) describes "The regulatory potential of upstream open reading frames in eukaryotic gene expression". Dvir, S et al. (PNAS (2013), E2792-E2801) describes "Deciphering the rules by which 5'-UTR sequences affect protein expression in yeast". Morris, DR et al. (Molecular and Cellular Biology (2000) 20(23), 8635-8642) describes "Upstream Open Reading Frames as Regulators of mRNA Translation".

### SUMMARY

The invention is as defined by the appended claims. References to methods are references to *in vitro* methods, and references to methods of treatment in the subsequent paragraphs of this description are to be interpreted as references to the compounds and pharmaceutical compositions of the present invention for use in a method of treatment of the human (or animal) body by therapy, as set out in the claims.

The invention provides a method of increasing the amount of a target protein in an *in vitro* cell, comprising contacting the cell with an antisense compound comprising a modified oligonucleotide, wherein the target protein is encoded by a target transcript comprising at least one translation suppression element, wherein the translation suppression element comprises a uORF, wherein the uORF comprises an AUG codon, and thereby increasing expression of the target protein in the cell, wherein the modified oligonucleotide: is complementary to the target transcript at the uORF start site region; has a nucleobase sequence that is 100% complementary to the target transcript; consists of 15 to 22 linked nucleosides; and comprises at least 15 modified nucleosides, each modified nucleoside independently comprising a modified sugar moiety, wherein the antisense compound does not recruit RNase H.

The invention also provides an antisense compound comprising a modified oligonucleotide for use in a method of treating a disease or condition associated with a deficiency of a target protein, wherein in said method the modified oligonucleotide increases the amount of the target protein in a human cell, wherein the target protein is encoded by a target transcript comprising at least one translation suppression element, wherein the translation suppression element comprises a uORF, wherein the uORF comprises an AUG codon, and thereby increasing expression of the target protein in the cell, wherein the modified oligonucleotide: is complementary to the target transcript at the uORF start site region; has a nucleobase sequence 100% complementary to the target transcript; consists of 15 to 22 linked nucleosides; and comprises at least 15 modified nucleosides, each modified nucleoside independently comprising a modified sugar moiety, wherein the antisense compound does not recruit RNase H.

The present disclosure provides compounds that interact with the 5'-UTR of a target transcript to increase translation of a target protein. It was discovered that certain compounds that interact with the 5'-UTR can increase translation of a given target transcript. For example, in certain embodiments, the present disclosure provides antisense compounds targeted to one or more regions of the 5'-UTR.

Antisense oligonucleotide technology has been used most often to reduce the amount an mRNA via antisense induced RNase H cleavage or to alter splicing of a pre-mRNA transcript in a cell. The present disclosure provides antisense compounds that increase expression of a target protein in a cell. In this manner, antisense oligonucleotides may be used to increase the expression of a desired protein in a cell. In the present disclosure, an increase in the expression of a target protein in a cell is achieved by having the antisense compound reduce ribosomal recognition of one or more upstream open reading frames. Recognition of an upstream open reading frame reduces expression of a target protein in a cell. Therefore, in the present disclosure, targeting the upstream open reading frame, or the nucleobase sequence upstream or downstream of the upstream open reading frame, reduces ribosomal recognition of the upstream open reading frame and thereby increases expression of one or more target proteins. Therefore, in the present disclosure, targeting the upstream open reading frame, or the nucleobase sequence upstream or downstream of the upstream open reading frame, reduces ribosomal recognition of the upstream open reading frame and thereby increases ribosomal recognition of a start codon in the primary open reading frame.

The present invention uses antisense compounds to increase expression of a target protein. In certain instances, a transcript encoding a protein of interest includes a pORF and one or more additional start sites, such as uORF start sites. The present disclosure provides modified oligonucleotides that are complementary to the target transcript at such uORF start sites. Antisense compounds designed to reduce the amount of a target protein typically induce cleavage of the target transcript (e.g., through recruitment of RNase H). In contrast, in the present invention, modified oligonucleotides are not designed to elicit cleavage The modified oligonucleotides of the present invention may mask a uORF start site in favor of increased translation at a pORF start site. The modified oligonucleotides of the present invention may disrupt initiation of translation at a uORF start site andthereby increase translation of the target protein. The modified oligonucleotides of the present invention may disrupt the regulatory function of the 5'-UTR. The modified oligonucleotides of the present invention may recruit proteins to the transcript that interfere with initiation of translation at the uORF start siteAntisense compounds of the invention may result in decreased translation of a uORF polypeptide.

The present disclosure provides methods of increasing translation of a target protein in a cell, comprising contacting the cell with an antisense compound comprising a modified oligonucleotide, wherein the target protein is encoded by a target transcript comprising at least one translation suppression element comprising a uORF and wherein the modified oligonucleotide is complementary to a target site within a translation suppression element at the uORF start site region of the target transcript; and thereby increasing translation of the target protein in the cell.

The present disclosure provides methods of decreasing suppression of translation of a target protein in a cell, comprising contacting the cell with an antisense compound comprising a modified oligonucleotide, wherein the target protein is encoded by a target transcript comprising at least one translation suppression element comprising a uORF and wherein the modified oligonucleotide is complementary to a target site within a translation suppression element at the uORF start site region of the target transcript; and thereby decreasing suppression of translation of the target protein in the cell.

The present disclosure provides methods of increasing the amount or activity of a target protein in a cell, comprising contacting the cell with an antisense compound comprising a modified oligonucleotide, wherein the target protein is encoded by a target transcript comprising at least one translation suppression element comprising a uORF and wherein the modified oligonucleotide is complementary to a target site within a translation suppression element at the uORF start site region of the target transcript; and thereby increasing the amount or activity of the target protein in the cell.

The present disclosure provides methods of increasing the amount a target protein in a cell, comprising contacting the cell with an antisense compound comprising a modified oligonucleotide, wherein the target protein is encoded by a target transcript comprising at least one translation suppression element comprising a uORF and wherein the modified oligonucleotide is complementary to a target site within a translation suppression element at the uORF start site region of the target transcript; and thereby increasing expression of the target protein in the cell.

### DETAILED DESCRIPTION

It is to be understood that both the foregoing general description and the following detailed description are exemplary and explanatory only and are not restrictive of the invention, as set out in the appended claims. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### DEFINITIONS

Unless otherwise indicated, the following terms have the following meanings:
As used herein, "target transcript" means a transcript that encodes a target protein. In certain embodiments, a target transcript contains a primary open reading frame that encodes a primary protein and one or more start sites at which translation of a polypeptide that is not the target protein may be initiated. In certain such embodiments, a target transcript contains a primary open reading frame and a uORF. In certain such embodiments, a target transcript contains a primary open reading frame and more than one uORF.

As used herein, "translation suppression element," means any sequence and/or secondary structure in the 5'-UTR of a target transcript that reduces, inhibits, and/or suppresses translation of the target transcript. In the claims, a translation suppression element comprises a uORF.

As used herein, "translation suppression element inhibitor," means any agent capable of specifically inhibiting the activity of a translation suppression element. In certain embodiments, the activity of a translation suppression element inhibitor is suppression of translation of the pORF polypeptide or protein on the same transcript. For example, translation suppression element inhibitors include nucleic acids (including antisense compounds and siRNA), peptides, antibodies, small molecules, and other agents capable of inhibiting the amount or activity of a translation suppression element.

As used herein, "translation suppression element region" means a portion of the target transcript that comprises one or more translation suppression elements. In certain embodiments, a translation suppression element region comprises a uORF. In certain embodiments, a translation suppression element region comprises more than one uORF. In certain embodiments, a translation suppression element region comprises a uORF and at least one translation suppression element that is not a uORF.

As used herein, "GC content" means the percentage of total nucleosides in a particular portion of a nucleic acid or oligonucleotide that are either G or C or that base pair with G or C.

As used herein, "consecutive GC nucleosides" or "consecutive GC nucleotides" means a portion of adjacent nucleosides in a nucleic acid or oligonucleotide that are all either G or C or that base pair with G or C.

As used herein, "target protein" means a protein that one desires to increase in amount, concentration, or activity. In certain embodiments, the target protein is encoded by the primary open reading frame of a target transcript.

As used herein, "target transcript" means a transcript that encodes a target protein. In certain embodiments, a target transcript contains a primary open reading frame that encodes a primary protein and one or more start sites at which translation of a polypeptide that is not the target protein may be initiated. In certain such embodiments, a target transcript contains a primary open reading frame and a uORF. In certain such embodiments, a target transcript contains a primary open reading frame and more than one uORF.

As used herein, "primary open reading frame" or "pORF" means the portion of the target transcript that encodes the primary protein associated with the transcript. In the present disclosure, the pORF encodes the target protein.

As used herein, "primary protein" means a protein encoded by a primary open reading frame.

As used herein, "target site" means the portion of the target transcript having a nucleobase sequence that is complementary to a portion of the nucleobase sequence of a modified oligonucleotide. In the present disclosure, the modified oligonucleotide is complementary to the target site across the entire length of the modified oligonucleotide.

As used herein, "start site" means a group of nucleobases on a transcript at which a ribosomal subunit is recruited. In certain embodiments, a start site may result in initiation of translation. In certain embodiments, a start site is an AUG codon. In certain embodiments, a start site is a non-canonical start codon.

As used herein, "upstream open reading frame start site" or "uORF start site" means a start site that is upstream of the pORF start codon. A uORF start site initiates translation of a polypeptide that is not the target protein.

As used herein, "uORF start site region" means a portion of the target transcript that comprises a uORF start site. In certain embodiments, a uORF start site region comprises a uORF start site and the 100 nucleosides upstream and downstream of the uORF start site. In certain embodiments, a uORF start site region comprises a uORF start site and the 75 nucleosides upstream and downstream of the uORF start site. In certain embodiments, a uORF start site region comprises a uORF start site and the 50 nucleosides upstream and downstream of the uORF start site. In certain embodiments, a uORF start site region comprises a uORF start site and the 30 nucleosides upstream and downstream of the uORF start site. In certain embodiments, a uORF start site region comprises a uORF start site and the 20 nucleosides upstream and downstream of the uORF start site. In certain embodiments, a uORF start site region comprises the 5' untranslated region. In certain embodiments, a uORF start site region consists of the 5'-UTR.

As used herein, "uORF" or "upstream open reading frame" means a portion of a target transcript that comprises a start site upstream of (i.e. 5' of) the pORF and an in frame termination codon. In certain embodiments, a uORF is the portion of the target transcript that is translated when translation is initiated at a uORF start site. In certain embodiments, a uORF does not overlap with a pORF. In certain embodiments, a uORF does overlap with a pORF. In certain embodiments a uORF overlaps with another uORF. In certain embodiments, a uORF is out of frame with a pORF.

As used herein, "wild-type uORF start site" means a uORF start site that does not arise from a mutation.

As used herein, "wild-type uORF start site region" means the uORF start site region of a wild-type uORF start site.

As used herein, "a uORF start site that arises from a mutation" means a uORF start site, where the same portion of the target transcript on the wild-type allele does not contain a uORF start site.

As used herein, "uORF polypeptide" means a polypeptide encoded by a uORF. In certain embodiments, a uORF polypeptide is a protein.

As used herein, "uORF inhibitor" means any agent capable of specifically inhibiting the activity of a uORF. In certain embodiments, the activity of a uORF is suppression of translation of the pORF polypeptide or protein on the same transcript. In certain embodiments, the activity of a uORF is suppression of translation of the pORF polypeptide or protein on a different transcript. For example, uORF specific inhibitors include nucleic acids (including antisense compounds and siRNA), peptides, antibodies, small molecules, and other agents capable of inhibiting the amount or activity of a uORF.

References to methods are references to in vitro methods, and references to methods of treatment in the subsequent paragraphs of this description are to be interpreted as references to the compounds, pharmaceutical compositions of the present invention for use in a method for treatment of the human (or animal) body by therapy, as set out in the appended claims.

As used herein, "suppression of translation of a target protein in a cell," means that translation of the target protein is less than the translation of the target protein in the absence of one or more TSEs.

As used herein, "nucleoside" means a compound comprising a nucleobase moiety and a sugar moiety. Nucleosides include, but are not limited to, naturally occurring nucleosides (as found in DNA and RNA) and modified nucleosides. Nucleosides may be linked to a phosphate moiety.

As used herein, "chemical modification" means a chemical difference in a compound when compared to a naturally occurring counterpart. Chemical modifications of oligonucleotides include nucleoside modifications (including sugar moiety modifications and nucleobase modifications) and internucleoside linkage modifications. In reference to an oligonucleotide, chemical modification does not include differences only in nucleobase sequence.

As used herein, "furanosyl" means a structure comprising a 5-membered ring comprising four carbon atoms and one oxygen atom.

As used herein, "naturally occurring sugar moiety" means a ribofuranosyl as found in naturally occurring RNA or a deoxyribofuranosyl as found in naturally occurring DNA.

As used herein, "sugar moiety" means a naturally occurring sugar moiety or a modified sugar moiety of a nucleoside.

As used herein, "modified sugar moiety" means a substituted sugar moiety or a sugar surrogate.

As used herein, "substituted sugar moiety" means a furanosyl that is not a naturally occurring sugar moiety. Substituted sugar moieties include, but are not limited to furanosyls comprising substituents at the 2'-position, the 3'-position, the 5'-position and/or the 4'-position. Certain substituted sugar moieties are bicyclic sugar moieties.

As used herein, "2'-substituted sugar moiety" means a furanosyl comprising a substituent at the 2'-position other than H or OH. Unless otherwise indicated, a 2'-substituted sugar moiety is not a bicyclic sugar moiety (i.e., the 2'-substituent of a 2'-substituted sugar moiety does not form a bridge to another atom of the furanosyl ring.

As used herein, "MOE" means -OCH₂CH₂OCH₃.

As used herein, "2'-F nucleoside" refers to a nucleoside comprising a sugar comprising fluorine at the 2' position. Unless otherwise indicated, the fluorine in a 2'-F nucleoside is in the ribo position (replacing the OH of a natural ribose).

As used herein, "2'-(ara)-F" refers to a 2'-F substituted nucleoside, wherein the fluoro group is in the arabino position.

As used herein the term "sugar surrogate" means a structure that does not comprise a furanosyl and that is capable of replacing the naturally occurring sugar moiety of a nucleoside, such that the resulting nucleoside sub-units are capable of linking together and/or linking to other nucleosides to form an oligonucleotide which is capable of hybridizing to a complementary oligonucleotide. Such structures include rings comprising a different number of atoms than furanosyl (e.g., 4, 6, or 7-membered rings); replacement of the oxygen of a furanosyl with a non-oxygen atom (e.g., carbon, sulfur, or nitrogen); or both a change in the number of atoms and a replacement of the oxygen. Such structures may also comprise substitutions corresponding to those described for substituted sugar moieties (e.g., 6-membered carbocyclic bicyclic sugar surrogates optionally comprising additional substituents). Sugar surrogates also include more complex sugar replacements (e.g., the non-ring systems of peptide nucleic acid). Sugar surrogates include without limitation morpholinos, cyclohexenyls and cyclohexitols.

As used herein, "bicyclic sugar moiety" means a modified sugar moiety comprising a 4 to 7 membered ring (including but not limited to a furanosyl) comprising a bridge connecting two atoms of the 4 to 7 membered ring to form a second ring, resulting in a bicyclic structure. In certain embodiments, the 4 to 7 membered ring is a sugar ring. In certain embodiments the 4 to 7 membered ring is a furanosyl. In certain such embodiments, the bridge connects the 2'-carbon and the 4'-carbon of the furanosyl.

As used herein, "nucleotide" means a nucleoside further comprising a phosphate linking group. As used herein, "linked nucleosides" may or may not be linked by phosphate linkages and thus includes, but is not limited to "linked nucleotides." As used herein, "linked nucleosides" are nucleosides that are connected in a continuous sequence (i.e. no additional nucleosides are present between those that are linked).

As used herein, "nucleobase" means a group of atoms that can be linked to a sugar moiety to create a nucleoside that is capable of incorporation into an oligonucleotide, and wherein the group of atoms is capable of bonding with a complementary naturally occurring nucleobase of another oligonucleotide or nucleic acid. Nucleobases may be naturally occurring or may be modified.

As used herein the terms, "unmodified nucleobase" or "naturally occurring nucleobase" means the naturally occurring heterocyclic nucleobases of RNA or DNA: the purine bases adenine (A) and guanine (G), and the pyrimidine bases thymine (T), cytosine (C) (including 5-methyl C), and uracil (U).

As used herein, "modified nucleobase" means any nucleobase that is not a naturally occurring nucleobase.

As used herein, "modified nucleoside" means a nucleoside comprising at least one chemical modification compared to naturally occurring RNA or DNA nucleosides. Modified nucleosides comprise a modified sugar moiety and/or a modified nucleobase.

As used herein, "bicyclic nucleoside" or "BNA" means a nucleoside comprising a bicyclic sugar moiety.

As used herein, "constrained ethyl nucleoside" or "cEt" means a nucleoside comprising a bicyclic sugar moiety comprising a 4'-CH(CH₃)-O-2'bridge.

As used herein, "locked nucleic acid nucleoside" or "LNA" means a nucleoside comprising a bicyclic sugar moiety comprising a 4'-CH₂-O-2'bridge.

As used herein, "2'-substituted nucleoside" means a nucleoside comprising a substituent at the 2'-position other than H or OH. Unless otherwise indicated, a 2'-substituted nucleoside is not a bicyclic nucleoside.

As used herein, "2'-deoxynucleoside" means a nucleoside comprising 2'-H furanosyl sugar moiety, as found in naturally occurring deoxyribonucleosides (DNA). In certain embodiments, a 2'-deoxynucleoside may comprise a modified nucleobase or may comprise an RNA nucleobase (e.g., uracil).

As used herein, "oligonucleotide" means a compound comprising a plurality of linked nucleosides. In certain embodiments, an oligonucleotide comprises one or more unmodified ribonucleosides (RNA) and/or unmodified deoxyribonucleosides (DNA) and/or one or more modified nucleosides.

As used herein "oligonucleoside" means an oligonucleotide in which none of the internucleoside linkages contains a phosphorus atom. As used herein, oligonucleotides include oligonucleosides.

As used herein, "modified oligonucleotide" means an oligonucleotide comprising at least one modified nucleoside and/or at least one modified internucleoside linkage. Examples of modified oligonucleotides include single-stranded and double-stranded compounds, such as, antisense compounds, siRNAs, shRNAs, ssRNAs, and occupancy-based compounds.

As used herein "internucleoside linkage" means a covalent linkage between adjacent nucleosides in an oligonucleotide.

As used herein "naturally occurring internucleoside linkage" means a 3' to 5' phosphodiester linkage.

As used herein, "modified internucleoside linkage" means any internucleoside linkage other than a naturally occurring internucleoside linkage.

As used herein, "oligomeric compound" means a polymeric structure comprising two or more sub-structures. In certain embodiments, the sub-structures are nucleotides or nucleosides. In certain embodiments, an oligomeric compound comprises an oligonucleotide. In certain embodiments, an oligomeric compound consists of an oligonucleotide. In certain embodiments, an oligomeric compound consists of an antisense compound.

As used herein, "terminal group" means one or more atom attached to either, or both, the 3' end or the 5' end of an oligonucleotide. In certain embodiments a terminal group is a conjugate group. In certain embodiments, a terminal group comprises one or more terminal group nucleosides.

As used herein, "conjugate group" means an atom or group of atoms bound to an oligonucleotide or oligomeric compound. In general, conjugate groups modify one or more properties of the oligonucleotide or oligomeric compound to which they are attached, including, but not limited to pharmacodynamic, pharmacokinetic, binding, absorption, cellular distribution, cellular uptake, charge and/or clearance properties.

As used herein, "conjugate linking group" means any atom or group of atoms used to attach a conjugate to an oligonucleotide or oligomeric compound.

As used herein, "antisense compound" means a compound comprising or consisting of an oligonucleotide at least a portion of which is complementary to a target nucleic acid to which it is capable of hybridizing, resulting in at least one antisense activity.

As used herein, "antisense activity" means any detectable and/or measurable change attributable to the hybridization of an antisense compound to its target nucleic acid.

As used herein, "detecting" or "measuring" means that a test or assay for detecting or measuring is performed. Such detection and/or measuring may result in a value of zero. Thus, if a test for detection or measuring results in a finding of no activity (activity of zero), the step of detecting or measuring the activity has nevertheless been performed.

As used herein, "detectable and/or measureable activity" means a measurable activity that is not zero.

As used herein, "essentially unchanged" means little or no change in a particular parameter, particularly relative to another parameter which changes much more. In certain embodiments, a parameter is essentially unchanged when it changes less than 5%. In certain embodiments, a parameter is essentially unchanged if it changes less than two-fold while another parameter changes at least ten-fold. For example, in certain embodiments, an antisense activity is a change in the amount of a target nucleic acid. In certain such embodiments, the amount of a non-target nucleic acid is essentially unchanged if it changes much less than the target nucleic acid does, but the change need not be zero.

As used herein, "expression" means the process by which a gene ultimately results in a protein. Expression includes, but is not limited to, transcription, post-transcriptional modification (e.g., splicing, polyadenlyation, addition of 5'-cap), translation, and post-translational modification.

As used herein, "translation" means the process in which a polypeptide (e.g. a protein) is translated from an mRNA. In certain embodiments, an increase in translation means an increase in the number of polypeptide (e.g. a protein) molecules that are made per copy of mRNA that encodes said polypeptide.

As used herein, "target nucleic acid" means a nucleic acid molecule to which an antisense compound is intended to hybridize.

As used herein, "mRNA" means an RNA molecule that encodes a protein.

As used herein, "pre-mRNA" means an RNA transcript that has not been fully processed into mRNA. Pre-RNA includes one or more intron.

As used herein, "targeting" or "targeted to" means the association of an antisense compound to a particular target nucleic acid molecule or a particular region of a target nucleic acid molecule. An antisense compound targets a target nucleic acid if it is sufficiently complementary to the target nucleic acid to allow hybridization under physiological conditions.

As used herein, "nucleobase complementarity" or "complementarity" when in reference to nucleobases means a nucleobase that is capable of base pairing with another nucleobase. For example, in DNA, adenine (A) is complementary to thymine (T). For example, in RNA, adenine (A) is complementary to uracil (U). In certain embodiments, complementary nucleobase means a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair. Nucleobases comprising certain modifications may maintain the ability to pair with a counterpart nucleobase and thus, are still capable of nucleobase complementarity.

As used herein, "non-complementary" in reference to nucleobases means a pair of nucleobases that do not form hydrogen bonds with one another.

As used herein, "complementary" in reference to oligomeric compounds (e.g., linked nucleosides, oligonucleotides, or nucleic acids) means the capacity of such oligomeric compounds or regions thereof to hybridize to another oligomeric compound or region thereof through nucleobase complementarity under stringent conditions. Complementary oligomeric compounds need not have nucleobase complementarity at each nucleoside. Rather, some mismatches are tolerated. In certain embodiments, complementary oligomeric compounds or regions are complementary at 70% of the nucleobases (70% complementary). In certain embodiments, complementary oligomeric compounds or regions are 80% complementary. In certain embodiments, complementary oligomeric compounds or regions are 90% complementary. In certain embodiments, complementary oligomeric compounds or regions are 95% complementary. In certain embodiments, complementary oligomeric compounds or regions are 100% complementary.

As used herein, "mismatch" means a nucleobase of a first oligomeric compound that is not capable of pairing with a nucleobase at a corresponding position of a second oligomeric compound, when the first and second oligomeric compound are aligned. Either or both of the first and second oligomeric compounds may be oligonucleotides.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleobases.

As used herein, "specifically hybridizes" means the ability of an oligomeric compound to hybridize to one nucleic acid site with greater affinity than it hybridizes to another nucleic acid site. In certain embodiments, an antisense compound specifically hybridizes to more than one target site.

As used herein, "fully complementary" in reference to an oligonucleotide or portion thereof means that each nucleobase of the oligonucleotide or portion thereof is capable of pairing with a nucleobase of a complementary nucleic acid or contiguous portion thereof. Thus, a fully complementary region comprises no mismatches or unhybridized nucleobases in either strand.

As used herein, "percent complementarity" means the percentage of nucleobases of an oligomeric compound that are complementary to an equal-length portion of a target nucleic acid. Percent complementarity is calculated by dividing the number of nucleobases of the oligomeric compound that are complementary to nucleobases at corresponding positions in the target nucleic acid by the total length of the oligomeric compound.

As used herein, "percent identity" means the number of nucleobases in a first nucleic acid that are the same type (independent of chemical modification) as nucleobases at corresponding positions in a second nucleic acid, divided by the total number of nucleobases in the first nucleic acid.

As used herein, "modulation" means a change of amount or quality of a molecule, function, or activity when compared to the amount or quality of a molecule, function, or activity prior to modulation. For example, modulation includes the change, either an increase (stimulation or induction) or a decrease (inhibition or reduction) in gene expression. As a further example, modulation of expression can include a change in splice site selection of pre-mRNA processing, resulting in a change in the absolute or relative amount of a particular splice-variant compared to the amount in the absence of modulation.

As used herein, "modification motif" means a pattern of chemical modifications in an oligomeric compound or a region thereof. Motifs may be defined by modifications at certain nucleosides and/or at certain linking groups of an oligomeric compound.

As used herein, "nucleoside motif" means a pattern of nucleoside modifications in an oligomeric compound or a region thereof. The linkages of such an oligomeric compound may be modified or unmodified. Unless otherwise indicated, motifs herein describing only nucleosides are intended to be nucleoside motifs. Thus, in such instances, the linkages are not limited.

As used herein, "sugar motif" means a pattern of sugar modifications in an oligomeric compound or a region thereof.

As used herein, "linkage motif" means a pattern of linkage modifications in an oligomeric compound or region thereof. The nucleosides of such an oligomeric compound may be modified or unmodified. Unless otherwise indicated, motifs herein describing only linkages are intended to be linkage motifs. Thus, in such instances, the nucleosides are not limited.

As used herein, "nucleobase modification motif" means a pattern of modifications to nucleobases along an oligonucleotide. Unless otherwise indicated, a nucleobase modification motif is independent of the nucleobase sequence.

As used herein, "sequence motif" means a pattern of nucleobases arranged along an oligonucleotide or portion thereof. Unless otherwise indicated, a sequence motif is independent of chemical modifications and thus may have any combination of chemical modifications, including no chemical modifications.

As used herein, "type of modification" in reference to a nucleoside or a nucleoside of a "type" means the chemical modification of a nucleoside and includes modified and unmodified nucleosides. Accordingly, unless otherwise indicated, a "nucleoside having a modification of a first type" may be an unmodified nucleoside.

As used herein, "differently modified" mean chemical modifications or chemical substituents that are different from one another, including absence of modifications. Thus, for example, a MOE nucleoside and an unmodified DNA nucleoside are "differently modified," even though the DNA nucleoside is unmodified. Likewise, DNA and RNA are "differently modified," even though both are naturally-occurring unmodified nucleosides. Nucleosides that are the same but for comprising different nucleobases are not differently modified. For example, a nucleoside comprising a 2'-OMe modified sugar and an unmodified adenine nucleobase and a nucleoside comprising a 2'-OMe modified sugar and an unmodified thymine nucleobase are not differently modified.

As used herein, "the same type of modifications" refers to modifications that are the same as one another, including absence of modifications. Thus, for example, two unmodified DNA nucleoside have "the same type of modification," even though the DNA nucleoside is unmodified. Such nucleosides having the same type modification may comprise different nucleobases.

As used herein, "pharmaceutically acceptable carrier or diluent" means any substance suitable for use in administering to an animal. In certain embodiments, a pharmaceutically acceptable carrier or diluent is sterile saline. In certain embodiments, such sterile saline is pharmaceutical grade saline.

As used herein, "substituent" and "substituent group," means an atom or group that replaces the atom or group of a named parent compound. For example a substituent of a modified nucleoside is any atom or group that differs from the atom or group found in a naturally occurring nucleoside (e.g., a modified 2'-substuent is any atom or group at the 2'-position of a nucleoside other than H or OH). Substituent groups can be protected or unprotected. In certain embodiments, compounds of the present invention have substituents at one or at more than one position of the parent compound. Substituents may also be further substituted with other substituent groups and may be attached directly or via a linking group such as an alkyl or hydrocarbyl group to a parent compound.

Likewise, as used herein, "substituent" in reference to a chemical functional group means an atom or group of atoms differs from the atom or a group of atoms normally present in the named functional group. In certain embodiments, a substituent replaces a hydrogen atom of the functional group (e.g., in certain embodiments, the substituent of a substituted methyl group is an atom or group other than hydrogen which replaces one of the hydrogen atoms of an unsubstituted methyl group). Unless otherwise indicated, groups amenable for use as substituents include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxy (-O-Rₐₐ), aryl, aralkyl, heterocyclic radical, heteroaryl, heteroarylalkyl, amino (-N(R_{bb})(R_{cc})), imino(=NR_{bb}), amido (-C(O)N(R_{bb})(R_{cc}) or -N(R_{bb})C(O)Rₐₐ), azido (-N₃), nitro (-NO₂), cyano (-CN), carbamido (-OC(O)N(R_{bb})(R_{cc}) or -N(R_{bb})C(O)ORₐₐ), ureido (-N(R_{bb})C(O)N(R_{bb})(R_{cc})), thioureido (-N(R_{bb})C(S)N(R_{bb})-(R_{cc})), guanidinyl (-N(R_{bb})C(=NR_{bb})N(R_{bb})(R_{cc})), amidinyl (-C(=NR_{bb})N(R_{bb})(R_{cc}) or -N(R_{bb})C(=NR_{bb})(Rₐₐ)), thiol (-SR_{bb}), sulfinyl (-S(O)R_{bb}), sulfonyl (-S(O)₂R_{bb}) and sulfonamidyl (-S(O)₂N(R_{bb})(R_{cc}) or -N(R_{bb})S-(O)₂R_{bb}). Wherein each Rₐₐ, R_{bb} and R_{cc} is, independently, H, an optionally linked chemical functional group or a further substituent group with a preferred list including without limitation, alkyl, alkenyl, alkynyl, aliphatic, alkoxy, acyl, aryl, aralkyl, heteroaryl, alicyclic, heterocyclic and heteroarylalkyl. Selected substituents within the compounds described herein are present to a recursive degree.

As used herein, "alkyl," as used herein, means a saturated straight or branched hydrocarbon radical containing up to twenty four carbon atoms. Examples of alkyl groups include without limitation, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred.

As used herein, "alkenyl," means a straight or branched hydrocarbon chain radical containing up to twenty four carbon atoms and having at least one carbon-carbon double bond. Examples of alkenyl groups include without limitation, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.

As used herein, "alkynyl," means a straight or branched hydrocarbon radical containing up to twenty four carbon atoms and having at least one carbon-carbon triple bond. Examples of alkynyl groups include, without limitation, ethynyl, 1-propynyl, 1-butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substituent groups.

As used herein, "acyl," means a radical formed by removal of a hydroxyl group from an organic acid and has the general Formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substituent groups.

As used herein, "alicyclic" means a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substituent groups.

As used herein, "aliphatic" means a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation, polyalkoxys, such as polyalkylene glycols, polyamines, and polyimines. Aliphatic groups as used herein may optionally include further substituent groups.

As used herein, "alkoxy" means a radical formed between an alkyl group and an oxygen atom wherein the oxygen atom is used to attach the alkoxy group to a parent molecule. Examples of alkoxy groups include without limitation, methoxy, ethoxy, propoxy, isopropoxy, *n*-butoxy, sec-butoxy, *tert*-butoxy, n-pentoxy, neopentoxy, n-hexoxy and the like. Alkoxy groups as used herein may optionally include further substituent groups.

As used herein, "aminoalkyl" means an amino substituted C₁-C₁₂ alkyl radical. The alkyl portion of the radical forms a covalent bond with a parent molecule. The amino group can be located at any position and the aminoalkyl group can be substituted with a further substituent group at the alkyl and/or amino portions.

As used herein, "aralkyl" and "arylalkyl" mean an aromatic group that is covalently linked to a C₁-C₁₂ alkyl radical. The alkyl radical portion of the resulting aralkyl (or arylalkyl) group forms a covalent bond with a parent molecule. Examples include without limitation, benzyl, phenethyl and the like. Aralkyl groups as used herein may optionally include further substituent groups attached to the alkyl, the aryl or both groups that form the radical group.

As used herein, "aryl" and "aromatic" mean a mono- or polycyclic carbocyclic ring system radicals having one or more aromatic rings. Examples of aryl groups include without limitation, phenyl, naphthyl, tetrahydronaphthyl, indanyl, idenyl and the like. Preferred aryl ring systems have from about 5 to about 20 carbon atoms in one or more rings. Aryl groups as used herein may optionally include further substituent groups.

As used herein, "halo" and "halogen," mean an atom selected from fluorine, chlorine, bromine and iodine.

As used herein, "heteroaryl," and "heteroaromatic," mean a radical comprising a mono- or polycyclic aromatic ring, ring system or fused ring system wherein at least one of the rings is aromatic and includes one or more heteroatoms. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include without limitation, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl and the like. Heteroaryl radicals can be attached to a parent molecule directly or through a linking moiety such as an aliphatic group or hetero atom. Heteroaryl groups as used herein may optionally include further substituent groups.

As used herein, "Intracerebroventricular" or "ICV" means administration into the ventricular system of the brain.

As used herein, "wherein the translation suppression element region comprises one and only one uORF," means that exactly one uORF is present in the translation suppression element region. In certain embodiments, exactly one uORF is present in the 5'-UTR.

The compounds described herein include variations in which one or more atoms are replaced with a non-radioactive isotope or radioactive isotope of the indicated element. For example, compounds herein that comprise hydrogen atoms encompass all possible deuterium substitutions for each of the 1H hydrogen atoms. Isotopic substitutions encompassed by the compounds herein include but are not limited to: 2H or 3H in place of 1H, 13C or 14C in place of 12C, 15N in place of 14N, 170 or 180 in place of 160, and 33S, 34S, 35S, or 36S in place of 32S. In certain embodiments, non-radioactive isotopic substitutions may impart new properties on the oligomeric compound that are beneficial for use as a therapeutic or research tool. In certain embodiments, radioactive isotopic substitutions may make the compound suitable for research purposes such as imaging.

### Certain Modified Oligonucleotides

The present invention provides antisense compounds. In the present disclosure, antisense compounds comprise a modified oligonucleotide. In certain embodiments, such antisense compounds comprise modified oligonucleotides and optionally one or more conjugate and/or terminal groups. In certain embodiments, an antisense compound consists of a modified oligonucleotide. In the present disclosure, modified oligonucleotides comprise one or more chemical modifications. Such chemical modifications include modifications of one or more nucleoside (including modifications to the sugar moiety and/or the nucleobase) and/or modifications to one or more internucleoside linkage.

### a. Certain Modified Nucleosides

In the present disclosure, provided herein are antisense compounds comprising or consisting of oligonucleotides comprising at least one modified nucleoside. Such modified nucleosides comprise a modified sugar moeity, a modified nucleobase, or both a modifed sugar moiety and a modified nucleobase.

### i. Certain Sugar Moieties

In the present disclosure, antisense compounds of the invention comprise one or more modifed nucleosides comprising a modifed sugar moiety. Such antisense compounds comprising one or more sugar-modified nucleosides may have desirable properties, such as enhanced nuclease stability or increased binding affinity with a target nucleic acid relative to antisense compounds comprising only nucleosides comprising naturally occurring sugar moieties. In certain embodiments, modified sugar moieties are substitued sugar moieties. In certain embodiments, modified sugar moieties are bicyclic or tricyclic sugar moieties. In certain embodiments, modified sugar moieties are sugar surrogates. Such sugar surogates may comprise one or more substitutions corresponding to those of substituted sugar moieties.

In certain embodiments, modified sugar moieties are substituted sugar moieties comprising one or more substituent, including but not limited to substituents at the 2' and/or 5' positions. Examples of sugar substituents suitable for the 2'-position, include, but are not limited to: 2'-F, 2'-OCH₃ ("OMe" or "O-methyl"), and 2'-O(CH₂)₂OCH₃ ("MOE"). In certain embodiments, sugar substituents at the 2' position is selected from allyl, amino, azido, thio, O-allyl, O-C₁-C₁₀ alkyl, O-C₁-C₁₀ substituted alkyl; O- C₁-C₁₀ alkoxy; O- C₁-C₁₀ substituted alkoxy, OCF₃, O(CH₂)₂SCH₃, O(CH₂)₂-O-N(Rm)(Rn), and O-CH₂-C(=O)-N(Rm)(Rn), where each Rm and Rn is, independently, H or substituted or unsubstituted C₁-C₁₀ alkyl. Examples of sugar substituents at the 5'-position, include, but are not limited to:, 5'-methyl (R or S); 5'-vinyl, and 5'-methoxy. In certain embodiments, substituted sugars comprise more than one non-bridging sugar substituent, for example, 2'-F-5'-methyl sugar moieties (*see,*e.g*.,* PCT International Application WO 2008/101157, for additional 5', 2'-bis substituted sugar moieties and nucleosides).

Nucleosides comprising 2'-substituted sugar moieties are referred to as 2'-substituted nucleosides. In certain embodiments, a 2'- substituted nucleoside comprises a 2'-substituent group selected from halo, allyl, amino, azido, O- C₁-C₁₀ alkoxy; O- C₁-C₁₀ substituted alkoxy, SH, CN, OCN, CF₃, OCF₃, O-alkyl, S-alkyl, N(Rₘ)-alkyl; O- alkenyl, S- alkenyl, or N(Rₘ)-alkenyl; O- alkynyl, S- alkynyl, N(Rₘ)-alkynyl; O-alkylenyl-O-alkyl, alkynyl, alkaryl, aralkyl, O-alkaryl, O-aralkyl, O(CH₂)₂SCH₃, O-(CH₂)₂-O-N(Rₘ)(Rₙ) or O-CH₂-C(=O)-N(Rₘ)(Rₙ), where each Rₘ and Rₙ is, independently, H, an amino protecting group or substituted or unsubstituted C₁-C₁₀ alkyl. These 2'-substituent groups can be further substituted with one or more substituent groups independently selected from hydroxyl, amino, alkoxy, carboxy, benzyl, phenyl, nitro (NO₂), thiol, thioalkoxy (S-alkyl), halogen, alkyl, aryl, alkenyl and alkynyl.

In certain embodiments, a 2'- substituted nucleoside comprises a 2'-substituent group selected from F, NH₂, N₃, OCF₃, O-CH₃, O(CH₂)₃NH₂, CH₂-CH=CH₂, O-CH₂-CH=CH₂, OCH₂CH₂OCH₃, O(CH₂)₂SCH₃, O-(CH₂)₂-O-N(Rₘ)(Rₙ), O(CH₂)₂O(CH₂)₂N(CH₃)₂, and N-substituted acetamide (O-CH₂-C(=O)-N(Rₘ)(Rₙ) where each Rₘ and Rₙ is, independently, H, an amino protecting group or substituted or unsubstituted C₁-C₁₀ alkyl.

In certain embodiments, a 2'- substituted nucleoside comprises a sugar moiety comprising a 2'-substituent group selected from F, OCF₃, O-CH₃, OCH₂CH₂OCH₃, O(CH₂)₂SCH₃, O-(CH₂)₂-ON(CH₃)₂, -O(CH₂)₂O(CH₂)₂N(CH₃)₂, and O-CH₂-C(=O)-N(H)CH₃.

In certain embodiments, a 2'- substituted nucleoside comprises a sugar moiety comprising a 2'-substituent group selected from F, O-CH₃, and OCH₂CH₂OCH₃.

Certain modifed sugar moieties comprise a bridging sugar substituent that forms a second ring resulting in a bicyclic sugar moiety. In certain such embodiments, the bicyclic sugar moiety comprises a bridge between the 4' and the 2' furanose ring atoms. Examples of such 4' to 2' sugar substituents, include, but are not limited to: -[C(Rₐ)(R_{b})]ₙ-, -[C(Rₐ)(R_{b})]ₙ-O-, -C(RₐR_{b})-N(R)-O- or, -C(RₐR_{b})-O-N(R)-; 4'-CH₂-2', 4'-(CH₂)₂-2', 4'-(CH₂)₃-2',. 4'-(CH₂)-O-2' (LNA); 4'-(CH₂)-S-2'; 4'-(CH₂)₂-O-2' (ENA); 4'-CH(CH₃)-O-2' (cEt) and 4'-CH(CH₂OCH₃)-O-2',and analogs thereof *(see, e.g.,* U.S. Patent 7,399,845, issued on July 15, 2008); 4'-C(CH₃)(CH₃)-O-2'and analogs thereof, *(see, e.g.,* WO2009/006478, published January 8, 2009); 4'-CH₂-N(OCH₃)-2' and analogs thereof *(see, e.g.,* WO2008/150729, published December 11, 2008); 4'-CH₂-ON(CH₃)-2' *(see, e.g.,* US2004/0171570, published September 2, 2004 ); 4'-CH₂-O-N(R)-2', and 4'-CH₂-N(R)-O-2'-, wherein each Ris, independently, H, a protecting group, or C₁-C₁₂ alkyl; 4'-CH₂-N(R)-O-2', wherein R is H, C₁-C₁₂ alkyl, or a protecting group *(see,* U.S. Patent 7,427,672, issued on September 23, 2008); 4'-CH₂-C(H)(CH₃)-2' *(see, e.g.,* Chattopadhyaya, et al., J. Org. Chem.,2009, 74, 118-134); and 4'-CH₂-C(=CH₂)-2' and analogs thereof *(see,* published PCT International Application WO 2008/154401, published on December 8, 2008).

In certain embodiments, such 4' to 2' bridges independently comprise from 1 to 4 linked groups independently selected from -[C(Rₐ)(R_{b})]ₙ-, -C(Rₐ)=C(R_{b})-, -C(Rₐ)=N-, -C(=NRₐ)-, -C(=O)-, -C(=S)-, -O-, - Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Nucleosides comprising bicyclic sugar moieties are referred to as bicyclic nucleosides or BNAs. Bicyclic nucleosides include, but are not limited to, (A) α-L-Methyleneoxy (4'-CH₂-O-2') BNA, (B) β-D-Methyleneoxy (4'-CH₂-O-2') BNA (also referred to as locked nucleic acid or LNA) , (C) Ethyleneoxy (4'-(CH₂)₂-O-2') BNA, (D) Aminooxy (4'-CH₂-O-N(R)-2') BNA, (E) Oxyamino (4'-CH₂-N(R)-O-2') BNA, (F) Methyl(methyleneoxy) (4'-CH(CH₃)-O-2') BNA (also referred to as constrained ethyl or cEt), (G) methylene-thio (4'-CH₂-S-2') BNA, (H) methylene-amino (4'-CH2-N(R)-2') BNA, (I) methyl carbocyclic (4'-CH₂-CH(CH₃)-2') BNA, (J) propylene carbocyclic (4'-(CH₂)₃-2') BNA, and (M) 4'-CH₂-O-CH₂-2' as depicted below. 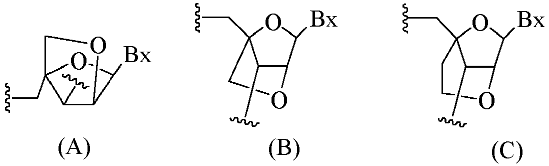 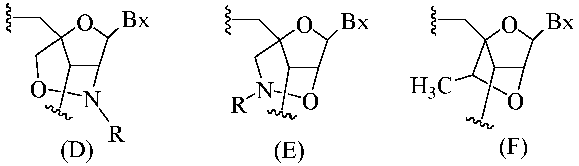 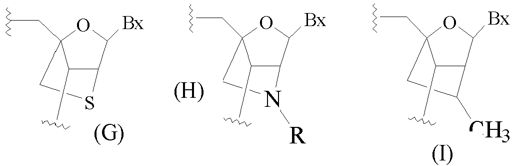 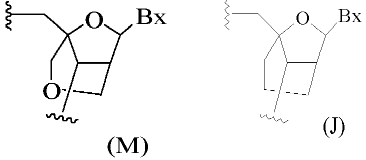 wherein Bx is a nucleobase moiety and R is, independently, H, a protecting group, or C₁-C₁₂ alkyl.

Additional bicyclic sugar moieties are known in the art, for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 129(26) 8362-8379 (Jul. 4, 2007); Elayadi et al., Curr. Opinion Invens. Drugs, 2001, 2, 558-561; Braasch et al., Chem. Biol., 2001, 8, 1-7; Orum et al., Curr. Opinion Mol. Ther., 2001, 3, 239-243; U.S. Patent Nos. 7,053,207, 6,268,490, 6,770,748, 6,794,499, 7,034,133, 6,525,191, 6,670,461, and 7,399,845; WO 2004/106356, WO 1994/14226, WO 2005/021570, and WO 2007/134181; U.S. Patent Publication Nos. US2004/0171570, US2007/0287831, and US2008/0039618; U.S. Patent Serial Nos. 12/129,154, 60/989,574, 61/026,995, 61/026,998, 61/056,564, 61/086,231, 61/097,787, and 61/099,844; and PCT International Applications Nos. PCT/US2008/064591, PCT/US2008/066154, and PCT/US2008/068922.

In certain embodiments, bicyclic sugar moieties and nucleosides incorporating such bicyclic sugar moieties are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') bicyclic nucleosides have been incorporated into antisense oligonucleotides that showed antisense activity (Frieden et al., Nucleic Acids Research, 2003, 21, 6365-6372).

In certain embodiments, substituted sugar moieties comprise one or more non-bridging sugar substituent and one or more bridging sugar substituent (e.g., 5'-substituted and 4'-2' bridged sugars). *(see,* PCT International Application WO 2007/134181, published on 11/22/07, wherein LNA is substituted with, for example, a 5'-methyl or a 5'-vinyl group).

In certain embodiments, modified sugar moieties are sugar surrogates. In certain such embodiments, the oxygen atom of the naturally occuring sugar is substituted, e.g., with a sulfer, carbon or nitrogen atom. In certain such embodiments, such modified sugar moiety also comprises bridging and/or non-bridging substituents as described above. For example, certain sugar surogates comprise a 4'-sulfer atom and a substitution at the 2'-position (*see,*e.g*.,* published U.S. Patent Application US2005/0130923, published on June 16, 2005) and/or the 5' position. By way of additional example, carbocyclic bicyclic nucleosides having a 4'-2' bridge have been described *(see, e.g.,* Freier et al., Nucleic Acids Research, 1997, 25(22), 4429-4443 and Albaek et al., J. Org. Chem., 2006, 71, 7731-7740).

In certain embodiments, sugar surrogates comprise rings having other than 5-atoms. For example, in certain embodiments, a sugar surrogate comprises a six-membered tetrahydropyran. Such tetrahydropyrans may be further modified or substituted. Nucleosides comprising such modified tetrahydropyrans include, but are not limited to, hexitol nucleic acid (HNA), anitol nucleic acid (ANA), manitol nucleic acid (MNA) *(see* Leumann, CJ. Bioorg. & Med. Chem. (2002) 10:841-854), fluoro HNA (F-HNA), and those compounds having Formula VII: 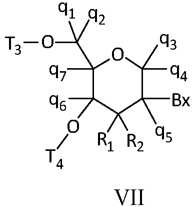 wherein independently for each of said at least one tetrahydropyran nucleoside analog of Formula VII:
Bx is a nucleobase moiety;
T₃ and T₄ are each, independently, an internucleoside linking group linking the tetrahydropyran nucleoside analog to the antisense compound or one of T₃ and T₄ is an internucleoside linking group linking the tetrahydropyran nucleoside analog to the antisense compound and the other of T₃ and T₄ is H, a hydroxyl protecting group, a linked conjugate group, or a 5' or 3'-terminal group;
   q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from among: hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J₁, J₂, and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is F. In certain embodiments, R₁ is fluoro and R₂ is H, R₁ is methoxy and R₂ is H, and R₁ is methoxyethoxy and R₂ is H.

Many other bicyclic and tricyclic sugar and sugar surrogate ring systems are known in the art that can be used to modify nucleosides *(see, e.g.,* review article: Leumann, J. C, Bioorganic & Medicinal Chemistry, 2002, 10, 841-854).

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following structure: 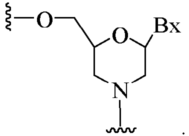 In certain embodiments, morpholinos may be modified, for example by adding or altering various substituent groups from the above morpholino structure. Such sugar surrogates are refered to herein as "modifed morpholinos."

Combinations of modifications are also provided without limitation, such as 2'-F-5'-methyl substituted nucleosides (see PCT International Application WO 2008/101157 Published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see, e.g.,* Srivastava et al., J. Am. Chem. Soc. 2007, 129(26), 8362-8379).

### ii. Certain Modified Nucleobases

In certain embodiments, nucleosides of the present invention comprise one or more unmodified nucleobases. In certain embodiments, nucleosides of the present invention comprise one or more modifed nucleobases.

In certain embodiments, modified nucleobases are selected from: universal bases, hydrophobic bases, promiscuous bases, size-expanded bases, and fluorinated bases as defined herein. 5-substituted pyrimidines, 6-azapyrimidines and N-2, N-6 and O-6 substituted purines, including 2-aminopropyladenine, 5-propynyluracil; 5-propynylcytosine; 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine, universal bases, hydrophobic bases, promiscuous bases, size-expanded bases, and fluorinated bases as defined herein. Further modified nucleobases include tricyclic pyrimidines such as phenoxazine cytidine( [5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, Kroschwitz, J.I., Ed., John Wiley & Sons, 1990, 858-859; those disclosed by Englisch et al., Angewandte Chemie, International Edition, 1991, 30, 613; and those disclosed by Sanghvi, Y.S., Chapter 15, Antisense Research and Applications, Crooke, S.T. and Lebleu, B., Eds., CRC Press, 1993, 273-288.

Representative United States patents that teach the preparation of certain of the above noted modified nucleobases as well as other modified nucleobases include without limitation, U.S. 3,687,808; 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121; 5,596,091; 5,614,617; 5,645,985; 5,681,941; 5,750,692; 5,763,588; 5,830,653 and 6,005,096, certain of which are commonly owned with the instant application.

### b. Certain Internucleoside Linkages

In certain embodiments, nucleosides may be linked together using any internucleoside linkage to form oligonucleotides. The two main classes of internucleoside linking groups are defined by the presence or absence of a phosphorus atom. Representative phosphorus containing internucleoside linkages include, but are not limited to, phosphodiesters (P=O), phosphotriesters, methylphosphonates, phosphoramidate, and phosphorothioates (P=S). Representative non-phosphorus containing internucleoside linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)₂-O-); and N,N'-dimethylhydrazine (-CH₂-N(CH₃)-N(CH₃)-). Modified linkages, compared to natural phosphodiester linkages, can be used to alter, typically increase, nuclease resistance of the oligonucleotide. In certain embodiments, internucleoside linkages having a chiral atom can be prepared as a racemic mixture, or as separate enantiomers. Representative chiral linkages include, but are not limited to, alkylphosphonates and phosphorothioates. Methods of preparation of phosphorous-containing and non-phosphorous-containing internucleoside linkages are well known to those skilled in the art.

The oligonucleotides described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids etc. Included in the antisense compounds provided herein are all such possible isomers, as well as their racemic and optically pure forms.

Neutral internucleoside linkages include without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### c. Certain Motifs

The invention provides modified oligocucleotides. In certain embodiments, modified oligonucleotides comprise one or more modified sugars. In certain embodiments, modified oligonucleotides comprise one or more modified nucleobases. In certain embodiments, modified oligonucleotides comprise one or more modified internucleoside linkages. In certain embodiments, the modifications (sugar modifications, nucleobase modifications, and/or linkage modifications) define a pattern or motif. In certain embodiments, the patterns of chemical modifications of sugar moieties, internucleoside linkages, and nucleobases are each independent of one another. Thus, a modifed oligonucleotide may be described by its sugar modification motif, internucleoside linkage motif and/or nucleobase modification motif (as used herein, nucleobase modification motif describes the chemical modifications to the nucleobases independent of the sequence of nucleobases).

In certain embodiments, every sugar moieity of the modified oligonucleotides of the present invention is modified. In certain embodiments, modified oligonucleotides include one or more unmodified sugar moiety.

### d. Certain Overall Lengths

In certain embodiments, the present invention provides modified oligonucleotides of any of a variety of ranges of lengths. In certain embodiments, the invention provides oligomeric compounds or oligonucleotides consisting of X to Y linked nucleosides, where X represents the fewest number of nucleosides in the range and Y represents the largest number of nucleosides in the range. In certain such embodiments, X and Y are each independently selected from 15, 16, 17, 18, 19, 20, 21, and 22; provided that X≤Y. For example, in certain embodiments, the invention provides modified oligonucleotides which comprise oligonucleotides consisting of 15 to 16, 15 to 17, 15 to 18, 15 to 19, 15 to 20, 15 to 21, 15 to 22, 16 to 17, 16 to 18, 16 to 19, 16 to 20, 16 to 21, 16 to 22, 17 to 18, 17 to 19, 17 to 20, 17 to 21, 17 to 22, 18 to 19, 18 to 20, 18 to 21, 18 to 22, 19 to 20, 19 to 21, 19 to 22, 20 to 21, 20 to 22, or 21 to 22 linked nucleosides. In embodiments where the number of nucleosides of an oligomeric compound or oligonucleotide is limited, whether to a range or to a specific number, the oligomeric compound or oligonucleotide may, nonetheless further comprise additional other substituents. For example, an oligonucleotide comprising 8-30 nucleosides excludes oligonucleotides having 31 nucleosides, but, unless otherwise indicated, such an oligonucleotide may further comprise, for example one or more conjugates, terminal groups, or other substituents. In certain embodiments, a modified oligonucleotides has any of the above lengths.

Further, where an oligonucleotide is described by an overall length range and by regions having specified lengths, and where the sum of specified lengths of the regions is less than the upper limit of the overall length range, the oligonucleotide may have additional nucleosides, beyond those of the specified regions, provided that the total number of nucleosides does not exceed the upper limit of the overall length range.

### e. Certain Oligonucleotides

In certain embodiments, oligonucleotides of the present invention are characterized by their modification motif and overall length. In certain embodiments, such parameters are each independent of one another.

### I. Certain Oligomeric Compounds

In certain embodiments, the invention provides oligomeric compounds, which consist of a modified oligonucleotide and optionally one or more conjugate groups and/or terminal groups. Conjugate groups consist of one or more conjugate moiety and a conjugate linker which links the conjugate moiety to the oligonucleotide. Conjugate groups may be attached to either or both ends of an oligonucleotide and/or at any internal position. In certain embodiments, conjugate groups are attached to the 2'-position of a nucleoside of a modified oligonucleotide. In certain embodiments, conjugate groups that are attached to either or both ends of an oligonucleotide are terminal groups. In certain such embodiments, conjugate groups or terminal groups are attached at the 3' and/or 5'-end of oligonucleotides. In certain such embodiments, conjugate groups (or terminal groups) are attached at the 3'-end of oligonucleotides. In certain embodiments, conjugate groups are attached near the 3'-end of oligonucleotides. In certain embodiments, conjugate groups (or terminal groups) are attached at the 5'-end of oligonucleotides. In certain embodiments, conjugate groups are attached near the 5'-end of oligonucleotides.

Examples of terminal groups include but are not limited to conjugate groups, capping groups, phosphate moieties, protecting groups, abasic nucleosides, modified or unmodified nucleosides, and two or more nucleosides that are independently modified or unmodified.

In certain embodiments, antisense compounds are provided wherein the 5'-terminal group comprises a 5'-terminal stabilized phosphate. A "5'-terminal stabilized phosphate" is a 5'-terminal phosphate group having one or more modificaitons that increase nuclease stability relative to a 5'-phosphate.

In certain embodiments, antisense compounds are provided wherein the 5'-terminal group has Formula IIe: 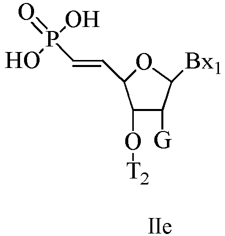 wherein:
Bx is uracil, thymine, cytosine, 5-methyl cytosine, adenine or guanine;
T2 is a phosphorothioate internucleoside linking group linking the compound of Formula IIe to the oligomeric compound; and
G is halogen, OCH3, OCF3, OCH2CH3, OCH2CF3, OCH2-CH=CH2, O(CH2)2-OCH3, O(CH2)2-O(CH2)2-N(CH3)2, OCH2C(=O)-N(H)CH3, OCH2C(=O)-N(H)-(CH2)2-N(CH3)2 or OCH2-N(H)-C(=NH)NH2.

In certain embodiments, antisense compounds are provided wherein said 5'-terminal compound has Formula IIe wherein G is F, OCH3 or O(CH2)2-OCH3.

In certain embodiments, the 5'-terminal group is a 5'-terminal stabilized phosphate comprising a vinyl phosphonate represented by Formula IIe above.

### f. Certain Conjugate Groups

In certain embodiments, the oligonucleotides or oligomeric compounds as provided herein are modified by covalent attachment of one or more conjugate groups. In general, conjugate groups modify one or more properties of the attached oligonucleotide or oligomeric compound including but not limited to pharmacodynamics, pharmacokinetics, stability, binding, absorption, cellular distribution, cellular uptake, charge and clearance. As used herein, "conjugate group" means a radical group comprising a group of atoms that are attached to an oligonucleotide or oligomeric compound. In general, conjugate groups modify one or more properties of the compound to which they are attached, including, but not limited to pharmacodynamic, pharmacokinetic, binding, absorption, cellular distribution, cellular uptake, charge and/or clearance properties. Conjugate groups are routinely used in the chemical arts and can include a conjugate linker that covalently links the conjugate group to an oligonucleotide or oligomeric compound. In certain embodiments, conjugate groups include a cleavable moiety that covalently links the conjugate group to an oligonucleotide or oligomeric compound. In certain embodiments, conjugate groups include a conjugate linker and a cleavable moiety to covalently link the conjugate group to an oligonucleotide or oligomeric compound. In certain embodiments, a conjugate group has the general formula: 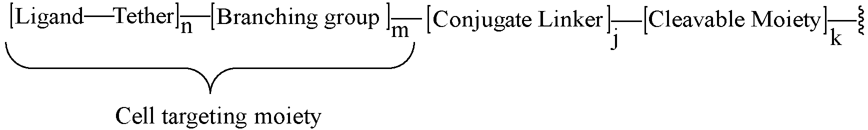 wherein n is from 1 to about 3, m is 0 when n is 1 or m is 1 when n is 2 or 3, j is 1 or 0, k is 1 or 0 and the sum of j and k is at least one.

In certain embodiments, n is 1, j is 1 and k is 0. In certain embodiments, n is 1, j is 0 and k is 1. In certain embodiments, n is 1, j is 1 and k is 1. In certain embodiments, n is 2, j is 1 and k is 0. In certain embodiments, n is 2, j is 0 and k is 1. In certain embodiments, n is 2, j is 1 and k is 1. In certain embodiments, n is 3, j is 1 and k is 0. In certain embodiments, n is 3, j is 0 and k is 1. In certain embodiments, n is 3, j is 1 and k is 1.

Conjugate groups are shown herein as radicals, providing a bond for forming covalent attachment to an oligomeric compound such as an oligonucleotide. In certain embodiments, the point of attachment on the oligomeric compound is at the 3'-terminal nucleoside or modified nucleoside. In certain embodiments, the point of attachment on the oligomeric compound is the 3'-oxygen atom of the 3'-hydroxyl group of the 3' terminal nucleoside or modified nucleoside. In certain embodiments, the point of attachment on the oligomeric compound is at the 5'-terminal nucleoside or modified nucleoside. In certain embodiments the point of attachment on the oligomeric compound is the 5'-oxygen atom of the 5'-hydroxyl group of the 5'-terminal nucleoside or modified nucleoside. In certain embodiments, the point of attachment on the oligomeric compound is at any reactive site on a nucleoside, a modified nucleoside or an internucleoside linkage.

As used herein, "cleavable moiety" and "cleavable bond" mean a cleavable bond or group of atoms that is capable of being split or cleaved under certain physiological conditions. In certain embodiments, a cleavable moiety is a cleavable bond. In certain embodiments, a cleavable moiety comprises a cleavable bond. In certain embodiments, a cleavable moiety is a group of atoms. In certain embodiments, a cleavable moiety is selectively cleaved inside a cell or sub-cellular compartment, such as a lysosome. In certain embodiments, a cleavable moiety is selectively cleaved by endogenous enzymes, such as nucleases. In certain embodiments, a cleavable moiety comprises a group of atoms having one, two, three, four, or more than four cleavable bonds.

In certain embodiments, conjugate groups comprise a cleavable moiety. In certain such embodiments, the cleavable moiety covalently attaches the oligomeric compound to the conjugate linker. In certain such embodiments, the cleavable moiety covalently attaches the oligomeric compound to the cell-targeting moiety.

In certain embodiments, a cleavable bond is selected from among: an amide, a polyamide, an ester, an ether, one or both esters of a phosphodiester, a phosphate ester, a carbamate, a di-sulfide, or a peptide. In certain embodiments, a cleavable bond is one of the esters of a phosphodiester. In certain embodiments, a cleavable bond is one or both esters of a phosphodiester. In certain embodiments, the cleavable moiety is a phosphodiester linkage between an oligomeric compound and the remainder of the conjugate group. In certain embodiments, the cleavable moiety comprises a phosphodiester linkage that is located between an oligomeric compound and the remainder of the conjugate group. In certain embodiments, the cleavable moiety comprises a phosphate or phosphodiester. In certain embodiments, the cleavable moiety is attached to the conjugate linker by either a phosphodiester or a phosphorothioate linkage. In certain embodiments, the cleavable moiety is attached to the conjugate linker by a phosphodiester linkage. In certain embodiments, the conjugate group does not include a cleavable moiety.

In certain embodiments, the cleavable moiety is a cleavable nucleoside or a modified nucleoside. In certain embodiments, the nucleoside or modified nucleoside comprises an optionally protected heterocyclic base selected from a purine, substituted purine, pyrimidine or substituted pyrimidine. In certain embodiments, the cleavable moiety is a nucleoside selected from uracil, thymine, cytosine, 4-N-benzoylcytosine, 5-methylcytosine, 4-N-benzoyl-5-methylcytosine, adenine, 6-N-benzoyladenine, guanine and 2-N-isobutyrylguanine.

In certain embodiments, the cleavable moiety is 2'-deoxy nucleoside that is attached to either the 3' or 5'-terminal nucleoside of an oligomeric compound by a phosphodiester linkage and covalently attached to the remainder of the conjugate group by a phosphodiester or phosphorothioate linkage. In certain embodiments, the cleavable moiety is 2'-deoxy adenosine that is attached to either the 3' or 5'-terminal nucleoside of an oligomeric compound by a phosphodiester linkage and covalently attached to the remainder of the conjugate group by a phosphodiester or phosphorothioate linkage. In certain embodiments, the cleavable moiety is 2'-deoxy adenosine that is attached to the 3'-oxygen atom of the 3'-hydroxyl group of the 3'-terminal nucleoside or modified nucleoside by a phosphodiester linkage. In certain embodiments, the cleavable moiety is 2'-deoxy adenosine that is attached to the 5'-oxygen atom of the 5'-hydroxyl group of the 5'-terminal nucleoside or modified nucleoside by a phosphodiester linkage. In certain embodiments, the cleavable moiety is attached to a 2'-position of a nucleoside or modified nucleoside of an oligomeric compound.

As used herein, "conjugate linker" in the context of a conjugate group means a portion of a conjugate group comprising any atom or group of atoms that covalently link the cell-targeting moiety to the oligomeric compound either directly or through the cleavable moiety. In certain embodiments, the conjugate linker comprises groups selected from alkyl, amino, oxo, amide, disulfide, polyethylene glycol, ether, thioether (-S-) and hydroxylamino (-O-N(H)-). In certain embodiments, the conjugate linker comprises groups selected from alkyl, amino, oxo, amide and ether groups. In certain embodiments, the conjugate linker comprises groups selected from alkyl and amide groups. In certain embodiments, the conjugate linker comprises groups selected from alkyl and ether groups. In certain embodiments, the conjugate linker comprises at least one phosphorus linking group. In certain embodiments, the conjugate linker comprises at least one phosphodiester group. In certain embodiments, the conjugate linker includes at least one neutral linking group.

In certain embodiments, the conjugate linker is covalently attached to the oligomeric compound. In certain embodiments, the conjugate linker is covalently attached to the oligomeric compound and the branching group. In certain embodiments, the conjugate linker is covalently attached to the oligomeric compound and a tethered ligand. In certain embodiments, the conjugate linker is covalently attached to the cleavable moiety. In certain embodiments, the conjugate linker is covalently attached to the cleavable moiety and the branching group. In certain embodiments, the conjugate linker is covalently attached to the cleavable moiety and a tethered ligand. In certain embodiments, the conjugate linker includes one or more cleavable bonds. In certain embodiments, the conjugate group does not include a conjugate linker.

As used herein, "branching group" means a group of atoms having at least 3 positions that are capable of forming covalent linkages to two or more tether-ligands and the remainder of the conjugate group. In general a branching group provides a plurality of reactive sites for connecting tethered ligands to the oligomeric compound through the conjugate linker and/or the cleavable moiety. In certain embodiments, the branching group comprises groups selected from alkyl, amino, oxo, amide, disulfide, polyethylene glycol, ether, thioether and hydroxylamino groups. In certain embodiments, the branching group comprises a branched aliphatic group comprising groups selected from alkyl, amino, oxo, amide, disulfide, polyethylene glycol, ether, thioether and hydroxylamino groups. In certain such embodiments, the branched aliphatic group comprises groups selected from alkyl, amino, oxo, amide and ether groups. In certain such embodiments, the branched aliphatic group comprises groups selected from alkyl, amino and ether groups. In certain such embodiments, the branched aliphatic group comprises groups selected from alkyl and ether groups. In certain embodiments, the branching group comprises a mono or polycyclic ring system.

In certain embodiments, the branching group is covalently attached to the conjugate linker. In certain embodiments, the branching group is covalently attached to the cleavable moiety. In certain embodiments, the branching group is covalently attached to the conjugate linker and each of the tethered ligands. In certain embodiments, the branching group comprises one or more cleavable bond. In certain embodiments, the conjugate group does not include a branching group.

In certain embodiments, conjugate groups as provided herein include a cell-targeting moiety that has at least one tethered ligand. In certain embodiments, the cell-targeting moiety comprises two tethered ligands covalently attached to a branching group. In certain embodiments, the cell-targeting moiety comprises three tethered ligands covalently attached to a branching group.

As used herein, "tether" means a group of atoms that connect a ligand to the remainder of the conjugate group. In certain embodiments, each tether is a linear aliphatic group comprising one or more groups selected from alkyl, substituted alkyl, ether, thioether, disulfide, amino, oxo, amide, phosphodiester and polyethylene glycol groups in any combination. In certain embodiments, each tether is a linear aliphatic group comprising one or more groups selected from alkyl, ether, thioether, disulfide, amino, oxo, amide and polyethylene glycol groups in any combination. In certain embodiments, each tether is a linear aliphatic group comprising one or more groups selected from alkyl, substituted alkyl, phosphodiester, ether and amino, oxo, amide groups in any combination. In certain embodiments, each tether is a linear aliphatic group comprising one or more groups selected from alkyl, ether and amino, oxo, amide groups in any combination. In certain embodiments, each tether is a linear aliphatic group comprising one or more groups selected from alkyl, amino and oxo groups in any combination. In certain embodiments, each tether is a linear aliphatic group comprising one or more groups selected from alkyl and oxo groups in any combination. In certain embodiments, each tether is a linear aliphatic group comprising one or more groups selected from alkyl and phosphodiester in any combination. In certain embodiments, each tether comprises at least one phosphorus linking group or neutral linking group.

In certain embodiments, tethers include one or more cleavable bond. In certain embodiments, each tethered ligand is attached to a branching group. In certain embodiments, each tethered ligand is attached to a branching group through an amide group. In certain embodiments, each tethered ligand is attached to a branching group through an ether group. In certain embodiments, each tethered ligand is attached to a branching group through a phosphorus linking group or neutral linking group. In certain embodiments, each tethered ligand is attached to a branching group through a phosphodiester group. In certain embodiments, each tether is attached to a ligand through either an amide or an ether group. In certain embodiments, each tether is attached to a ligand through an ether group.

In certain embodiments, each tether comprises from about 8 to about 20 atoms in chain length between the ligand and the branching group. In certain embodiments, each tether comprises from about 10 to about 18 atoms in chain length between the ligand and the branching group. In certain embodiments, each tether comprises about 13 atoms in chain length.

In certain embodiments, ligands are provided wherein each ligand is covalently attached to the remainder of the conjugate group through a tether. In certain embodiments, each ligand is selected to have an affinity for at least one type of receptor on a target cell. In certain embodiments, ligands are selected that have an affinity for at least one type of receptor on the surface of a mammalian liver cell. In certain embodiments, ligands are selected that have an affinity for the hepatic asialoglycoprotein receptor (ASGP-R). In certain embodiments, each ligand is a carbohydrate. In certain embodiments, each ligand is, independently selected from galactose, N-acetyl galactoseamine, mannose, glucose, glucosamone and fucose. In certain embodiments, each ligand is N-acetyl galactoseamine (GalNAc). In certain embodiments, the targeting moiety comprises 1 to 3 ligands. In certain embodiments, the targeting moiety comprises 3 ligands. In certain embodiments, the targeting moiety comprises 2 ligands. In certain embodiments, the targeting moiety comprises 1 ligand. In certain embodiments, the targeting moiety comprises 3 N-acetyl galactoseamine ligands. In certain embodiments, the targeting moiety comprises 2 N-acetyl galactoseamine ligands. In certain embodiments, the targeting moiety comprises 1 N-acetyl galactoseamine ligand.

In certain embodiments, each ligand is a carbohydrate, carbohydrate derivative, modified carbohydrate, multivalent carbohydrate cluster, polysaccharide, modified polysaccharide, or polysaccharide derivative. In certain embodiments, each ligand is an amino sugar or a thio sugar. For example, amino sugars may be selected from any number of compounds known in the art, for example glucosamine, sialic acid, α-D-galactosamine, N-Acetylgalactosamine, 2-acetamido-2-deoxy-D-galactopyranose (GalNAc), 2-Amino-3-*O*-[(*R*)-1-carboxyethyl]-2-deoxy-β-D-glucopyranose (β-muramic acid), 2-Deoxy-2-methylamino-L-glucopyranose, 4,6-Dideoxy-4-formamido-2,3-di-*O*-methyl-D-mannopyranose, 2-Deoxy-2-sulfoamino-D-glucopyranose and *N*-sulfo-D-glucosamine, and *N*-Glycoloyl-α-neuraminic acid. For example, thio sugars may be selected from the group consisting of 5-Thio-β-D-glucopyranose, Methyl 2,3,4-tri-*O*-acetyl-1-thio-6-*O*-trityl-α-D-glucopyranoside, 4-Thio-β-D-galactopyranose, and ethyl 3,4,6,7-tetra-*O*-acetyl-2-deoxy-1,5-dithio-α-D-*gluco*-heptopyranoside.

In certain embodiments, conjugate groups as provided herein comprise a carbohydrate cluster. As used herein, "carbohydrate cluster" means a portion of a conjugate group wherein two or more carbohydrate residues are attached to a branching group through tether groups. (*see,* e.g., Maier et al., "Synthesis of Antisense Oligonucleotides Conjugated to a Multivalent Carbohydrate Cluster for Cellular Targeting," Bioconjugate Chemistry, 2003, (14): 18-29, or Rensen et al., "Design and Synthesis of Novel N-Acetylgalactosamine-Terminated Glycolipids for Targeting of Lipoproteins to the Hepatic Asiaglycoprotein Receptor," J. Med. Chem. 2004, (47): 5798-5808, for examples of carbohydrate conjugate clusters).

As used herein, "modified carbohydrate" means any carbohydrate having one or more chemical modifications relative to naturally occurring carbohydrates.

As used herein, "carbohydrate derivative" means any compound which may be synthesized using a carbohydrate as a starting material or intermediate.

As used herein, "carbohydrate" means a naturally occurring carbohydrate, a modified carbohydrate, or a carbohydrate derivative.

In certain embodiments, conjugate groups are provided wherein the cell-targeting moiety has the formula:

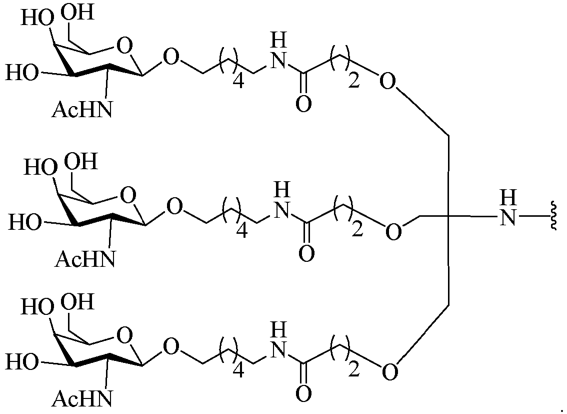

In certain embodiments, conjugate groups are provided wherein the cell-targeting moiety has the formula:

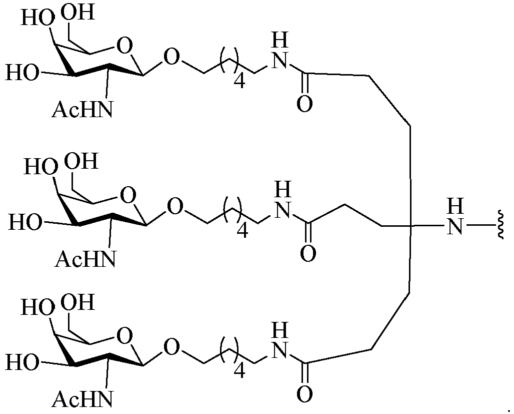

In certain embodiments, conjugate groups are provided wherein the cell-targeting moiety has the formula:

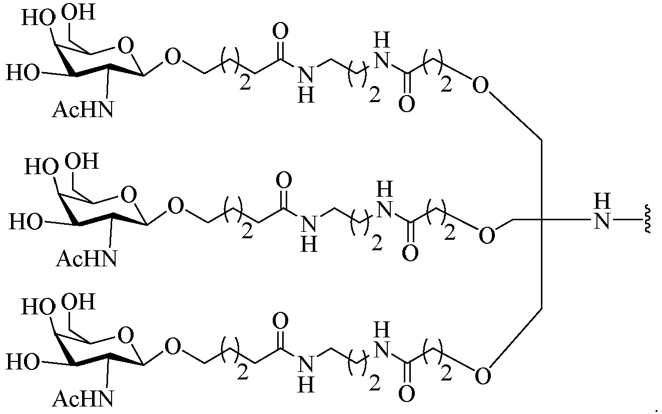

In certain embodiments, conjugate groups have the formula:

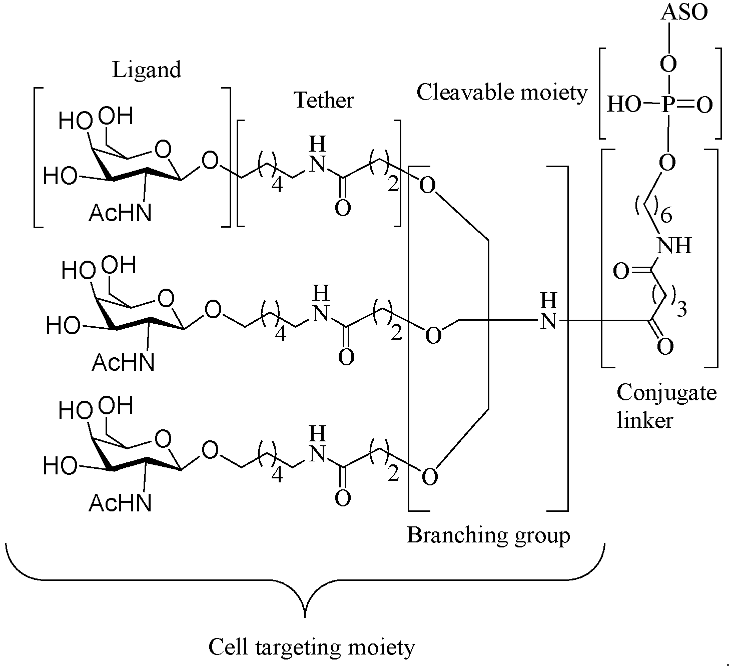

Representative United States patents, United States patent application publications, and international patent application publications that teach the preparation of certain of the above noted conjugate groups, conjugated oligomeric compounds such as antisense compounds comprising a conjugate group, tethers, conjugate linkers, branching groups, ligands, cleavable moieties as well as other modifications include without limitation, US 5,994,517, US 6,300,319, US 6,660,720, US 6,906,182, US 7,262,177, US 7,491,805, US 8,106,022, US 7,723,509, US 2006/0148740, US 2011/0123520, WO 2013/033230 and WO 2012/037254.

Representative publications that teach the preparation of certain of the above noted conjugate groups, conjugated oligomeric compounds such as antisense compounds comprising a conjugate group, tethers, conjugate linkers, branching groups, ligands, cleavable moieties as well as other modifications include without limitation, BIESSEN et al., "The Cholesterol Derivative of a Triantennary Galactoside with High Affinity for the Hepatic Asialoglycoprotein Receptor: a Potent Cholesterol Lowering Agent" J. Med. Chem. (1995) 38:1846-1852, BIESSEN et al., "Synthesis of Cluster Galactosides with High Affinity for the Hepatic Asialoglycoprotein Receptor" J. Med. Chem. (1995) 38:1538-1546, LEE et al., "New and more efficient multivalent glyco-ligands for asialoglycoprotein receptor of mammalian hepatocytes" Bioorganic & Medicinal Chemistry (2011) 19:2494-2500, RENSEN et al., "Determination of the Upper Size Limit for Uptake and Processing of Ligands by the Asialoglycoprotein Receptor on Hepatocytes in Vitro and in Vivo" J. Biol. Chem. (2001) 276(40):37577-37584, RENSEN et al., "Design and Synthesis of Novel N-Acetylgalactosamine-Terminated Glycolipids for Targeting of Lipoproteins to the Hepatic Asialoglycoprotein Receptor" J. Med. Chem. (2004) 47:5798-5808, SLIEDREGT et al., "Design and Synthesis of Novel Amphiphilic Dendritic Galactosides for Selective Targeting of Liposomes to the Hepatic Asialoglycoprotein Receptor" J. Med. Chem. (1999) 42:609-618, and Valentijn et al., "Solid-phase synthesis of lysine-based cluster galactosides with high affinity for the Asialoglycoprotein Receptor" Tetrahedron, 1997, 53(2), 759-770.

In certain embodiments, conjugate groups include without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. Certain conjugate groups have been described previously, for example: cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

In certain embodiments, a conjugate group comprises an active drug substance, for example, aspirin, warfarin, phenylbutazone, ibuprofen, suprofen, fen-bufen, ketoprofen, (*S*)-(+)-pranoprofen, carprofen, dansylsarcosine, 2,3,5-triiodobenzoic acid, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

Some nonlimiting examples of conjugate linkers include pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other connugate linkers include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

Conjugate groups may be attached to either or both ends of an oligonucleotide (terminal conjugate groups) and/or at any internal position.

In certain embodiments, conjugate groups are at the 3'-end of an oligonucleotide of an oligomeric compound. In certain embodiments, conjugate groups are near the 3'-end. In certain embodiments, conjugates are attached at the 3'end of an oligomeric compound, but before one or more terminal group nucleosides. In certain embodiments, conjugate groups are placed within a terminal group.

### B. Antisense Compounds

In the present disclosure, modified oligonucleotides provided herein are antisense compounds. Such antisense compounds are capable of hybridizing to a target nucleic acid, resulting in at least one antisense activity. In certain embodiments, antisense compounds specifically hybridize to one or more target nucleic acid. In certain embodiments, a specifically hybridizing antisense compound has a nucleobase sequence comprising a region having sufficient complementarity to a target nucleic acid to allow hybridization and result in antisense activity and insufficient complementarity to any non-target so as to avoid non-specific hybridization to any non-target nucleic acid sequences under conditions in which specific hybridization is desired (e.g., under physiological conditions for *in vivo* or therapeutic uses, and under conditions in which assays are performed in the case of *in vitro* assays).

In certain embodiments, the present invention provides antisense compounds comprising oligonucleotides that are fully complementary to the target nucleic acid over the entire length of the oligonucleotide.

### a. Certain Antisense Compounds

In certain embodiments, a modified oligonucleotide described herein has a nucleobase sequence comprising at least 14 contiguous nucleobases of any of the nucleobase sequences of SEQ ID NOs: 3, 4, 5, 6, 7, 8, 9, 10, 11, 12, 13, 14, 15, 16, 26, 27, 28, 29, 30, 31, 32, 33, 34, 35, 36, 37, 38, 39, 40, 45, 54, 55, 56, 57, 58, 59, 60, 61, 62, 63, 64, 65, 66, 67, 68, 69, 70, 71, 72, 73, 74, 75, 76, or 77.

In certain embodiments, a modified oligonucleotide described herein has a nucleobase sequence comprising at least 14 contiguous nucleobases of any of the nucleobase sequences of SEQ ID NOs: 17, 18, 19, 41, or 32.

In certain embodiments, a modified oligonucleotide described herein has a nucleobase sequence comprising at least 14 contiguous nucleobases of any of the nucleobase sequences of SEQ ID NO: 21.

In certain embodiments, a modified oligonucleotide described herein has a nucleobase sequence comprising at least 14 contiguous nucleobases of any of the nucleobase sequences of SEQ ID NOs: 25 or 44.

In certain embodiments, a modified oligonucleotide described herein has a nucleobase sequence comprising at least 14 contiguous nucleobases of any of the nucleobase sequences of SEQ ID NO: 23.

In certain embodiments, a modified oligonucleotide described herein has a nucleobase sequence comprising at least 14 contiguous nucleobases of any of the nucleobase sequences of SEQ ID NOs: 79, 80, 84, 85, 86, 87, 88, 89, 90, 92, or 93.

In certain embodiments, a modified oligonucleotide described herein has a nucleobase sequence comprising at least 14 contiguous nucleobases of any of the nucleobase sequences of SEQ ID NOs: 82 or 83.

In certain embodiments, a modified oligonucleotide described herein has a nucleobase sequence comprising at least 14 contiguous nucleobases of any of the nucleobase sequences of SEQ ID NO: 95.

In certain embodiments, a modified oligonucleotide described herein has a nucleobase sequence comprising at least 14 contiguous nucleobases of any of the nucleobase sequences of SEQ ID NO: 97.

In certain embodiments, a modified oligonucleotide described herein has a nucleobase sequence comprising at least 14 contiguous nucleobases of any of the nucleobase sequences of SEQ ID NOs: 99, 101, 102, 103, or 104.

In certain embodiments, a modified oligonucleotide described herein has a nucleobase sequence consisting of the nucleobase sequence of any of SEQ ID NOs: 3, 4, 5, 6, 7, 8, 9, 10, 11, 12, 13, 14, 15, 16, 26, 27, 28, 29, 30, 31, 32, 33, 38, 39, 40, 45, 54, 55, 56, 57, 58, 59, 60, 61, 62, 63, 64, 65, 66, 67, 68, 69, 70, 71, 72, 73, 74, 75, 76, or 77.

In certain embodiments, a modified oligonucleotide described herein has a nucleobase sequence consisting of the nucleobase sequence of any of SEQ ID NOs: 17, 18, 19, 41, or 32.

In certain embodiments, a modified oligonucleotide described herein has a nucleobase sequence consisting of the nucleobase sequence of SEQ ID NO: 21.

In certain embodiments, a modified oligonucleotide described herein has a nucleobase sequence consisting of the nucleobase sequence of any of SEQ ID NOs: 25 or 44.

In certain embodiments, a modified oligonucleotide described herein has a nucleobase sequence consisting of the nucleobase sequence of SEQ ID NO: 23.

In certain embodiments, a modified oligonucleotide described herein has a nucleobase sequence consisting of the nucleobase sequence of any of SEQ ID NOs: 79, 80, 90, 92, or 93.

In certain embodiments, a modified oligonucleotide described herein has a nucleobase sequence consisting of the nucleobase sequence of any of SEQ ID NOs: 82 or 83.

In certain embodiments, a modified oligonucleotide described herein has a nucleobase sequence consisting of the nucleobase sequence of SEQ ID NO: 95.

In certain embodiments, a modified oligonucleotide described herein has a nucleobase sequence consisting of the nucleobase sequence of SEQ ID NO: 97.

In certain embodiments, a modified oligonucleotide described herein has a nucleobase sequence consisting of the nucleobase sequence of any of SEQ ID NOs: 99, 101, 102, 103, or 104.

Certain embodiments provide methods for increasing the expression of RNAse H1 by contacting a cell with a modified oligonucleotide described herein has a nucleobase sequence comprising at least 14 contiguous nucleobases of any of the nucleobase sequences of SEQ ID NOs: 3, 4, 5, 6, 7, 8, 9, 10, 11, 12, 13, 14, 15, 16, 26, 27, 28, 29, 30, 31, 32, 33, 34, 35, 36, 37, 38, 39, 40, 45, 54, 55, 56, 57, 58, 59, 60, 61, 62, 63, 64, 65, 66, 67, 68, 69, 70, 71, 72, 73, 74, 75, 76, or 77.

Certain embodiments provide methods for increasing the expression of LRPPRC by contacting a cell with a modified oligonucleotide described herein has a nucleobase sequence comprising at least 14 contiguous nucleobases of any of the nucleobase sequences of SEQ ID NOs: 17, 18, 19, 41, or 32.

Certain embodiments provide methods for increasing the expression of SFXN3 by contacting a cell with a modified oligonucleotide described herein has a nucleobase sequence comprising at least 14 contiguous nucleobases of the nucleobase sequences of SEQ ID NO: 21.

Certain embodiments provide methods for increasing the expression of THPO by contacting a cell with a modified oligonucleotide described herein has a nucleobase sequence comprising at least 14 contiguous nucleobases of any of the nucleobase sequences of SEQ ID NOs: 25 or 44.

Certain embodiments provide methods for increasing the expression of MRPL11 by contacting a cell with a modified oligonucleotide described herein has a nucleobase sequence comprising at least 14 contiguous nucleobases of the nucleobase sequences of SEQ ID NO: 23.

Certain embodiments provide methods for increasing the expression of ACP1 by contacting a cell with a modified oligonucleotide described herein has a nucleobase sequence comprising at least 14 contiguous nucleobases of any of the nucleobase sequences of SEQ ID NOs: 79, 80, 84, 85, 86, 87, 88, 89, 90, 92, or 93.

Certain embodiments provide methods for increasing the expression of CFTR by contacting a cell with a modified oligonucleotide described herein has a nucleobase sequence comprising at least 14 contiguous nucleobases of any of the nucleobase sequences of SEQ ID NOs: 82 or 83.

Certain embodiments provide methods for increasing the expression of ARF1 by contacting a cell with a modified oligonucleotide described herein has a nucleobase sequence comprising at least 14 contiguous nucleobases of the nucleobase sequences of SEQ ID NO: 95.

Certain embodiments provide methods for increasing the expression of USP16 by contacting a cell with a modified oligonucleotide described herein has a nucleobase sequence comprising at least 14 contiguous nucleobases of the nucleobase sequences of SEQ ID NO: 97.

Certain embodiments provide methods for increasing the expression of LDLr by contacting a cell with a modified oligonucleotide described herein has a nucleobase sequence comprising at least 14 contiguous nucleobases of any of the nucleobase sequences of SEQ ID NOs: 99, 101, 102, 103, or 104.

### b. Certain Antisense Activities and Mechanisms

The 5'-UTR has emerged as playing an important role in the regulation of translation of many transcripts. The 5'-UTR contains translation suppression elements (TSEs), which serve to suppress translation of a target protein. In the claims, translation suppression elements comprise uORFs. Disruption of a TSE will decrease the TSE's suppression of translation of a given transcript and will therefore result in an increase in translation of a target protein. In the present disclosure, antisense compounds are used to disrupt a uORFand to increase translation of a target protein.

Upstream open reading frames have emerged as an important mechanism by which translation is regulated. Approximately 50 % of human transcripts have uORFs and most appear to be functional. When functional, uORFs typically reduce the translation of a polypeptide or protein from the downstream pORF. Characteristics of uORFs such as the strength of Kozak sequence and\or the distance from the 5'cap, affect the effectiveness of each uORF in reducing the translation of the downstream protein. Other factors, such as the secondary structure of the uORF or the number of uORFs per transcript also affect the effectiveness of each uORF in reducing the translation of the downstream protein. The present disclosure provides uORF inhibitors (e.g. antisense compounds) that may serve to block the initiation of translation from a uORF.

The invention provides a method of increasing the amount of a target protein in an *in vitro* cell, comprising contacting the cell with an antisense compound comprising a modified oligonucleotide, wherein the target protein is encoded by a target transcript comprising at least one translation suppression element, wherein the translation suppression element comprises a uORF, wherein the uORF comprises an AUG codon, and thereby increasing expression of the target protein in the cell, wherein the modified oligonucleotide: is complementary to the target transcript at the uORF start site region; has a nucleobase sequence that is 100% complementary to the target transcript; consists of 15 to 22 linked nucleosides; and comprises at least 15 modified nucleosides, each modified nucleoside independently comprising a modified sugar moiety, wherein the antisense compound does not recruit RNase H.

The invention also provides an antisense compound comprising a modified oligonucleotide for use in a method of treating a disease or condition associated with a deficiency of a target protein, wherein in said method the modified oligonucleotide increases the amount of the target protein in a human cell, wherein the target protein is encoded by a target transcript comprising at least one translation suppression element, wherein the translation suppression element comprises a uORF, wherein the uORF comprises an AUG codon, and thereby increasing expression of the target protein in the cell, wherein the modified oligonucleotide: is complementary to the target transcript at the uORF start site region; has a nucleobase sequence 100% complementary to the target transcript; consists of 15 to 22 linked nucleosides; and comprises at least 15 modified nucleosides, each modified nucleoside independently comprising a modified sugar moiety, wherein the antisense compound does not recruit RNase H.
Certain embodiments of the present invention block the initiation of translation from a uORF with a uORF inhibitor. In such embodiments, translation of the protein encoded by the downstream ORF (e.g., the pORF) is enhanced. The present disclosure provides uORF inhibitors (i.e. antisense compounds), wherein the antisense compounds are modified oligonucleotides that are not gapmers.

Upstream open reading frames can regulate translation of polypeptides or proteins encoded by pORFs by a variety of mechanisms. The polypeptide or protein encoded by the uORF may be translated, and the resulting uORF polypeptide may block translation of the polypeptide or protein encoded by the pORF on the same mRNA molecule (*cis* regulation) or on a separate mRNA molecule (*trans* regulation). In another example of *cis* regulation, the ribosomal subunits may dissociate from the mRNA following translation of the uORF polypeptide, thus failing to recognize and translate the pORF polypeptide or protein. Alternatively, the uORF stop codon may be recognized as a premature stop codon and initiate nonsense mediated decay. The extent to which a uORF suppresses translation of a pORF polypeptide or protein depends on how often it is recognized by the translational machinery, which is in turn affected by many factors, including the strength of the associated Kozak sequence, the number of uORFs in the 5'-UTR, the position of the stop codon, and the secondary structure of the uORF. uORF inhibitors may be employed to disrupt, alter, or exploit any of these mechanisms. In certain embodiments, uORF inhibitors are antisense compounds, and antisense compounds may be employed to disrupt, alter, or exploit any of these mechanisms. In certain embodiments, uORF inhibitors are antisense compounds, and antisense compounds may be employed to disrupt, alter, or exploit any of these mechanisms to increase expression of a target protein in a cell.

In certain embodiments, a uORF inhibitor (i.e. an antisense compound) disrupts one or more of the factors that contribute to the recognition of a uORF start site by a ribosomal subunit. In certain embodiments, a uORF inhibitor (i.e. an antisense compound) may prevent or decrease one or more of the uORF mediated translational suppressors of pORF polypeptide or protein translation discussed above. For example, in certain embodiments, a uORF inhibitor (i.e. an antisense compound) will disrupt one or more elements of a Kozak sequence, and thereby inhibit recognition of a uORF start site by a ribosomal subunit. For example, in certain embodiments, an antisense compound complementary to a portion of a uORF start site may prevent one or more ribosomal subunits from recognizing a Kozak consensus sequence. In certain embodiments, an antisense compound complementary to a portion of a uORF start site may prevent one or more ribosomal subunits from recognizing a Kozak consensus sequence and would therefore cause the ribosome to pass by the uORF start site and initiate translation at a downstream start site (e.g. the pORF start site). A uORF inhibitor (i.e. an antisense compound) would thereby increase the amount or activity of the target protein encoded by the pORF.

In certain embodiments, an antisense compound complementary to a portion of a target transcript upstream of a uORF start site may prevent one or more ribosomal subunits from recognizing a Kozak consensus sequence. In certain embodiments, an antisense compound complementary to a portion of a target transcript upstream of a uORF start site may prevent one or more ribosomal subunits from recognizing the uORF start site. In certain embodiments, an antisense compound complementary to a portion of a target transcript 10 nucleobases upstream of a uORF start site may prevent one or more ribosomal subunits from recognizing a Kozak consensus sequence. In certain embodiments, an antisense compound complementary to a portion of a target transcript 20 nucleobases upstream of a uORF start site may prevent one or more ribosomal subunits from recognizing a Kozak consensus sequence. Therefore, in certain embodiments, an antisense compound complementary to a portion of a target transcript upstream of a uORF start site would increase the amount or activity the target protein encoded by the pORF.

In certain embodiments, an antisense compound complementary to a portion of a target transcript downstream of a uORF start site may prevent one or more ribosomal subunits from recognizing a Kozak consensus sequence. In certain embodiments, an antisense compound complementary to a portion of a target transcript downstream of a uORF start site may prevent one or more ribosomal subunits from recognizing the uORF start site. Therefore, in certain embodiments, an antisense compound complementary to a portion of a target transcript downstream of a uORF start site would increase the amount or activity the target protein encoded by the pORF.

In certain embodiments, disruption one or more of the factors that contribute to the recognition of a uORF start site by a ribosomal subunit by a uORF inhibitor (i.e. an antisense compound) increases expression of a downstream pORF. For example, a uORF inhibitor (i.e. an antisense compound) may prevent the dissociation of ribosomal submits after uORF polypeptide translation and thereby increase expression of a downstream pORF. For example, a uORF inhibitor (i.e. an antisense compound) may prevent the recognition of the uORF termination codon as premature and prevent nonsense-mediated decay of the transcript comprising the pORF.

The ribosomal subunits recognize a uORF start site and after translation of all or part of the uORF polypeptide, the ribosomal subunits dissociate from the transcript before recognition of the pORF start site. The ribosomal subunits are the 60s ribosomal subunit and/or the 40s ribosomal subunit. In certain embodiments, a uORF inhibitor (i.e. an antisense compound) may increase the amount of translation reinitiation at the pORF after uORF polypeptide translation. For example, in certain embodiments, a uORF inhibitor (i.e. an antisense compound) prevents the amount of the 60s ribosomal subunit and/or the 40s ribosomal subunit that dissociate from the transcript after translation of all or part of the uORF polypeptide and thereby increases translation of the pORF polypeptide or protein.

Upstream open reading frames are one type of translation suppression element. In addition to uORFs, translation can be regulated by other types of translation suppression elements (TSEs) in the 5'-UTR. Translation may be suppressed by structural elements in the 5'-UTR, such as stem-loops and hairpins. The extent to which a structural element suppresses translation may be correlated with the sequence and/or stability of the structural element. For example, the extent to which a structural element suppresses translation may increase as the distance between the 5'-cap of the target transcript and the structural element decreases. For another example, GC content and/or the number of consecutive GC nucleosides in the structural element may positively correlate with the extent to which translation is suppressed, due to increased stability of the structural element. Thus, transcripts with a 5'-UTR containing multiple stretches of at least 3 consecutive GC nucleosides may comprise at least one TSE. Transcripts with a 5'-UTR containing at least one stretch of at least 7 GC nucleosides may comprise at least one TSE.

TSEs that are structural elements may sterically block one or more ribosomal subunits from accessing the coding region. Without wishing to be bound by mechanism, TSE inhibitors may increase translation of a target protein by relieving such steric blockage.

A third type of TSE is a G quartet. The extent to which a G quartet suppresses translation may increase as the distance between the 5'-cap of the target transcript and the G quartet decrases.

A fourth type of TSE is a stem-loop structure. Transcripts having a stem-loop structure within the 5'-UTR suppress translation of the target protein. The extent to which a stem-loop suppresses translation may increase as the distance between the 5'-cap of the target transcript and the stem-loop decrases.

### c. Certain Compositions and Methods for Increasing Antisense Activity

Certain embodiments provide methods for increasing the activity of an antisense compound in a cell, comprising contacting the cell with a translation suppression element inhibitor and thereby increasing the activity of an antisense compound.

In the present disclosure, a uORF inhibitor (i.e. an antisense compound) as described herein increases the amount or activity of a target protein in a cell. In certain embodiments, the target protein plays a role in antisense activity. Therefore, in certain embodiments, increasing the amount or activity of a target protein may also increase the amount or activity of an antisense compound. For example, in certain embodiments, the target protein may play a role in subcellular localization of antisense compounds. In certain embodiments, the target protein may play a role in RNA binding. In certain embodiments, the target protein may play a role in nuclear transport. In certain embodiments, the target protein may play a role in membrane binding. In certain embodiments, the target protein may play a role in DNA binding. In certain embodiments, the target protein may play a role in nuclear import. In certain embodiments, the target protein is a heat shock protein.

In certain embodiments, a translation suppression element inhibitor is used to increase the amount or activity of a target protein that plays a role in antisense activity, thereby increasing the amount or activity of an antisense compound. In this manner, a translation suppression element inhibitor may be used to increase the activity of an antisense compound by contacting a cell with the translation suppression element inhibitor and then contacting the cell with an antisense compound. In certain embodiments, a uORF inhibitor is used to increase the amount or activity of a target protein that plays a role in antisense activity, thereby increasing the amount or activity of an antisense compound. In this manner, a uORF inhibitor may be used to increase the activity of an antisense compound by contacting a cell with the uORF inhibitor and then contacting the cell with an antisense compound.

For example, in certain embodiments, the target protein is RNase H. In certain embodiments, a uORF inhibitor targeted to the 5'-UTR of RNase H increases the amount or activity of RNase H protein in a cell, thereby increasing antisense activity in the cell. In certain embodiments, the target protein is La/SSB. In certain embodiments, a uORF inhibitor targeted to the 5'-UTR of La/SSB increases the amount or activity of La/SSB protein in a cell, thereby increasing antisense activity in the cell. In certain embodiments, the target protein is NPM1. In certain embodiments, a uORF inhibitor targeted to the 5'-UTR of NPM1 increases the amount or activity of NPM1 protein in a cell, thereby increasing antisense activity in the cell. In certain embodiments, the target protein is TCP1-alpha. In certain embodiments, a uORF inhibitor targeted to the 5'-UTR of TCP1-alpha increases the amount or activity of TCP1-alpha protein in a cell, thereby increasing antisense activity in the cell.

In certain embodiments, the target protein is TCP1-epsilon. In certain embodiments, a uORF inhibitor targeted to the 5'-UTR of TCP1-epsilon increases the amount or activity of TCP1- epsilon protein in a cell, thereby increasing antisense activity in the cell. In certain embodiments, the target protein is TCP1-beta. In certain embodiments, a uORF inhibitor targeted to the 5'-UTR of TCP1-beta increases the amount or activity of TCP1-beta protein in a cell, thereby increasing antisense activity in the cell. In certain embodiments, the target protein is HSP90-AA1. In certain embodiments, a uORF inhibitor targeted to the 5'-UTR of HSP90-AA1 increases the amount or activity of HSP90-AA1 protein in a cell, thereby increasing antisense activity in the cell. In certain embodiments, the target protein is HSP90-AB. In certain embodiments, a uORF inhibitor targeted to the 5'-UTR of HSP90-AB increases the amount or activity of HSP90-AB protein in a cell, thereby increasing antisense activity in the cell. In certain embodiments, the target protein is HSPA1L. In certain embodiments, a uORF inhibitor targeted to the 5'-UTR of HSPA1L increases the amount or activity of HSPA1L protein in a cell, thereby increasing antisense activity in the cell. In certain embodiments, the target protein is RAN. In certain embodiments, a uORF inhibitor targeted to the 5'-UTR of RAN increases the amount or activity of RAN protein in a cell, thereby increasing antisense activity in the cell.

In certain embodiments, the target protein is IMP9. In certain embodiments, a uORF inhibitor targeted to the 5'-UTR of IMP9 increases the amount or activity of IMP9 protein in a cell, thereby increasing antisense activity in the cell. In certain embodiments, the target protein is Annexin A2. In certain embodiments, a uORF inhibitor targeted to the 5'-UTR of Annexin A2 increases the amount or activity of Annexin A2 protein in a cell, thereby increasing antisense activity in the cell. In certain embodiments, the target protein is FTCD/58k. In certain embodiments, a uORF inhibitor targeted to the 5'-UTR of FTCD/58k increases the amount or activity of FTCD/58k protein in a cell, thereby increasing antisense activity in the cell.

In certain embodiments, the target protein is PC4/SUB1. In certain embodiments, a uORF inhibitor targeted to the 5'-UTR of PC4/SUB1 increases the amount or activity of PC4/SUB1 protein in a cell, thereby increasing antisense activity in the cell. In certain embodiments, the target protein is VARS. In certain embodiments, a uORF inhibitor targeted to the 5'-UTR of VARS increases the amount or activity of VARS protein in a cell, thereby increasing antisense activity in the cell. In certain embodiments, the target protein is DHX36. In certain embodiments, a uORF inhibitor targeted to the 5'-UTR of DHX36 increases the amount or activity of DHX36 protein in a cell, thereby increasing antisense activity in the cell.

In certain embodiments, the target protein is LDLr. Increasing expression of the LDLr protein decreases cholesteral levels.

In certain embodiments, the target protein is CFTR. Mutations in the CFTR gene lead to cystic fibrosis. In certain embodiments, increasing expression of the CFTR protein may ameliorate one or more symptoms of cystic fibrosis.

### C. Certain Target Genes

The present disclosure provides compounds and methods that may be used to increase expression of any target protein, provided there is at least one uORF on a transcript encoding the target protein. The present disclosure also provides compounds and methods that may be used to increase expression of any target protein, provided there is at least one TSE on a transcript encoding the target protein. In the present disclosure, the at least one TSE comprises a uORF. In certain embodiments, there are at least two TSEs on a transcript encoding a target protein. In certain embodiments, one of the at least two TSEs comprises a uORF. In certain embodiments, both of the at least two TSEs comprise a uORF. In certain embodiments, a deficiency of the target protein is associated with disease and so increasing the amount or activity of the protein is expected to ameliorate one or more symptoms of the disease or delay the onset of one or more symptoms of the disease. Table 1 and Table 2, below, list certain genes and associated diseases. In certain embodiments, a target transcript is encoded by a gene listed in Table 1 or Table 2. In certain embodiments, the associated disease or disorder in Table 1 or Table 2 is treated by use of an antisense compound of the present invention targeting such transcript. The diseases associated with each gene in Table 1 include diseases that arise due to or are correlated with an insufficiency of the corresponding uORF-containing gene as well as diseases that could be ameliorated by up-regulation of the corresponding uORF-containing gene. The diseases associated with each gene in Table 2 include diseases that correlate with a mutation or SNP that introduces a uORF in the corresponding gene.

In certain embodiments, antisense compounds are targeted to one or more TSEs in the 5'UTR of a gene in Table 1 or Table 2. In certain embodiments, antisense compounds are not targeted to one or more TSEs in the 5'UTR of a gene in Table 1 or Table 2.

**Table 1**

| **uORF-containing genes and associated diseases** | | |
|---|---|---|
| Gene | NCBI Gene ID | Associated disease(s) |
| ABCA1 | 19 | Cardiovascular, Dry AMD, dyslipidemia, and atherosclerosis |
| ABCB11 | 8647 | Cholestasis, primary sclerosing cholangitis and biliary cirrhosis |
| ABCC2 | 1244 | Dubin-Johnson syndrome- but overexpressed in cancer |
| ABCG5 | 64240 | Cholestasis, primary sclerosing cholangitis and biliary cirrhosis |
| ADAM10 | 102 | Alzheimer's Disease |
| ALB | 213 | liver disease, nephrotic syndrome, renal disease, and analbuminemia |
| ANK1 | 286 | Hereditary spherocytosis |
| APOE | 348 | Cancer, melanoma, pulmonary hypertension, dyslipidemia, atherosclerosis, Alzheimer disease, Lipoprotein glomerulopathy, and Sea-blue histiocyte disease |
| ATP2A2 | 488 | cardiac diseases, congenital heart disease, aortic aneurysms, aortic dissections, arrhythmia, cardiomyopathy, congestive heart failure, Darier-White disease, muscular dystrophy, and Acrokeratosis verruciformis |
| ATP7B | 540 | wilson disease, and menkes disease. |
| ATRX | 546 | alpha-thalassemia myelodysplasia syndrome, somatic, and mental retardation-hypotonic facies syndrome, x-linked. |
| ATXN1 | 6310 | Spinocerebellar ataxia-1 |
| ATXN1L | 342371 | Spinocerebellar ataxia-1 |
| BAX | 581 | Cancer |
| BCL2L11 | 10018 | Cancer, e.g. human T-cell acute lymphoblastic leukemia and lymphoma |
| BDNF | 627 | neurodegeneration diseases, amyotrophic lateral sclerosis, Alzheimer's Disease, Huntington' s disease (HD), or Parkinson's Disease (PD) |
| BLM | 641 | bloom syndrome, and rothmund-thomson syndrome. |
| BRCA1 | 672 | Cancer, e.g. breast cancer, pancreatic cancer |
| C/EBPa | 1050 | B-cell maligancy (B-ALL, DLBCL), AML |
| CA2 | 760 | autoimmune retinopathy, and multifocal fibrosclerosis. |
| CASP8 | 841 | CASP8 deficiency, breast cancer, HCC, lung cancer |
| CCBE1 | 147372 | hennekam syndrome, and immune hydrops fetalis. |
| CD36 | 948 | platelet glycoprotein IV deficiency, coronary heart disease, CHDS7 |
| CD3D | 915 | severe combined immune deficiency, autosomal recessive, t cell-negative, b cell-positive, nk cell-positive, cd3d-related, and immunodeficiency 19. |
| CDKN1B | 1027 | cancer, multiple endocrine neoplasia |
| CDKN2A | 1029 | cancer, melanoma |
| CEP290 | 80184 | Leber's congenital amaurosis (LCA), Bardet-Biedl syndrome (BBS), Joubert syndrome, Meckel syndrome, Sior-Loken syndrome |
| CFH | 3075 | C3 glomerulopathy, AMD, PNH, RA etc |
| CFTR | 1080 | Cystic fibrosis, Disseminated bronchiectasis, congenital bilateral absence of vas deferens (CBAVD) |
| CHRNA4 | 1137 | nicotine addiction |
| CHRNA5 | 1138 | nicotine addiction |
| CNTF | 1270 | Multiple Sclerosis |
| CNTFR | 1271 | Multiple Sclerosis |
| COL1A1 | 1277 | Osteogenesis Imperfecta Type I |
| CR1 | 1378 | Alzherimer's Disease |
| CSPP1 | 79848 | joubert syndrome 21, and joubert syndrome with jeune asphyxiating thoracic dystrophy. |
| CTNND2 | 1501 | Cri-du-chat syndrome |
| CTNS | 1497 | intermediate cystinosis, and cystinosis, atypical nephropathic |
| CYP1B1 | 1545 | Glaucoma, Peters anomaly |
| DBT | 1629 | maple syrup urine disease type 2, and maple syrup urine disease type 1a |
| DCAF17 | 80067 | sakati syndrome, and hypogonadism, alopecia, diabetes mellitus, mental retardation, and extrapvramidal syndrome |
| DNASE1 | 1773 | cystic fibrosis, acute bronchitis |
| DDIT3 | 1649 | Myxoid liposarcoma |
| DICER1 | 23405 | DICER1 symdrome, pleuropulmonary blastoma, cystic nephroma, Sertoli-Leydig tumors, multinodular goiter, cancer |
| DRD3 | 1814 | mood disorders |
| EED | 8726 | HIV-1 |
| EFNB1 | 1947 | CFNS |
| EPO | 2056 | erythropoiesis and anemia |
| ESR1 | 2099 | inhibits ERBB1, breast cancer |
| ETHE1 | 23474 | ethylmalonic encephalopathy |
| EZH2 | 2146 | weaver syndrome, ezh2-related overgrowth, lymphomas and leukemias |
| F8 (and F2, 3, 5, 7, 11, 13) | 2147, '52, '53, '55, '57, '60 | Hemophilia, bleeding |
| FAP | 2191 | glomuvenous malformations |
| FMR1 | 2332 | Fragile X syndrome and premature ovarian failure |
| FNDC5 | 252995 | Obesity, Type 2 Diabetes |
| FXN | 2395 | Friedreich's ataxia |
| GALNS | 2588 | mucopolysaccharidosis iv, and kniest dysplasia |
| GATA3 | 2625 | Cancer |
| GBA | 2629 | Synucleinopathies, Gaucher's disease |
| GCH1 | 2643 | gtp cyclohydrolase I deficiency, Parkinson's disease, movement disorders, CNS disease, doparesponsive dystonia, hyperpehnylalaninemia, and atypical severe phenylketonuria |
| GCK | 2645 | Obesity, Type 2 Diabetes, and Hyperinsulinemic hypoglycemia |
| GH2 | 2689 | idiopathic short stature, growth delay |
| GRN | 2896 | autoimmune, inflammatory, dementia, FTD, cancer, e.g. hepatic cancer |
| HBB | 3043 | thallasemia, sickle cell disease, and anemia |
| HBD | 3045 | thallasemia, sickle cell disease, and anemia |
| HBE1 | 3046 | thallasemia, sickle cell disease, and anemia |
| HBG1 | 3047 | Anemia (e.g., Fanconi's anemia), thalassemia (e.g., beta-thalassemia etc.), sickle cell disease, leukemia, cellular dyscrasia, dyserythropoiesis, anisocytosis and poikilocytosis. |
| HBG2 | 3048 | thallasemia, sickle cell disease, and anemia |
| HCRT | 3060 | Narcolepsy/Excessive Daytime Sleepiness |
| HGF | 3082 | Ischemic disease, restenosis after percutaneous transluminal coronary angioplasty (PTCA), arteriosclerosis, insufficiency of peripheral circulation, myocardial infarction, myocardia, peripheral angiostenosis, cardiac insufficiency, nerve degeneration, neuropathy, neurotoxin induced lesions, injury of nerve cell, lesions of nerve cell by infection, epilepsy, head trauma, dementia, cerebral stroke, cerebral infarction, amyotrophic lateral sclerosis, Parkinson's disease, Alzheimer's disease, cancer, tumor, liver cirrhosis, Nonalcoholic fatty liver disease, renal fibrosis, rhabdomyolysis, pulmonary fibrosis, blood coagulopathy, adenosine deaminase deficiency, Chronic Ulcerative Colitis, Crohn's Disease, necrotizing enterocolitis, severe acute gastroenteritis, chronic gastroenteritis, cholera, chronic infections of the bowel, AIDS, pustulous fibrosis, fibrosis, osteoporosis, Arterial sclerosis, chronic glomerulonephritis, cutis keloid formation, progressive systemic sclerosis (PSS), liver fibrosis, pulmonary fibrosis, cystic fibrosis, chronic graft versus host disease, scleroderma (local and systemic), Peyronie's disease, penis fibrosis, inner accretion after surgery, myelofibrosis, idiopathic retroperitoneal fibrosis, hemophilia, decubitus ulcer, scar, atopic dermatitis, or skin damage following a skin graft |
| HNF4a | 3172 | HCC, fibrosis |
| HR | 55806 | atrichia with papular lesions, and hypotrichosis 4 |
| HSD17B4 | 3295 | D-bifunctional protein deficiency |
| IDO1 | 3620 | autoimmune and inflammatory diseases |
| IFNE and other interferon genes | 338376, others | Cancer, HBV, and other virus infection |
| IFRD1 | 3475 | Cystic fibrosis, Chronic obstructive pulmonary disease (COPD), inflammation, lung cancer, sensory/motor neuropathy, a neuronal injury |
| IGF1 | 3479 | CNS diseases, metabolic disease, delayed growth, cancer |
| IGF1R | 3480 | Insulin-like growth factor I resistance |
| IGF2 | 3481 | Russell-Silver syndrome |
| IGF2BP2 | 10644 | Type 2 diabetes, insulin resistance susceptibility |
| IGFBP3 | 3486 | growth delay |
| IGHMBP2 | 3508 | progressive multifocal leukoencephalopathy, and spinal muscular atrophy with respiratory distress 1 |
| IL6 | 3569 | infectious disease, vaccination, and cancer |
| INS | 3630 | Diabetes or related disorders thereof, an insulin resistant non diabetic state, obesity, impaired glucose tolerance (IGT), Metabolic Syndrome, MODY syndrome,Polycystic Ovary Syndrome, cancer, inflammation, hirsuitism, and hypertension. |
| IQGAP 1 | 8826 | Cancer, obesity, diabetes, multiple sclerosis, neoplastic transformation, inflammation, Nonsmall cell lung carcinoma (NSCLCs), hypercholesterolemia, liposarcoma, gastric cancer, immunodeficiency, glomerulonephritis, venous thrombosis, glioma |
| IQGAP2 | 10788 | Obesity, diabetes, multiple sclerosis, neoplastic transformation, inflammation, Nonsmall cell lung carcinoma (NSCLCs), hypercholesterolemia, liposarcoma, gastric cancer, immunodeficiency, glomerulonephritis, venous thrombosis, glioma |
| IRF6 | 3664 | van der Woude syndrome |
| IRS2 | 8660 | Alzheimer's disease, Parkinson's disease, amyotrophic lateral sclerosis, insulin resistance, diabetes, Polycystic Ovary Syndrome, atherosclerosis, cancer |
| ITGA7 | 3679 | muscular dystrophy, congenital, due to itga7 deficiency, and congenital muscular dystrophy due to integrin alpha-7 deficiency |
| JAG1 | 182 | Alagille syndrome |
| KCNJ11 | 3767 | Congenital hyperinsulinism, hyperinsulinemic hypoglycemia, 2 |
| KCNMA1 | 3778 | vascular disease, kidney disease, Obesity, Type 2 Diabetes, inflammatory disease, autoimmune disease, and cancer, e.g. kidney, lung, or ovarian cancer |
| KCNMB1 | 3779 | vascular disease, kidney disease, Obesity, Type 2 Diabetes, inflammatory disease, autoimmune disease, and cancer, e.g. kidney, lung, or ovarian cancer |
| KCNMB2 | 10242 | vascular disease, kidney disease, Obesity, Type 2 Diabetes, inflammatory disease, autoimmune disease, and cancer, e.g. kidney, lung, or ovarian cancer |
| KCNMB3 | 27094 | vascular disease, kidney disease, Obesity, Type 2 Diabetes, inflammatory disease, autoimmune disease, and cancer, e.g. kidney, lung, or ovarian cancer |
| KCNQ3 | 3786 | kcnq3-related benign familial neonatal epilepsy, and seizures, benign neonatal, type 2 |
| KLF4 | 9314 | thallasemia, sickle cell disease, and anemia |
| KMT2D | 8085 | Kabuki Syndrome |
| LDLR | 3949 | dyslipidemias, atherosclerosis, and hypercholesterolemia, cardiovascular disease |
| LRP1 | 4035 | Cancer, melanoma |
| LRP5, LRP5L | 4041, 91355 | exudative vitreoretinopathy 4, and hyperostosis, endosteal |
| LRP8 | 7804 | Cancer, melanoma |
| LRPPRC | 10128 | Leigh syndrome Frencn-Canadian type, Cytochrome c oxidase deficiency |
| MBTPS1 | 8720 | Colitis, obesity, diabetes, hypercholesterolemia, dyslipidemia, Crimean-Congo hemorrhagic fever, chondrodysplasia |
| MECP2 | 4204 | Rett Syndrome, MECP2-related severe neonatal encephalopathy, Angelman syndrome, and PPM-X syndrome |
| MSRA | 4482 | cancer, macular degeneration, eye aging, cataract |
| MSX2 | 4488 | tooth agenesis (dentin dysplasia), developmental disorders e.g. Craniosynostosis and Parietal foramina |
| MTR | 4548 | Homocystinuria |
| MUTYH | 4595 | Familial adenomatous polyposis |
| MYCN | 4613 | Feingold syndrome |
| MYF6 | 4618 | Centronuclear Myopathy 3 |
| NAMPT | 10135 | cancer, cytopenia of the myeloid or lymphoid lineage, neutropenia, leukaemia, acute myeloid leukaemia (AML), atherosclerosis, inflammatory bowel disease, Crohn's disease, ulcerative colitis, psoriasis, arthritis, chronic ulcer, ischemic stroke, myocardial infarction, angina and vascular dementia, inflammation, nonalcoholic fatty liver disease |
| NANOG | 79923 | diabetes, osteoarthritis, rheumatoid arthritis, cancer, Duchenne muscular dystrophy, Parkinson's, Alzheimer's, Gaucher disease, type I diabetes, spinal cord injury, burns (tissue regeneration) |
| NEU4 | 129807 | cancer, diabetes, Tay Sachs disease, inflammatory bowel disease, Crohn's disease , ulcerative colitis, psoriasis, arthritis, inflammation, insulin resistance syndrome, hyperlipidemia, fatty liver disease, cachexia, obesity, atherosclerosis, artcriosccrlosis, elevated blood pressure, viral infection |
| NF1 | 4763 | neurofibromatosis and cancer, e.g., neurofibrosarcoma, malignant peripheral nerve sheath tumors, and myelomonocytic leukemia |
| NKX2-3, -5, -8 | 159296, 1482, 26257 | cancer, e.g., lung cancer |
| NOD2 | 64127 | Crohn disease |
| NR5A1 | 2516 | nr5a1-related 46,xy dsd and 46,xy cgd, and adrenocortical insufficiency, without ovarian defect |
| NRF1 | 4899 | Alzheimer's disease, Parkinson's disease, amyotrophic lateral sclerosis, insulin resistance, diabetes, hepatic tumor, non-small cell bronchopulmonary cancer, mitochondrial disease |
| NSD1 | 64324 | Sotos syndrome (cerebral gigantism)autosomal dominant disorder. The cause is haploinsufficiency of the NSD1 gene |
| PAH | 5053 | Phenylketonuria (PKU) |
| PARK2 | 5071 | Parkinson's |
| PKD1 | 5310 | Polycystic kidney disease |
| PLAT | 5327 | ischemic stroke |
| PON1, 2 | 5444, 5445 | diabetes, obesity, hypercholesterolemia, high blood pressure, atherosclerosis, coronary heart disease, autism/autism spectrum disorder, epilepsy, cancer, inflammation, stroke, trauma, a renal disease, rheumatoid arthritis, Fish-Eye disease, purpura, Polycystic Ovary Syndrome, hyperthyroidism, a hepatic diseases, vascular dementia, an infectious disease |
| PPARD | 5467 | Metabolic disease |
| PRKAR1A | 5573 | Carney complex |
| PRPF31 | 26121 | adRP |
| PTEN | 5728 | cancer |
| PYCR1 | 5831 | cystic fibrosis, myocardial fibrosis, myelofibrosis, hepatic fibrosis, interstitial lung fibrosis, neoplastic fibrosis, pancreatic fibrosis, pulmonary fibrosis, subepidermal fibrosis, panmural fibrosis of the bladder, proliferative fibrosis, replacement fibrosis, retroperitoneal fibrosis and root sleeve fibrosis, osteogenesis imperfecta, Ehlers-Danlos syndrome, chondrodysplasias, Marfan syndrome, Alport syndrome, familial aortic aneurysm, achondroplasia, mucopolysaccharidoses, osteoporosis, osteopetrosis, Paget's disease, rickets, osteomalacia, hyperparathyroidism, renal osteodystrophy, osteonecrosis, osteomyelitis, osteoma, osteoid osteoma, osteoblastoma, osteosarcoma, osteochondroma, chondroma, chondroblastoma, chondromyxoid fibroma, chondrosarcoma, fibrous cortical defect, nonossifying fibroma, fibrous dysplasia, fibrosarcoma, malignant fibrous histiocytoma, Ewing's sarcoma |
| RB1, RBL1, RBL2 | 5925, 5933, 5934 | cancer, e.g. bladder cancer, osteosarcoma, retinoblastoma, small cell lung cancer |
| RBBP4 | 5928 | intermediate charcot-marie-tooth neuropathy, retinoblastoma, Alzheimer's |
| RNASEH1 | 246243 | leishmaniasis, a disease or disorder associated with mitochondrial dysfunction, cancer, Aicardi-Goutieres syndrome, AIDS |
| ROR2 | 4920 | brachydactyly, type b1, and brachydactyly type b |
| RPS 14 | 6208 | 5q syndrome (myelodysplastic syndrome) |
| RPS19 | 6223 | Diamond-Blackfan Anemia |
| SCN1A | 6323 | convulsion, pain, paralysis, hyperkalemic periodic paralysis, paramyotonia congenita, potassium-aggravated myotonia, long Q-T syndrome 3, motor endplate disease, ataxia, colitis, ileitis, inflammatory bowel syndrome, hypertension, congestive heart failure, benign prostrate hyperplasia, impotence, muscular dystrophy, multiple sclerosis, epilepsy, autism, migraine, severe myoclonic epilepsy of infancy (SMEI or Dravet's syndrome) |
| SCN2A | 6326 | epileptic encephalopathy, early infantile, 11, and benign familial neonatal-infantile seizures |
| SERPINF1 | 5176 | cancer, choroidal neovascularization, cardiovascular disease, diabetes, and osteogenesis imperfecta |
| SERPING1 | 710 | Hereditary Angioedema |
| SHBG | 6462 | disorders of mood and affect, a memory dysfunction disease or disorder, an amnestic disease or disorder, a motor and tic disorder, substance abuse disease or disorder, a psychotic disease or disorder, an anxiety disease or disorder, schizophrenia, schizofreniform disorder, schizoaffective disorder, and delusional disorder, panic disorder, phobias, an obsessive-compulsive disorder, posttraumatic stress disorder, infertility, hirsutism, Tourette's disorder, Asperger syndrome, hypothyroidism, fibromyalgia, chronic fatigue syndrome, hypothalamic-pituitary axis dysregulation, chronic sleep deprivation, alopecia, prostate cancer, breast cancer, polycystic ovary syndrome, osteoporosis, hyperinsulinemia, glucose intolerance, insulin resistance, diabetes |
| SIRT1 | 23411 | cancer, Alzheimer's Disease (AD), Huntington's disease, Parkinson's disease, Amyotrophic Lateral Sclerosis (ALS), Multiple Sclerosis, Duchene muscular dystrophy, skeletal muscle atrophy, Becker's dystrophy, myotonic dystrophy, insulin resistance, diabetes, obesity, Hypercholesterolemia, dyslipidemia hyperlipidemia, sensory neuropathy, autonomic neuropathy, motor neuropathy, retinopathy, hepatitis, fatty liver disease, age-related macular degeneration, osteoporosis, leukemia, bone resorption, dementia, Bell's Palsy, atherosclerosis, cardiac dysrhymias, chronic congestive heart failure, ischemic stroke, coronary artery disease, cardiac muscle disease, chronic renal failure, ulceration, cataract, presbiopia, glomerulonephritis, Guillan-Barre syndrome, hemorrhagic stroke, rheumatoid arthritis, inflammatory bowel disease, SLE, Crohn's disease, osteoarthritis, Chronic Obstructive Pulmonary Disease (COPD), pneumonia, urinary incontinence, mitochondrial myopathy, encephalopathy, Leber's disease, Leigh encephalopathia, Pearson's disease, lactic acidosis, mitochondrial encephalopathy, lactic acidosis and stroke like symptoms (MELAS), inflammation |
| SLC1A2 | 6506 | ALS |
| SMAD7 | 4092 | Acute kidney injury (anti-TGFb), colorectal cancer |
| SMCHD1 | 23347 | FSHD |
| SMN1, SMN2 | 6606, 6607 | Spinal muscular atrophy |
| SNX27 | 81609 | Downs' Syndrome |
| SPINK1 | 6690 | Pancreatits |
| SRB1 | 949 | Cardiovascular disease |
| SRY | 6736 | Gonadal dysgenesis |
| ST7, ST7L | 7982, 54879 | cancer, e.g. myeloid cancer, head and neck squamous cell carcinomas, breast cancer, colon carcinoma, and prostate cancer |
| STAT3 | 6774 | tissue regeneration and Hyper-IgE recurrent infection syndrome |
| TFE3 | 7030 | diabetes, obesity, impaired glucose tolerance (IGT) and Metabolic Syndrome, Polycystic Ovary Syndrome, atherosclerosis, cancer, Alzheimer's disease, Parkinson's disease, amyotrophic lateral sclerosis, diabetic retinopathy, diabetic neuropathy, diabetic amyotrophy, diabetic nephropathy, diabetic cardiomyopathy, angina, myocardial infarction, stroke, a peripheral vascular disease |
| TFEB | 7942 | Lysosomal storage diseases |
| TGFB3 | 7043 | Rienhoff syndrome |
| THPO | 7066 | Myelosuppressive chemo, Bleeding disorders |
| TP63 | 8626 | cancer, tumor, Corneal dystrophy, premature menopause, alopecia, cctrodaclyly-ectodermal dysplasia-cleft syndrome, Hay-Wells syndrome, limb mammary syndrome, acro-dermato-ungual-lacrimal-tooth syndrome, nonsyndromic split-hand/foot malformation, isolated cleft lip/palate, Rapp-Hodgkin syndrome |
| TP73 | 7161 | Cancer |
| UCP2 | 7351 | cancer, obesity, cachexia, anorexia nervosa, bulimia nerv osa, diabetes, hyperinsulinemia, glucose intolerance, atherosclerosis, inflammation |
| USP9Y/SP3 | 8287 | Y chromosome infertility |
| UTRN | 7402 | muscular dystrophies, Duchenne muscular dystrophy (DMD), Becker Muscular Dystrophy (BMD), and myotonic dystrophy |
| VEGFA | 7422 | diabetes, coronary artery disease, congestive heart failure, and peripheral vascular disease, cancer, infectious diseases, rheumatoid arthritis, DiGeorge syndrome, HHT, cavernous hemangioma, atherosclerosis, transplant ateriopathy, obesity, psoriasis, warts, allergic dermatitis, scar keloids, pyogenic granulomas, blistering disease, Kaposi sarcoma, persistent hyperplastic vitreous syndrome, Autosomal dominant polycystic kidney disease (ADPKD), diabetic retinopathy, retinopathy of prematurity, macular degeneration, choroidal neovascularization, primary pulmonary hypertension, asthma, nasal polyps, inflammatory bowel disease, Alzheimer's disease, Parkinson's disease, amyotrophic lateral sclerosis, periodontal disease, ascites, peritoneal adhesions, endometriosis, uterine bleeding, ovarian cysts, ovarian hyperstimulation, arthritis, synovitis, osteomyelitis, and/or osteophyte formation, ulceration, verruca vulgaris, angiofibroma of tuberous sclerosis, pot-wine stains, Sturge Weber syndrome, Kippel-Trenaunay-Weber syndrome, Osler-Weber-Rendu syndrome |

**Table 2**

| **Genes with mutations or SNPs that create uORFs and associated diseases** | | |
|---|---|---|
| Gene | NCBI Gene ID | Associated disease(s) |
| ATP7B | 540 | wilson disease, and menkes disease. |
| ATRX | 546 | alpha-thalassemia myelodysplasia syndrome, somatic, and mental retardation-hypotonic facies syndrome, x-linked. |
| BLM | 641 | bloom syndrome, and rothmund-thomson syndrome. |
| BRCA1 | 672 | primary peritoneal carcinoma, and hereditary site-specific ovarian cancer syndrome. |
| CA2 | 760 | autoimmune retinopathy, and multifocal fibrosclerosis. |
| CCBE1 | 147372 | hennekam syndrome, and immune hydrops fetalis. |
| CD3D | 915 | severe combined immune deficiency, autosomal recessive, t cell-negative, b cell-positive, nk cell-positive, cd3d-related, and immunodeficiency 19. |
| CD4 | 920 | okt4 epitope deficiency, and lymphatic system disease. |
| CDKN2A | 1029 | Melanoma predisposition, Melanoma |
| CFL2 | 1073 | cf12-related nemaline myopathy, and nemaline myopathy 7, autosomal recessive. |
| CFTR | 1080 | Cystic fibrosis, Disseminated bronchiectasis |
| CSPP1 | 79848 | joubert syndrome 21, and joubert syndrome with jeune asphyxiating thoracic dystrophy. |
| CTNS | 1497 | intermediate cystinosis, and cystinosis, atypical nephropathic |
| DBT | 1629 | maple syrup urine disease type 2, and maple syrup urine disease type 1a |
| DCAF17 | 80067 | sakati syndrome, and hypogonadism, alopecia, diabetes mellitus, mental retardation, and extrapyramidal syndrome |
| DCLRE1C | 64421 | severe combined immunodeficiency, athabascan type, and artemis deficiency. |
| DFNB31 | 25861 | deafness, autosomal recessive 31, and dfnb31 nonsyndromic hearing loss and deafness. |
| DLG4 | 1742 | schizophrenia. |
| DMD | 1756 | duchenne muscular dystrophy, and becker muscular dystrophy. |
| DNASE1 | 1773 | cystic fibrosis, acute bronchitis |
| ETHE1 | 23474 | ethylmalonic encephalopathy |
| GALNS | 2588 | mucopolysaccharidosis iv, and kniest dysplasia |
| GCH1 | 2643 | Levodopa responsive dystonia |
| HAMP | 57817 | Juvenile hemochromatosis, thalassemia |
| HBB | 3043 | Beta-Thalassemia |
| HMBS | 3145 | histrionic personality disorder, and acute porphyria. |
| HR | 55806 | atrichia with papular lesions, and hypotrichosis 4 |
| IGHMBP2 | 3508 | progressive multifocal leukoencephalopathy, and spinal muscular atrophy with respiratory distress 1 |
| IRF6 | 3664 | Van der Woude syndrome |
| ITGA7 | 3679 | muscular dystrophy, congenital, due to itga7 deficiency, and congenital muscular dystrophy due to integrin alpha-7 deficiency |
| ITGB2 | 3689 | leukocyte adhesion deficiency type 1, and leukocyte adhesion deficiency. |
| KCNJ11 | 3767 | Congenital hyperinsulinism, hyperinsulinemic hypoglycemia, 2 |
| KCNQ3 | 3786 | kcnq3-related benign familial neonatal epilepsy, and seizures, benign neonatal, type 2 |
| LDLR | 3949 | Cardiovascular disease, Familial hypercholesterolemia |
| LRP5, LRP5L | 4041, 91355 | exudative vitreoretinopathy 4, and hyperostosis, endosteal |
| MECP2 | 4204 | autism susceptibility, x-linked 3, and bruxism. |
| MLH1 | 4292 | mlh1-related lynch syndrome, and solitary rectal ulcer syndrome. |
| MSH6 | 2956 | msh6-related lynch syndrome, colorectal cancer, hereditary nonpolyposis, type 5 |
| MUTYH | 4595 | adenomas, multiple colorectal, stomach cancer. |
| NR5A1 | 2516 | nr5a1-related 46,xy dsd and 46,xy cgd, and adrenocortical insufficiency, without ovarian defect |
| PALB2 | 79728 | fanconi anemia, complementation group n, and pancreatic cancer susceptibility 3. |
| PANK2 | 80025 | classic pantothenate kinase-associated neurodegeneration, and harp syndrome. |
| PEX7 | 5191 | peroxisome biogenesis disorder 9b, and rhizomelic chondrodysplasia punctata. |
| PHYH | 5264 | phyh-related refsum disease, and refsum disease. |
| PIK3R5 | 23533 | ataxia-oculomotor apraxia 3, and spinocerebellar ataxia autosomal recessive 1 |
| POMC | 5443 | Proopiomelanocortin deficiency |
| POMT1 | 10585 | pomt1-related muscle diseases, and walker-warburg syndrome. |
| ROR2 | 4920 | brachydactyly, type b1, and brachydactyly type b |
| SCN2A | 6326 | epileptic encephalopathy, early infantile, 11, and benign familial neonatalinfantile seizures |
| SGCA | 6442 | sarcoglycanopathies, and limb-girdle muscular dystrophy, type 2d. |
| SGCD | 6444 | limb-girdle muscular dystrophy type 2f, and delta-sarcoglycanopathy. |
| SLC16A1 | 6566 | erythrocyte lactate transporter defect, and exercise-induced hyperinsulinemic hypoglycemia. |
| SLC19A3 | 80704 | basal ganglia disease, and biotin-responsive basal ganglia disease. |
| SLC2A2 | 6514 | fanconi bickel syndrome,fanconi syndrome |
| SLC7A9 | 11136 | stinuria, and aminoaciduria. |
| SPINK1 | 6690 | Hereditary pancreatitis |
| SRY | 6736 | Gonadal dysgenesis |
| STIL | 6491 | primary autosomal recessive microcephaly type 7, and ideomotor apraxia. |
| TK2 | 7084 | mitochondrial dna depletion syndrome, myopathic form, and tk2-related mitochondrial dna depletion syndrome, myopathic form. |
| TMPRSS3 | 64699 | deafness, autosomal recessive 8/10, and dfnb 8/10 nonsyndromic hearing loss and deafness. |
| TP53 | 7157 | hepatocellular carcinoma, and osteosarcoma |
| TPI1 | 7167 | hemolytic anemia due to triosephosphate isomerase deficiency, and triose phosphate-isomerase deficiency. |
| TPM3 | 7170 | nemaline myopathy, and nemaline myopathy 1 |
| TRMU | 55687 | liver failure acute infantile, and melas, mt-th-related. |
| TSEN54 | 283989 | tsen54-related pontocerebellar hypoplasia, and pontocerebellar hypoplasia type 4. |
| ZEB1 | 6935 | corneal dystrophy, posterior polymorphous, 3, and corneal dystrophy, fuchs endothelial, 6. |

In certain embodiments, a uORF inhibitor (i.e. an antisense compound), inhibits ribosomal recognition or uORF activity of a uORF start site on the CDKN2A transcript and thereby increases expression of CDKN2A. In certain embodiments, a uORF inhibitor (i.e. an antisense compound), inhibits ribosomal recognition or uORF activity of a uORF start site on the CFTR transcript and thereby increases expression of CFTR. In certain embodiments, a uORF inhibitor (i.e. an antisense compound), inhibits ribosomal recognition or uORF activity of a uORF start site on the FXII transcript and thereby increases expression of FXII. In certain embodiments, a uORF inhibitor (i.e. an antisense compound), inhibits ribosomal recognition or uORF activity of a uORF start site on the GCH1 transcript and thereby increases expression of GCH1. In certain embodiments, a uORF inhibitor (i.e. an antisense compound), inhibits ribosomal recognition or uORF activity of a uORF start site on the HAMP transcript and thereby increases expression of HAMP.

In certain embodiments, a uORF inhibitor (i.e. an antisense compound), inhibits ribosomal recognition or uORF activity of a uORF start site on the HBB transcript and thereby increases expression of HBB. In certain embodiments, a uORF inhibitor (i.e. an antisense compound), inhibits ribosomal recognition or uORF activity of a uORF start site on the IRF6 transcript and thereby increases expression of IRF6. In certain embodiments, a uORF inhibitor (i.e. an antisense compound), inhibits ribosomal recognition or uORF activity of a uORF start site on the KCNJ11 transcript and thereby increases expression of KCNJ11. In certain embodiments, a uORF inhibitor (i.e. an antisense compound), inhibits ribosomal recognition or uORF activity of a uORF start site on the LDLR transcript and thereby increases expression of LDLR. In certain embodiments, a uORF inhibitor (i.e. an antisense compound), inhibits ribosomal recognition or uORF activity of a uORF start site on the PEX7 transcript and thereby increases expression of PEX7.

In certain embodiments, a uORF inhibitor (i.e. an antisense compound), inhibits ribosomal recognition or uORF activity of a uORF start site on the POMC transcript and thereby increases expression of POMC. In certain embodiments, a uORF inhibitor (i.e. an antisense compound), inhibits ribosomal recognition or uORF activity of a uORF start site on the PRKAR1A transcript and thereby increases expression of PRKAR1A. In certain embodiments, a uORF inhibitor (i.e. an antisense compound), inhibits ribosomal recognition or uORF activity of a uORF start site on the SPINK1 transcript and thereby increases expression of SPINK1. In certain embodiments, a uORF inhibitor (i.e. an antisense compound), inhibits ribosomal recognition or uORF activity of a uORF start site on the SRY transcript and thereby increases expression of SRY.

In certain embodiments, a TSE inhibitor (i.e. an antisense compound) may be used to upregulate a expression of ATM, lipoprotein lipase (LPL), DMD, sphingomyelinase, Factor VIII, insulin, growth hormone, thyroid stimulating hormone, follicle stimulating hormone, or hepcidin.

### D. Certain Pharmaceutical Compositions

In certain embodiments, the present invention provides pharmaceutical compositions comprising one or more antisense compound. In certain embodiments, such pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one or more antisense compound and sterile water. In certain embodiments, the sterile saline is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile phosphate-buffered saline (PBS). In certain embodiments, the sterile saline is pharmaceutical grade PBS.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

Pharmaceutical compositions comprising antisense compounds encompass any pharmaceutically acceptable salts, esters, or salts of such esters. In certain embodiments, pharmaceutical compositions comprising antisense compounds comprise one or more oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an oligomeric compound which are cleaved by endogenous nucleases within the body, to form the active compound.

Lipid moieties have been used in nucleic acid therapies in a variety of methods. In certain such methods, the nucleic acid is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions provided herein comprise one or more modified oligonucleotides and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition provided herein comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition provided herein comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition provided herein comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80^{™} and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80^{™}; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition provided herein is prepared for oral administration. In certain embodiments, pharmaceutical compositions are prepared for buccal administration.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain.

### E. Administration

In certain embodiments, the compounds and compositions as described herein are administered parenterally.

In certain embodiments, parenteral administration is by infusion. Infusion can be chronic or continuous or short or intermittent. In certain embodiments, infused pharmaceutical agents are delivered with a pump. In certain embodiments, parenteral administration is by injection.

In certain embodiments, compounds and compositions are delivered to the CNS. In certain embodiments, compounds and compositions are delivered to the cerebrospinal fluid. In certain embodiments, compounds and compositions are administered to the brain parenchyma. In certain embodiments, compounds and compositions are delivered to an animal by intrathecal administration, or intracerebroventricular administration. Broad distribution of compounds and compositions, described herein, within the central nervous system may be achieved with intraparenchymal administration, intrathecal administration, or intracerebroventricular administration.

In certain embodiments, parenteral administration is by injection. The injection may be delivered with a syringe or a pump. In certain embodiments, the injection is a bolus injection. In certain embodiments, the injection is administered directly to a tissue, such as striatum, caudate, cortex, hippocampus and cerebellum.

Therefore, in certain embodiments, delivery of a compound or composition described herein can affect the pharmacokinetic profile of the compound or composition. In certain embodiments, injection of a compound or composition described herein, to a targeted tissue improves the pharmacokinetic profile of the compound or composition as compared to infusion of the compound or composition. In a certain embodiment, the injection of a compound or composition improves potency compared to broad diffusion, requiring less of the compound or composition to achieve similar pharmacology. In certain embodiments, similar pharmacology refers to the amount of time that a target mRNA and/or target protein is up-regulated (e.g. duration of action). In certain embodiments, methods of specifically localizing a pharmaceutical agent, such as by bolus injection, decreases median effective concentration (EC50) by a factor of about 50 (e.g. 50 fold less concentration in tissue is required to achieve the same or similar pharmacodynamic effect). In certain embodiments, methods of specifically localizing a pharmaceutical agent, such as by bolus injection, decreases median effective concentration (EC50) by a factor of 20, 25, 30, 35, 40, 45 or 50. In certain embodiments the pharmaceutical agent in an antisense compound as further described herein. In certain enbodiments, the targeted tissue is brain tissue. In certain enbodiments the targeted tissue is striatal tissue. In certain embodiments, decreasing EC50 is desirable because it reduces the dose required to achieve a pharmacological result in a patient in need thereof.

In certain embodiments, an antisense compound is delivered by injection or infusion once every month, every two months, every 90 days, every 3 months, every 6 months, twice a year or once a year.

### F. Certain Combination Therapies

In certain embodiments, one or more pharmaceutical compositions are co-administered with one or more other pharmaceutical agents. In certain embodiments, such one or more other pharmaceutical agents are designed to treat the same disease, disorder, or condition as the one or more pharmaceutical compositions described herein. In certain embodiments, such one or more other pharmaceutical agents are designed to treat a different disease, disorder, or condition as the one or more pharmaceutical compositions described herein. In certain embodiments, such one or more other pharmaceutical agents are designed to treat an undesired side effect of one or more pharmaceutical compositions as described herein. In certain embodiments, one or more pharmaceutical compositions are co-administered with another pharmaceutical agent to treat an undesired effect of that other pharmaceutical agent. In certain embodiments, one or more pharmaceutical compositions are co-administered with another pharmaceutical agent to produce a combinational effect. In certain embodiments, one or more pharmaceutical compositions are co-administered with another pharmaceutical agent to produce a synergistic effect.

In certain embodiments, one or more pharmaceutical compositions and one or more other pharmaceutical agents are administered at the same time. In certain embodiments, one or more pharmaceutical compositions and one or more other pharmaceutical agents are administered at different times. In certain embodiments, one or more pharmaceutical compositions and one or more other pharmaceutical agents are prepared together in a single formulation. In certain embodiments, one or more pharmaceutical compositions and one or more other pharmaceutical agents are prepared separately.

In certain embodiments, pharmaceutical agents that may be co-administered with a pharmaceutical composition of include antipsychotic agents, such as, e.g., haloperidol, chlorpromazine, clozapine, quetapine, and olanzapine; antidepressant agents, such as, e.g., fluoxetine, sertraline hydrochloride, venlafaxine and nortriptyline; tranquilizing agents such as, e.g., benzodiazepines, clonazepam, paroxetine, venlafaxin, and beta-blockers; mood-stabilizing agents such as, *e.g*., lithium, valproate, lamotrigine, and carbamazepine; paralytic agents such as, *e.g*., Botulinum toxin; and/or other experimental agents including, but not limited to, tetrabenazine (Xenazine), creatine, conezyme Q10, trehalose, docosahexanoic acids, ACR16, ethyl-EPA, atomoxetine, citalopram, dimebon, memantine, sodium phenylbutyrate, ramelteon, ursodiol, zyprexa, xenasine, tiapride, riluzole, amantadine, [123I]MNI-420, atomoxetine, tetrabenazine, digoxin, detromethorphan, warfarin, alprozam, ketoconazole, omeprazole, and minocycline.

In certain embodiments, the present invention may be used to increase expression of a protein, which sensitizes the cell to other treatment. For example, in certain embodiments, the invention may be used to increase expression of RNase H. Cells with increased RNase H may be more sensitive to subsequent treatment with RNase H-dependent antisense compounds.

While certain compounds, compositions and methods described herein have been described with specificity in accordance with certain embodiments, the following examples serve only to illustrate the compounds described herein and are not intended to limit the same.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH for the natural 2'-H of DNA) or as an RNA having a modified base (thymine (methylated uracil) for natural uracil of RNA).

Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified or naturally occurring bases, such as "AT^{m}CGAUCG," wherein ^{m}C indicates a cytosine base comprising a methyl group at the 5-position.

### Examples

The following examples illustrate certain embodiments of the present invention as defined in the appended claims, and are not limiting.
Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

### Example 1: Effects of antisense oligonucleotides targeting the uORF of RNase H1 on RNase H1 protein expression

Human RNase H1 mRNA (GENBANK accession number NM_001286834.1, designated herein as SEQ ID NO: 1) comprises an upstream open reading frame (uORF). Antisense oligonucleotides designed to target the start codon of the uORF of human RNase H1 were tested for their effects on RNase H1 expression *in vitro.* These antisense oligonucleotides, described in Table 3, were uniformly modified in order to avoid inducing cleavage of the target RNA. The start and stop sites listed in Table 3 indicate the positions on SEQ ID NO: 1 that the antisense oligonucleotides target. The targeted uORF begins at position 86. HeLa cells were transfected with Lipofectamine RNAiMAX (Life Technologies) and one of the antisense oligonucleotides at a final concentration of 25 nM or were mock transfected as a control. Thirty hours after transfection, the cells were lysed, and expression of RNase H1 was analyzed by western blot and RT-PCR. The primary antibody used for the western blot was made as described in Wu et al. Determination of the Role of the Human RNase H1 in the Pharmacology of DNA-like Antisense Drugs. J. Biol. Chem. 279, 17181 (2004), and the secondary antibody was purchased from Biorad (catalog #170-6515). The western blot was quantified using Image J, and the results are shown in Table 3 as percent protein levels relative to mock transfected cells following normalization to the Annexin A2 loading control. The RT-PCR results are shown in Table 3 as percent mRNA levels relative to mock transfected cells following normalization to Ribogreen. The results show that antisense oligonucleotides targeting a portion of the RNase H1 uORF that includes the uORF start codon increased RNase H1 expression. Furthermore, certain antisense oligonucleotides did not significantly affect RNase H1 mRNA levels, indicating that the increased RNase H1 expression exhibited by those oligonucleotides occurred mainly via increased translation.

**Table 3: RNase H1 expression**

| Isis No. | Sequence | Start Site | Stop Site | RNase H1 protein (% mock) | RNase H1 mRNA (% mock) | SEQ ID NO. |
|---|---|---|---|---|---|---|
| 761909 | CmoAmoUmoUmoUmoCmoGmoAmoCm oUmoCmoCmoCmoGmoGmoCm | 73 | 88 | 166 | 106 | 3 |
| 761910 | AmoGmoCmoAmoUmoUmoUmoCmoGm oAmoCmoUmoCmoCmoCmoGm | 75 | 90 | 146 | 113 | 4 |
| 761911 | GmoAmoAmoGmoCmoAmoUmoUmoUm oCmoGmoAmoCmoUmoCmoCm | 77 | 92 | 139 | 114 | 5 |
| 761912 | GmoGmoGmoAmoAmoGmoCmoAmoUm oUmoUmoCmoGmoAmoCmoUm | 79 | 94 | 106 | 109 | 6 |
| 761913 | CmoCmoGmoGmoGmoAmoAmoGmoCm oAmoUmoUmoUmoCmoGmoAm | 81 | 96 | 104 | 113 | 7 |

| | | | | | | |
|---|---|---|---|---|---|---|
| Subscripts: "m" indicates a 2'-O-methyl modification, and "o" indicates a phosphodiester internucleoside linkage. | | | | | | |

### Example 2: Time course of RNase H1 protein expression following treatment with an antisense oligonucleotide targeting the uORF in vitro

HeLa cells were transfected with 25 nM Isis No. 761909 (see Table 3). At various time points following transfection, the cells were harvested and RNase H1 expression was analyzed by western blot as described in Example 1. The results are shown in Table 4 as the percent RNase H1 protein expression relative to cells that did not receive antisense oligonucleotide treatment (harvested at the 0 hour time point) following normalization to the Annexin A2 loading control. The results show that the induction of RNase H1 expression was observed within 4 hours following transfection, and maximal expression was observed at approximately 12 hours following transfection.

**Table 4: RNase H1 expression**

| RNase H1 (% 0 h) following transfection with 25 nM 761909 | | | |
|---|---|---|---|
| 4h | 8h | 12 h | 15 h |
| 143 | 145 | 214 | 176 |

### Example 3: Dose response effect of an antisense oligonucleotide targeting the uORF of RNase H1 on RNase H1 protein expression in vitro

Isis No. 761909 (see Table 3) was tested at five different doses for its effect on RNase H1 expression *in vitro.* HeLa cells were transfected with 761909 at a concentration listed in Table 5 or were not treated as a control. Fifteen hours after transfection, RNase H1 expression was analyzed as described in Example 1. The results are shown in Table 5 as the percent protein levels relative to untreated control cells following normalization to the Annexin A2 loading control and as mRNA levels relative to untreated control cells following normalization to Ribogreen. The results show that an antisense oligonucleotide targeting a portion of the RNase H1 uORF increased RNase H1 protein levels in a dose dependent manner; and mRNA levels did not increase, indicating that the antisense oligonucleotide increased RNase H1 expression via increased translation.

**Table 5: RNase H1 expression following transfection with 761909**

| 761909 concentration (nM) | Protein (% untreated cells) | mRNA (% untreated cells) |
|---|---|---|
| 10 | 108 | 100 |
| 15 | 93 | 100 |
| 20 | 183 | 96 |
| 25 | 169 | 92 |
| 30 | 182 | 96 |

### Example 4: Effects of antisense oligonucleotides targeting the 5'-untranslated region upstream of the RNase H1 uORF on RNase H1 protein expression

Antisense oligonucleotides designed to target the 5'-untranslated region upstream of the uORF of human RNase H1 were tested for their effects on RNase H1 protein expression *in vitro.* These antisense oligonucleotides, described in Table 6, were uniformly modified in order to avoid inducing cleavage of the target RNA. The start and stop sites listed in Table 6 indicate the positions on SEQ ID NO: 1 that the antisense oligonucleotides target. Isis 761909 was included for reference. HeLa cells were transfected with 20 nM antisense oligonucleotide or were mock transfected as a control. Fifteen hours after transfection, RNase H1 expression was analyzed as described in Example 1. The results are shown in Table 6 below as percent protein expression relative to mock transfected cells following normalization to the γ-tubulin loading control and percent mRNA levels relative to mock transfected cells following normalization to Ribogreen. The results show that antisense oligonucleotides do not necessarily need to target the start codon of an uORF in order to induce increased translation of the target. Antisense oligonucleotides that target the 5'-untranslated region upstream of an uORF and antisense oligonucleotides that target at least a portion of the uORF itself can induce translation of the target. Furthermore, the antisense oligonucleotides targeting the 5'-untranslated region upstream of the uORF did not increase mRNA levels, indicating that they increased RNase H1 expression via increased translation.

**Table 6: RNase H1 expression**

| Isis No. | Sequence | Start Site | Stop Site | Protein (% mock) | mRNA (% mock) | SEQ ID NO. |
|---|---|---|---|---|---|---|
| 761918 | UmoCmoAmoCmoCmoGmoGmoCmoGmoCmo GmoGmoGmoAmoAmoGm | 14 | 29 | 155 | 96 | 8 |
| 761919 | CmoUmoCmoAmoAmoCmoAmoCmoCmoGmo CmoΛmoCmoUmoUmoCm | 32 | 47 | 214 | 100 | 9 |
| 761917 | AmoCmoUmoCmoCmoCmoGmoGmoCmoCmo CmoAmoGmoCmoGmoUm | 66 | 81 | 116 | 98 | 10 |
| 761916 | CmoGmoAmoCmoUmoCmoCmoCmoGmoGmo CmoCmoCmoAmoGmoCm | 68 | 83 | 142 | 96 | 11 |
| 761915 | UmoUmoCmoGmoAmoCmoUmoCmoCmoCmo GmoGmoCmoCmoCmoAm | 70 | 85 | 218 | 96 | 12 |
| 761909 | CmoAmoUmoUmoUmoCmoGmoAmoCmoUmo CmoCmoCmoGmoGmoCm | 73 | 88 | 151 | 94 | 3 |

| | | | | | | |
|---|---|---|---|---|---|---|
| Subscripts: "m" indicates a 2'-O-methyl modification, and "o" indicates a phosphodiester internucleoside linkage. | | | | | | |

### Example 5: Effects of antisense oligonucleotides of varying lengths on RNase H1 protein expression

Antisense oligonucleotides of varying lengths designed to target at least a portion of the uORF of human RNase H1 were tested for their effects on RNase H1 expression *in vitro.* These antisense oligonucleotides, described in Table 7, were uniformly modified in order to avoid inducing cleavage of the target RNA. The start and stop sites listed in Table 7 indicate the positions on SEQ ID NO: 1 that the antisense oligonucleotides target. HeLa cells were transfected with 20 nM antisense oligonucleotide or were mock transfected as a control. Fifteen hours after transfection, RNase H1 expression was analyzed as described in Example 1. The results are shown in Table 7 below as the percent protein expression relative to mock transfected cells following normalization to the γ-tubulin loading control and percent mRNA levels relative to mock transfected cells following normalization to Ribogreen. The results show that antisense oligonucleotides of various lengths increased RNase H1 expression mainly via increased translation of the target.

**Table 7: RNase H1 expression following transfection with 5'-UTR targeting antisense oligonucleotides**

| Isis No. | Sequence | Start Site | Stop Site | Length | Protein (% mock) | mRNA (% mock) | SEQ ID NO. |
|---|---|---|---|---|---|---|---|
| 761928 | CmoAmoUmoUmoUmoCmoGmoAmoCmoUmoCmo Cm | 77 | 88 | 12 | 143 | 104 | 13 |
| 761927 | CmoAmoUmoUmoUmoCmoGmoAmoCmoUmoCmo CmoCmoGm | 75 | 88 | 14 | 142 | 101 | 14 |
| 761909 | CmoAmoUmoUmoUmoCmoGmoAmoCmoUmoCmo CmoCmoGmoGmoCm | 73 | 88 | 16 | 244 | 94 | 3 |
| 761926 | CmoAmoUmoUmoUmoCmoGmoAmoCmoUmoCmo CmoCmoGmoGmoCmoCmoCm | 71 | 88 | 18 | 187 | 110 | 15 |
| 761925 | CmoAmoUmoUmoUmoCmoGmoAmoCmoUmoCmo CmoCmoGmoGmoCmoCmoCmoAmoGm | 69 | 88 | 20 | 126 | 100 | 16 |

| | | | | | | | |
|---|---|---|---|---|---|---|---|
| Subscripts: "m" indicates a 2'-O-methyl modification, and "o" indicates a phosphodiester internucleoside linkage. | | | | | | | |

### Example 6: Effects of antisense oligonucleotides targeting the uORF of mouse LRPPRC on LRPPRC protein expression

Mouse Leucine-Rich PPR-Motif Containing (LRPPRC) mRNA (GENBANK accession number NM_028233.2, designated herein as SEQ ID NO: 2) comprises an upstream open reading frame. Antisense oligonucleotides with various lengths and internucleoside linkages designed to target the start codon of the uORF of mouse LRPPRC were tested for their effects on LRPPRC protein expression *in vitro.* These antisense oligonucleotides, described in Table 8, were uniformly modified in order to avoid inducing cleavage of the target RNA. The start and stop sites listed in Table 8 indicate the positions on SEQ ID NO: 2 that the antisense oligonucleotides target. The targeted uORF begins at position 70. MHT cells were transfected with Lipofectamine RNAiMAX (Life Technologies) and an antisense oligonucleotide at a concentration listed in Table 8 or were untreated as a control. Fifteen hours after transfection, the cells were lysed, and a western blot was performed with an LRPPRC antibody purchased from Abcam (catalog # ab97505) to analyze expression of LRPPRC. The western blot was quantified using Image J, and the results are shown in Table 8 below as the percent expression relative to untreated control cells following normalization to the loading control (Annexin A2 for 761932 and 761933, and hnRNP K for 759704 and 761930). The results show that antisense oligonucleotides of various lengths targeting the start codon of the uORF of mouse LRPPRC increased LRPPRC expression, including oligonucleotides comprising phosphodiester internucleoside linkages and an oligonucleotide comprising phosphorothioate internucleoside linkages. Thus, the results in Table 8 along with the results in the Examples above show that various antisense oligonucleotides targeting at least a portion of an uORF can increase expression of multiple targets in multiple species.

**Table 8: LRPPRC expression**

| Isis No. | Sequence | Start Site | Stop Site | Concentration (nM) | LRPPRC (% UTC) | SEQ ID NO. |
|---|---|---|---|---|---|---|
| 761932 | CmoAmoUmoUmoGmoUmoUmoUmoUmoUmoUm oGmoUmoCmoUmoUmoCmoCm | 55 | 72 | 10 | 127 | 17 |
| | | | | 20 | 184 | |
| | | | | 30 | 214 | |
| | | | | 40 | 173 | |
| | | | | 50 | 178 | |
| | | | | 60 | 228 | |
| 761933 | CmsAmsUmsUmsGmsUmsUmsUmsUmsUmsUms GmsUmsCmsUmsUmsCmsCm | 55 | 72 | 10 | 123 | 17 |
| | | | | 20 | 141 | |
| | | | | 30 | 126 | |
| | | | | 40 | 131 | |
| | | | | 50 | 105 | |
| 759704 | Cmo Amo Umo Umo Gmo Umo Umo Umo Umo Umo Umo Gmo Umo Cmo Umo Um | 57 | 72 | 10 | 187 | 18 |
| | | | | 20 | 182 | |
| | | | | 30 | 189 | |
| | | | | 40 | 170 | |
| | | | | 50 | 243 | |
| | | | | 60 | 203 | |
| 761930 | C_mo A_mo U_mo U_mo G_mo U_mo U_mo U_mo U_mo U_mo U_mo G_mo U_mo C_mo U_mo U_mo C_mo C_mo G_mo U_m | 53 | 72 | 10 | 164 | 19 |
| | | | | 20 | 149 | |
| | | | | 30 | 218 | |
| | | | | 40 | 142 | |
| | | | | 50 | 219 | |
| | | | | 60 | 255 | |

| | | | | | | |
|---|---|---|---|---|---|---|
| Subscripts: "m" indicates a 2'-O-methyl modification, "o" indicates a phosphodiester internucleoside linkage, and "s" indicates a phosphorothioate internucleoside linkage. | | | | | | |

### Example 7: Effect of antisense oligonucleotide targeting the uORF of human SFXN3 on SFXN3 protein expression

Human Sideroflexin 3 (SFXN3) mRNA (GENBANK accession number NM_030971.3, designated herein as SEQ ID NO: 20) comprises an upstream open reading frame. An antisense oligonucleotide designed to target the start codon of the uORF of human SFXN3 was tested for its effect on SFXN3 protein expression *in vitro.* The antisense oligonucleotide, described in Table 9, was uniformly modified in order to avoid inducing cleavage of the target RNA. The start and stop sites listed in Table 9 indicate the positions on SEQ ID NO: 20 that the antisense oligonucleotide targets. The targeted uORF begins at position 388. HeLa cells were transfected with Lipofectamine RNAiMAX (Life Technologies) and an antisense oligonucleotide at a concentration listed in Table 9 or were untreated as a control. Ten hours after transfection, the cells were lysed, and SFXN3 mRNA and protein expression were analyzed by RT-PCR and western blot, respectively. The western blot was performed with an SFXN3 antibody purchased from Abcam (catalog # ab181163) and quantified using Image J. The results are shown in Table 9 below as the percent protein expression relative to untreated control cells ("UTC") following normalization to the loading control (Ku70, detected with Abcam antibody, catalog # ab3114). SFXN3 mRNA levels normalized to Ribogreen are also shown. The results show that an antisense oligonucleotide targeting the start codon of the uORF of human SFXN3 increased SFXN3 protein expression. SFXN3 mRNA levels did not increase, indicating that the antisense oligonucleotide increased SFXN3 expression via increased translation.

**Table 9: SFXN3 expression**

| Isis No. | Sequence | Start Site | Stop Site | Concentration (nM) | Protein (% UTC) | mRNA (% UTC) | SEQ ID NO. |
|---|---|---|---|---|---|---|---|
| | | | | 10 | 100 | 106 | |
| | | | | 20 | 120 | 102 | |
| | CmoAmoUmoCmoAmoCmoGmo CmoGmo GmoGmo AmoCmoGmoUmoCm | | | 30 | 124 | 97 | |
| 759677 | | 375 | 390 | 40 | 126 | 94 | 21 |
| | | | | 60 | 134 | 109 | |
| | | | | 80 | 133 | 103 | |
| | | | | 100 | 125 | 107 | |

| | | | | | | | |
|---|---|---|---|---|---|---|---|
| Subscripts: "m" indicates a 2'-O-methyl modification, "o" indicates a phosphodiester internucleoside linkage. | | | | | | | |

### Example 8: Effect of antisense oligonucleotide targeting the uORF of mouse MRPL11 on MRPL11 protein expression

Mouse Mitochondrial Ribosomal protein L11 (MRPL11) mRNA (GENBANK accession number NM_025553.4, designated herein as SEQ ID NO: 22) comprises an upstream open reading frame. An antisense oligonucleotide designed to target the start codon of the uORF of mouse MRPL11 was tested for its effect on MRPL11 protein expression *in vitro.* The antisense oligonucleotide, described in Table 10, was uniformly modified in order to avoid inducing cleavage of the target RNA. The start and stop sites listed in Table 10 indicate the positions on SEQ ID NO: 22 that the antisense oligonucleotide targets. The targeted uORF begins at position 24. bEND cells were transfected with Lipofectamine RNAiMAX (Life Technologies) and an antisense oligonucleotide at a concentration listed in Table 10 or were untreated as a control. Ten hours after transfection, the cells were lysed, and MRPL11 mRNA and protein expression were analyzed by RT-PCR and western blot, respectively. The western blot was performed with an MRPL11 antibody purchased from Abcam (catalog # ab2066s) and quantified using Image J. The results are shown in Table 10 below as the percent protein expression relative to untreated control cells ("UTC") following normalization to the loading control (GAPDH, detected with Santa Cruz Biotechnology antibody, catalog # sc-32233). MRPL11 mRNA levels normalized to Ribogreen are also shown. The results show that an antisense oligonucleotide targeting the start codon of the uORF of mouse MRPL11 increased MRPL 11 protein expression. MRPL11 mRNA levels did not increase, indicating that the antisense oligonucleotide increased MRPL11 expression via increased translation.

**Table 10: MRPL11 expression**

| Isis No. | Sequence | Start Site | Stop Site | Concentration (nM) | Protein (% UTC) | mRNA (% UTC) | SEQ ID NO. |
|---|---|---|---|---|---|---|---|
| | CmoAmoUmoUmoUmoUmoGmo GmoGmo UmoCmo AmoGmoAmoGmoGmoUmoGm | | | 5 | 95 | 93 | |
| | | | | 10 | 146 | 95 | |
| 773534 | | 9 | 26 | 20 | 137 | 96 | 23 |
| | | | | 40 | 134 | 109 | |
| | | | | 80 | 125 | 104 | |

| | | | | | | | |
|---|---|---|---|---|---|---|---|
| Subscripts: "m" indicates a 2'-O-methyl modification, "o" indicates a phosphodiester internucleoside linkage. | | | | | | | |

### Example 9: Effects of antisense oligonucleotides targeting the uORF of human THPO on THPO protein expression

Human Thrombopoietin (THPO) mRNA (GENBANK accession number NM_000460.3, designated herein as SEQ ID NO: 24) comprises seven upstream open reading frames. An antisense oligonucleotide designed to target the start codon of the last uORF of human THPO was tested for its effect on THPO protein expression *in vitro.* The antisense oligonucleotide, described in Table 11, was uniformly modified in order to avoid inducing cleavage of the target RNA. The start and stop sites listed in Table 11 indicate the positions on SEQ ID NO: 24 that the antisense oligonucleotide targets. The targeted uORF begins at position 210. Hep3B cells were transfected with Lipofectamine RNAiMAX (Life Technologies) and an antisense oligonucleotide at a concentration listed in Table 11 or cells were untreated as a control. Ten hours after transfection, medium was changed to serum-free medium and cells were incubated for an additional 12 hr. Proteins from the medium were precipitated using trichloroacetic acid, and THPO protein expression was analyzed by western blot using a THPO antibody purchased from Abcam (catalog # ab196026) and quantified using Image J. The results are shown in Table 11 below as the percent protein expression relative to untreated control cells ("UTC") following normalization to the loading control (Transferrin). The results show that an antisense oligonucleotide targeting the start codon of the uORF of human THPO increased THPO protein expression.

**Table 11: THPO expression**

| Isis No. | Sequence | Start Site | Stop Site | Concentration (nM) | Protein (% UTC) | SEQ ID NO. |
|---|---|---|---|---|---|---|
| | CmoAmoUmoGmoGmoAmoGmo GmoCmo GmoGmo CmoUmoUmoAmoGmoGmoCm | | | 5 | 155 | |
| | | | | 10 | 176 | |
| 806734 | | 195 | 212 | 20 | 167 | 25 |
| | | | | 40 | 172 | |
| | | | | 60 | 130 | |

| | | | | | | |
|---|---|---|---|---|---|---|
| Subscripts: "m" indicates a 2'-O-methyl modification, "o" indicates a phosphodiester internucleoside linkage. | | | | | | |

### Example 10: Effects of mismatched antisense oligonucleotides targeting the uORF of RNase H1 on RNase H1 protein expression

Antisense oligonucleotides designed to target the start codon of the uORF of human RNase H1 with two mismatches between the antisense oligonucleotide and the target sequence were tested for their effects on RNase H1 expression *in vitro.* These antisense oligonucleotides, described in Table 12, were uniformly modified in order to avoid inducing cleavage of the target RNA. The start and stop sites listed in Table 12 indicate the positions on SEQ ID NO: 1 that the antisense oligonucleotides target, and the mismatched nucleotides are bolded. The targeted uORF begins at position 86. HeLa cells were transfected with Lipofectamine RNAiMAX (Life Technologies) and an antisense oligonucleotide at a final concentration of 25 nM or were mock transfected as a control. Twenty-four hours after transfection, the cells were lysed, and expression of RNase H1 was analyzed by western blot as described in Example 1. The results are shown in Table 12 as percent protein levels relative to mock transfected cells following normalization to the Ku70 loading control. The results show that antisense oligonucleotides with mismatches to the uORF start codon or near to the uORF start codon had little to no effect on RNase H1 protein expression, whereas antisense oligonucleotides with mismatches further away from the uORF start codon increased RNase H1 protein expression.

**Table 12: RNase H1 expression**

| Isis No. | Sequence | Start Site | Stop Site | RNase H1 protein (% mock) | SEQ ID NO. |
|---|---|---|---|---|---|
| 773519 | GmoUmoUmoUmoUmoCmoGmoAmoCmoUm oCmoCmoCmoGmoGmoCm | 73 | 88 | 96 | 26 |
| 773520 | CmoAmoAmoAmoUmoCmoGmoAmoCmoUm | 73 | 88 | 82 | 27 |
| | oCmoCmoCmoGmoGmoCm | | | | |
| 773521 | CmoAmoUmoUmoAmoGmoGmoAmoCmoUm oCmoCmoCmoGmoGmoCm | 73 | 88 | 103 | 28 |
| 773522 | CmoAmoUmoUmoUmoCmoCmoUmoCmoUm oCmoCmoCmoGmoGmoCm | 73 | 88 | 89 | 29 |
| 773523 | CmoAmoUmoUmoUmoCmoGmoAmoGmoAm oCmoCmoCmoGmoGmoCm | 73 | 88 | 121 | 30 |
| 773524 | CmoAmoUmoUmoUmoCmoGmoAmoCmoUmo GmoGmoCmoGmoGmoCm | 73 | 88 | 158 | 31 |
| 773525 | CmoAmoUmoUmoUmoCmoGmoAmoCmoUmo CmoCmoGmoCmoGmoCm | 73 | 88 | 150 | 32 |
| 773526 | CmoAmoUmoUmoUmoCmoGmoAmoCmoUmo CmoCmoCmoGmoCmoGm | 73 | 88 | 153 | 33 |

| | | | | | |
|---|---|---|---|---|---|
| Subscripts: "m" indicates a 2'-O-methyl modification, and "o" indicates a phosphodiester internucleoside linkage. | | | | | |

### Example 11: Effects of antisense oligonucleotides comprising various modifications

Antisense oligonucleotides targeting the start codon of the uORF in human RNase H1 (SEQ ID NO: 1) or mouse LRPPRC (SEQ ID NO: 2) were designed with various lengths and with various modifications to the internucleoside linkages and to the sugars. These antisense oligonucleotides were tested for their effects on target protein expression *in vitro.* HEK293 cells (RNase H1 targeting oligonucleotides) or MHT cells (LRPPRC targeting oligonucleotides) were transfected with Lipofectamine RNAiMAX (Life Technologies) and an antisense oligonucleotide at a concentration listed in the tables below. Ten hours after transfection, the cells were lysed, and target protein expression was analyzed by western blot as described in Example 1 (for RNase H1) or Example 6 (for LRPPRC). The results are shown in the tables below as the percent protein expression relative to untreated control cells ("UTC") following normalization to the loading control (Ku70, γ-tubulin, Annexin A2, or a non-specific band detected by the primary antibody). The results show that antisense oligonucleotides of various lengths and with modified internucleoside linkages and various 2'-modifications and bicyclic nucleosides increased target protein expression.

**Table 13: RNase H1 expression**

| Isis No. | Sequence | Start Site | Stop Site | Length | Concentra tion (nM) | Protein (% mock) | SEQ ID NO. |
|---|---|---|---|---|---|---|---|
| 783683 | CmsAmsUmsUmsUmsCmsGmsAmsCmsUms Cms Cm | 77 | 88 | 12 | 20 | 191 | 34 |
| 783681 | CmsAmsUmsUmsUmsCmsGmsAmsCmsUms Cms CmsCmsGm | 75 | 88 | 14 | 20 | 178 | 35 |
| 783682 | CmsAmsUmsUmsUmsCmsGmsAmsCmsUms Cms CmsCmsGmsGmsCm | 73 | 88 | 16 | 20 | 186 | 3 |
| 783679 | CmsAmsUmsUmsUmsCmsGmsAmsCmsUms Cms CmsCmsGmsGmsCmsCmsCm | 71 | 88 | 18 | 20 | 149 | 15 |
| 783680 | CmsAmsUmsUmsUmsCmsGmsAmsCmsUms Cms CmsCmsGmsGmsCmsCmsCmsΛmsGm | 69 | 88 | 20 | 20 | 83 | 16 |

| | | | | | | | |
|---|---|---|---|---|---|---|---|
| Subscripts: "m" indicates a 2'-O-methyl modification, and "s" indicates a phosphorothioate internucleoside linkage. | | | | | | | |

**Table 14: RNase H1 expression**

| Isis No. 783679 concentration (nM) | Protein (% UTC) |
|---|---|
| 5 | 140 |
| 10 | 179 |
| 20 | 186 |
| 40 | 174 |
| 60 | 195 |

**Table 15: RNase H1 expression**

| Isis No. | Sequence | Start Site | Stop Site | Length | Concentra tion (nM) | Protein (% mock) | SEQ ID NO. |
|---|---|---|---|---|---|---|---|
| 783674 | ^{m}CₑₛAₑₛTₑₛTₑₛTₑₛ^{m}CₑₛGₑₛAₑₛ^{m}CₑₛTₑₛ^{m}Cₑₛ^{m}Cₑ | 77 | 88 | 12 | 20 | 70 | 36 |
| 783673 | ^mC_es A_es T_es T_es T_es ^mC_es G_es A_es ^mC_es T_es ^mC_es ^mC_es ^mC_es G_e | 75 | 88 | 14 | 20 | 104 | 37 |
| 759304 | ᵐCesAesTesTesTesᵐCesGesAesᵐCesTesᵐCes ᵐCesᵐCesGesGesᵐCe | 73 | 88 | 16 | 20 | 136 | 38 |
| 773517 | mCesAesTesTesTesmCesGesAesmCesTesmCes mCesmCesGesGesmCesmCesmCe | 71 | 88 | 18 | 20 | 178 | 39 |
| 773516 | mCesAesTesTesTesmCesGesAesmCesTesmCes mCesmCesGesGesmCesmCesmCesAesGe | 69 | 88 | 20 | 20 | 122 | 40 |

| | | | | | | | |
|---|---|---|---|---|---|---|---|
| Subscripts: "e" indicates a 2'-O-methoxyethyl modification, and "s" indicates a phosphorothioate internucleoside linkage. Superscript "m" in front of a "C" indicates a 5'-methylcytosine. | | | | | | | |

**Table 16: RNase H1 expression**

| Isis No. 759304 concentration (nM) | Protein (% UTC) |
|---|---|
| 5 | 137 |
| 10 | 211 |
| 20 | 218 |
| 40 | 208 |
| 60 | 193 |

**Table 17: RNase H1 expression**

| Isis No. | Sequence | Start Site | Stop Site | Length | Concentra tion (nM) | Protein (% mock) | SEQ ID NO. |
|---|---|---|---|---|---|---|---|
| 783678 | mCcoAcoTcoTcoTcomCcoGcoAcomCcoTcomCco mCe | 77 | 88 | 12 | 5 | 187 | 36 |
| 783677 | mCeoAeoTeoTeoTeomCeoGeoAeomCeoTeomCeo mCeomCeoGe | 75 | 88 | 14 | 5 | 144 | 37 |
| 759388 | ᵐCₑₒAₑₒTₑₒTₑₒTₑₒᵐCₑₒGₑₒAₑₒᵐCₑₒTₑₒᵐCₑₒ ᵐCₑₒᵐCₑₒGₑₒGₑₒᵐCₑ | 73 | 88 | 16 | 5 | 148 | 38 |
| 783676 | mCeoAeoTeoTeoTeomCeoGeoAeomCeoTeomCeo mCeomCeoGeoGeomCeomCeomCe | 71 | 88 | 18 | 5 | 101 | 39 |
| 783675 | mCeoAeoTeoTeoTeomCeoGeoAeomCeoTeomCeo mCeomCeoGeoGeomCeomCeomCeoAeoGe | 69 | 88 | 20 | 5 | 84 | 40 |

| | | | | | | | |
|---|---|---|---|---|---|---|---|
| Subscripts: "e" indicates a 2'-O-methoxyethyl modification, and "s" indicates a phosphorothioate internucleoside linkage. Superscript "m" in front of a "C" indicates a 5'-methylcytosine. | | | | | | | |

**Table 18: RNase H1 expression**

| Isis No. | Sequence | Start Site | Stop Site | Concentration (nM) | Protein (% UTC) | SEQ ID NO. |
|---|---|---|---|---|---|---|
| | CfoAfoUfoUfoUfoCfoGfoAfoCfo UfoCfoCfoCfoGfoGfoCf | | | 5 | 115 | |
| | | | | 10 | 126 | |
| 766733 | | 73 | 88 | 20 | 137 | 3 |
| | | | | 40 | 134 | |
| | | | | 60 | 127 | |

| | | | | | | |
|---|---|---|---|---|---|---|
| Subscripts: "f' indicates a 2'-fluoro modification, "o" indicates a phosphodiester internucleoside linkage. | | | | | | |

**Table 19: RNase H1 expression**

| Isis No. | Sequence | Start Site | Stop Site | Concentration (nM) | Protein (% UTC) | SEQ ID NO. |
|---|---|---|---|---|---|---|
| | mCesAesTesTesTesmCesGesAes mCesTesmCes mCesCfsGfsGfsCf | | | 5 | 150 | |
| | | | | 10 | 125 | |
| 768080 | | 73 | 88 | 20 | 100 | 38 |
| | | | | 40 | 81 | |
| | | | | 60 | 78 | |

| | | | | | | |
|---|---|---|---|---|---|---|
| Subscripts: "e" indicates a 2'-O-methoxyethyl modification, "f' indicates a 2'-fluoro modification, "s" indicates a phosphorothioate internucleoside linkage. Superscript "m" in front of a "C" indicates a 5'-methylcytosine. | | | | | | |

**Table 20: RNase H1 expression**

| Isis No. | Sequence | Start Site | Stop Site | Concentration (nM) | Protein (% UTC) | SEQ ID NO. |
|---|---|---|---|---|---|---|
| | mCesAesTesTesTesmCesGesAes mCesTesmCesmCesmCes GksGksmCk | | | 5 | 126 | |
| | | | | 10 | 151 | |
| 766741 | | 73 | 88 | 20 | 122 | 38 |
| | | | | 40 | 121 | |
| | | | | 80 | 62 | |

| | | | | | | |
|---|---|---|---|---|---|---|
| Subscripts: "e" indicates a 2'-O-methoxyethyl modification, "k" indicates a cEt bicyclic nucleoside, "s" indicates a phosphorothioate internucleoside linkage. Superscript "m" in front of a "C" indicates a 5'-methylcytosine. | | | | | | |

**Table 21: RNase H1 expression**

| Isis No. | Sequence | Start Site | Stop Site | Concentra tion (nM) | Protein (% mock) | SEQ ID NO. |
|---|---|---|---|---|---|---|
| 806735 | CmsAmsUmsUmsUmsCmsGmsAmsCmsUmsCms CmsCmsGmsGksmCk | 73 | 88 | 20 | 152 | 3 |
| 806736 | CmsAmsUmsUmsUmsCmsGmsAmsCmsUmsCms CmsCmsGksGksmCk | 73 | 88 | 20 | 165 | 3 |
| 806737 | CmsAmsUmsUmsUmsCmsGmsAmsCmsUmsCms CmsmCksGksGksmCk | 73 | 88 | 20 | 183 | 3 |
| 806738 | CmsAmsUmsUmsUmsCmsGmsAmsCmsUmsCms mCksmCksGksGksmCk | 73 | 88 | 20 | 145 | 3 |

| | | | | | | |
|---|---|---|---|---|---|---|
| Subscripts: "m" indicates a 2'-O-methyl modification, "k" indicates a cEt bicyclic nucleoside, and "s" indicates a phosphorothioate internucleoside linkage. Superscript "m" in front of a "C" indicates a 5'-methylcytosine. | | | | | | |

**Table 22: LRPPRC expression**

| Isis No. | Sequence | Start Site | Stop Site | Concentra tion (nM) | Protein (% mock) | SEQ ID NO. |
|---|---|---|---|---|---|---|
| 806739 | CmsAmsUmsUmsGmsUmsUmsUmsUmsUmsUms GmsUmsCmsUmsUmsmCksmCk | 55 | 72 | 20 | 149 | 17 |
| 806740 | CmsAmsUmsUmsGmsUmsUmsUmsUmsUmsUms GmsUmsCmsUmsTksmCksmCk | 55 | 72 | 20 | 191 | 41 |
| 806741 | CmsAmsUmsUmsGmsUmsUmsUmsUmsUmsUms GmsUmsCmsTksTksmCksmCk | 55 | 72 | 20 | 212 | 42 |
| 806742 | CmsAmsUmsUmsGmsUmsUmsUmsUmsUmsUms GmsUmsmCksTksTksmCksmCk | 55 | 72 | 20 | 213 | 42 |

| | | | | | | |
|---|---|---|---|---|---|---|
| Subscripts: "m" indicates a 2'-O-methyl modification, "k" indicates a cEt bicyclic nucleoside, and "s" indicates a phosphorothioate internucleoside linkage. Superscript "m" in front of a "C" indicates a 5'-methylcytosine. | | | | | | |

### Example 12: Effects of antisense oligonucleotides targeting the uORF of mouse LRPPRC in vivo

Isis No. 761933 (see Example 6) was tested for its effect on LRPPRC protein expression *in vivo.* Groups of three or four male, seven week old BALB/c mice were systemically administered Isis No. 761933, at a dose listed in the tables below, or saline by subcutaneous injection. Animals were sacrificed (Table 23) or received a second dose (Table 24) 48 hours later. Animals that received a second dose were sacrificed 48 hours after the second dose. Liver homogenates were prepared, and LRPPRC protein expression was analyzed by western blot, as described in Example 6. The results are shown in the tables below as the percent expression relative to saline treated animals following normalization to the loading control (GAPDH or hnRNP K). The results show that LRPPRC protein expression was increased *in vivo* following treatment with an antisense oligonucleotide targeting the LRPPRC uORF.

**Table 23: LRPPRC expression following single dose in vivo**

| Isis No. 761933 dose (mg/kg) | Protein (% UTC) |
|---|---|
| 75 | 137 |

**Table 24: LRPPRC expression following two doses in vivo**

| Isis No. 761933 dose (mg/kg) | Protein (% UTC) |
|---|---|
| 3.125 | 109 |
| 6.25 | 124 |
| 12.5 | 136 |
| 25 | 183 |
| 50 | 169 |
| 100 | 109 |
| 200 | 98 |

### Example 13: Effects of antisense oligonucleotides targeting the uORF of mouse THPO in vivo

Mouse Thrombopoietin (THPO) mRNA (GENBANK accession number NM_009379.2, designated herein as SEQ ID NO: 43) comprises an upstream open reading frame. Isis No. 809793 (see the table below) was designed to target the start codon of the uORF of mouse THPO and tested for its effect on THPO protein expression *in vivo.* The start and stop sites listed in Table 25 indicate the positions on SEQ ID NO: 43 that the antisense oligonucleotide targets. Groups of three mice were systemically administered Isis No. 809793, at a dose listed in the table below, or saline by subcutaneous injection. The animals were sacrificed 48 hours later, and serum and bone marrow was collected. Serum THPO protein expression was analyzed by western blot, as described in Example 9, and bone marrow THPO mRNA expression was analyzed by RT-PCR. Bone marrow THPO mRNA levels were unaffected by the antisense oligonucleotide treatment (data not shown). The results shown in the table below are the average of two independent experiments and are shown as the percent expression relative to saline treated animals following normalization to the loading control (Transferrin). The results show that THPO protein expression was increased *in vivo* following treatment with an antisense oligonucleotide targeting the THPO uORF.

**Table 25: Mouse THPO expression**

| Isis No. | Sequence | Start Site | Stop Site | Dose (mg/kg) | THPO protein (% mock) | SEQ ID NO. |
|---|---|---|---|---|---|---|
| | CmsAmsUmsGmsGmsAmsGmsGmsCmsGms GmsCmsUmsUmsGmsAmsGm | | | 12.5 | 146 | |
| 809793 | | 309 | 325 | 25 | 457 | 44 |
| | | | | 50 | 240 | |
| | | | | 75 | 244 | |

| | | | | | | |
|---|---|---|---|---|---|---|
| Subscripts: "m" indicates a 2'-O-methyl modification, and "s" indicates a phosphorothioate internucleoside linkage. | | | | | | |

### Example 14: Inhibition of a uORF targeting oligonucleotide with a complementary oligonucleotide

In order to test whether increased translation that is mediated by an oligonucleotide targeting a uORF could be reversed, the effect of an oligonucleotide complementary to the uORF targeting oligonucleotide was tested *in vitro.* HeLa cells were transfected with 20 nM of Isis No. 761909, as described in Example 1. Five hours later, the cells were transfected with a concentration of Isis No. 761929, which is complementary to Isis No. 761909, listed in the table below. Five hours after the second transfection, cells were lysed and RNase H1 protein expression was analyzed as described in Example 1, with γ-tubulin used as the loading control. The results are shown in the table below as the percent expression relative to mock transfected cells following normalization to the γ-tubulin loading control. The results show that an oligonucleotide targeted to a uORF targeting oligonucleotide blocked the uORF targeting oligonucleotide's ability to increase translation in a dose dependent manner.

**Table 26: RNase H1 expression**

| Isis No. | Sequence | Concentration 761909 (nM) | Concentration 761929 (nM) | RNase H1 protein (% mock) | SEQ ID NO. |
|---|---|---|---|---|---|
| | GmoCmoCmoGmoGmoGmoAmo GmoUmoCmoGmoAmoAmoAmo UmoGm | 20 | 0 | 185 | |
| | | 20 | 10 | 161 | |
| 761929 | | 20 | 20 | 120 | 45 |
| | | 20 | 30 | 103 | |
| | | 20 | 40 | 97 | |

| | | | | | |
|---|---|---|---|---|---|
| Subscripts: "m" indicates a 2 -O-methyl modification and "o" indicates a phosphodiester internucleoside linkage. | | | | | |

### Example 15: Inhibition of a uORF targeting oligonucleotide by globally blocking translation

In order to test whether increases in protein expression mediated by antisense oligonucleotides targeting a uORF are due to increased protein stability, translation was blocked following transfection of the oligonucleotide. HeLa cells were transfected with Isis No. 761909 (see Example 1) or mock transfected. Twelve hours later, cells were treated with DMSO or 15 µg/ml cycloheximide at 37 °C. Cells were then collected at various time points and cell lysates were prepared and subjected to western analysis, as described in Example 1. A duplicate SDS-PAGE gel was silver-stained and served as loading control. RNase H1 protein levels were calculated relative to mock transfected cells following normalization to the loading control. The results (data not shown) showed that the rates of reduction of RNase H1 protein levels following cycloheximide treatment were similar for mock and Isis No. 761909 transfected cells. Thus, these results, along with the lack of increase in mRNA shown in several above examples, show that increases in protein expression mediated by antisense oligonucletoides targeting a uORF are due to increased protein translation.

### Example 16: Effect of a uORF targeting antisense oligonucleotide on nascent protein translation

In order to further confirm that antisense oligonucleotides targeting a uORF mediate an increase in translation of new protein, pulse-chase labeling and immunoprecipitation of LRPPRC was performed. MHT cells were transfected with Isis No. 761930 for 7 hours, and incubated with pre-warmed medium lacking methionine (Invitrogen, RPMI 1640) at 37 °C for 30 minutes. Cells were then pulse-labeled with 35 µCi/ml S³⁵-methionine in RPMI1640 medium for 20 minutes and chased with 1 mM methionine for 40 minutes. Cells were washed with cold PBS containing 10 µg/ml cycloheximide and cell lysate were prepared using IP buffer (Life Technologies). Immunoprecipitation was conducted using anti-LRPPRC antibody (Proteintech) at 4_{°}C for 3 hours. After 4 washes, the precipitated proteins were analyzed on a 4-12% SDS-PAGE gel, transferred to a membrane, and visualized by autoradiography. The LRPPRC band in the immunoprecipitate from the mock treated cells was significantly lighter than in the immunoprecipitate from the Isis No. 761930 treated cells (data not shown), further confirming that antisense oligonucleotides targeting a uORF increase protein expression via increasing target protein translation. Furthermore, the lysate inputs for both the mock and Isis No. 761930 transfected cells were also analyzed via autoradioagraphy and were very similar, indicating that the increased translation was target specific.

### Example 17: Effect of an antisense oligonucleotide targeting the uORF of RNase H1 on RNase H1 activity

In order to test whether the increase in RNase H1 protein levels mediated by antisense oligonucleotides targeting a RNase H1 uORF results in an increase in RNase H1 activity, cells were first transfected with Isis No. 761909 (see Example 1) or a control oligonucleotide that does not target RNase H1, Isis No. 759704, then transfected with an antisense oligonucleotide targeting U16 snoRNA or NCL1 mRNA. Ten hours after the first transfection, the cells were reseeded at approximately 50% confluency. Cells were transfected a second time 14 hours later with Isis No. 462026 (U16) or Isis No. 110074 (NCL1) for an additional 4 hours. Cells were then collected, lysed, and U16 snoRNA or NCL1 mRNA levels were analyzed by RT-qPCR using TaqMan primer probe sets. The primer probe set sequences used were: Forward: 5'-CTTGCAATGATGTCGTAATTTGC -3', SEQ ID NO: 46, Reverse: 5'-TCGTCAACCTTCTGTACCAGCTT -3', SEQ ID NO: 47, and Probe: 5'-TTACTCTGTTCTCAGCGACAGTTGCCTGC -3', SEQ ID NO: 48 for U16; and Forward: 5'-GCTTGGCTTCTTCTGGACTCA -3', SEQ ID NO: 49, Reverse: 5'- TCGCGAGCTTCACCATGA -3', SEQ ID NO: 50, and Probe: 5'- CGCCACTTGTCCGCTTCACACTCC-3', SEQ ID NO: 51 for NCL1. The results are shown in the tables below as the percent RNA levels relative to untreated control cells that were not transfected with any oligonucleotide, normalized to total RNA as measured using Ribogreen. The results show that Isis No. 761909 targeting a RNase H1 uORF increased the antisense activity of U16 and NCl1 antisense oligonucleotides.

**Table 27: U16 snoRNA expression**

| 1^{st} ASO | | | SEQ ID No. | 2^{nd} ASO | | | mRNA (%) | SEQ ID No. |
|---|---|---|---|---|---|---|---|---|
| Isis No. | Sequence | Concentration (nM) | | Isis No. | Sequence | Concentration (nM) | | |
| Control 759704 | Cmo Amo Umo Umo Gmo Umo Umo Umo Umo Umo Umo Gmo Umo Cmo Umo Um | 20 | 18 | 462026 | mCcsAcsGcsmCcsAcsG dsGdsmCdsAdsAdsmCds TdsGdsTdsmCdsGesmC esTesGesAe | 1.25 | 97 | 52 |
| | | 20 | | | | 2.5 | 98 | |
| | | 20 | | | | 5 | 97 | |
| | | 20 | | | | 10 | 83 | |
| | | 20 | | | | 20 | 75 | |
| uORF 761909 | CmoAmoUmoUmoU moCmoGmoAmoCmo UmoCmoCmoCmoG moGmoCm | 20 | 3 | 462026 | mCcsAcsGcsmCcsAcsG dsGdsmCdsAdsAdsmCds TdsGdsTdsmCdsGesmC esTesGesAe | 1.25 | 90 | 52 |
| | | 20 | | | | 2.5 | 76 | |
| | | 20 | | | | 5 | 70 | |
| | | 20 | | | | 10 | 61 | |
| | | 20 | | | | 20 | 49 | |

| | | | | | | | | |
|---|---|---|---|---|---|---|---|---|
| Subscripts: "e" indicates a 2 -O-methoxyethyl modification, "d" indicates a 2 -deoxynucleoside, and "s" indicates a phosphorothioate internucleoside linkage. Superscript "m" in front of a "C" indicates a 5'-methylcytosine. | | | | | | | | |

**Table 28: NCL1 mRNA expression**

| 1^{st} ASO | | | SEQ ID No. | 2^{nd} ASO | | | mRNA (%) | SEQ ID No. |
|---|---|---|---|---|---|---|---|---|
| Isis No. | Sequence | Concentration (nM) | | Isis No. | Sequence | Concentration (nM) | | |
| Control 759704 | Cmo Amo Umo Umo Gmo Umo Umo Umo Umo Umo Umo Gmo Umo Cmo Umo Um | 20 | 18 | 110074 | GesTesmCesAesTesmCd sGdsTdsmCdsAdsTds mCdsmCdsTdsmCdsAes TesmCesAesTe | 1.25 | 96 | 53 |
| | | 20 | | | | 2.5 | 95 | |
| | | 20 | | | | 5 | 96 | |
| | | 20 | | | | 10 | 79 | |
| | | 20 | | | | 20 | 45 | |
| uORF 761909 | CmoAmoUmoUmoU moCmoGmoAmoCmo UmoCmoCmoCmoG moGmoCm | 20 | 3 | 110074 | GesTesmCesAesTesmCd sGdsTdsmCdsAdsTds mCdsmCdsTdsmCdsAes TesmCesAesTe | 1.25 | 93 | 53 |
| | | 20 | | | | 2.5 | 91 | |
| | | 20 | | | | 5 | 87 | |
| | | 20 | | | | 10 | 62 | |
| | | 20 | | | | 20 | 32 | |

| | | | | | | | | |
|---|---|---|---|---|---|---|---|---|
| Subscripts: "e" indicates a 2'-O-methoxyethyl modification, "d" indicates a 2'-deoxynucleoside, and "s" indicates a phosphorothioate internucleoside linkage. Superscript "m" in front of a "C" indicates a 5'-methylcytosine. | | | | | | | | |

### Example 18: Effects of antisense oligonucleotides targeting the 5'-untranslated region of RNase H1

Antisense oligonucleotides designed to target the 5'-untranslated region upstream of the uORF of human RNase H1 were tested for their effects on RNase H1 protein expression *in vitro.* The start and stop sites listed in the tables below indicate the positions on SEQ ID NO: 1 that the antisense oligonucleotides target. HEK 293 cells were transfected with 20 nM antisense oligonucleotide or were mock transfected as a control. Ten hours after transfection, RNase H1 protein expression was analyzed as described in Example 1. The results are shown in the tables below as percent protein expression relative to mock transfected cells following normalization to the GAPDH loading control. The results show that many antisense oligonucleotides targeting the 5'-untranslated region upstream of the RNase H1 uORF induced translation of the target.

**Table 29: RNase H1 expression**

| Isis No. | Sequence | Start Site | Stop Site | Protein (% mock) | SEQ ID NO. |
|---|---|---|---|---|---|
| 761772 | Amo Amo Gmo Amo Umo Gmo Amo Cmo Gmo Cmo Amo Cmo Gmo Umo Cmo Um | 1 | 16 | 142 | 54 |
| 761773 | Gmo Gmo Amo Amo Gmo Amo Umo Gmo Amo Cmo Gmo Cmo Amo Cmo Gmo Um | 3 | 18 | 155 | 55 |
| 761774 | Cmo Gmo Gmo Gmo Amo Amo Gmo Amo Umo Gmo Amo Cmo Gmo Cmo Amo Cm | 5 | 20 | 179 | 56 |
| 761775 | Cmo Gmo Cmo Gmo Gmo Gmo Amo Amo Gmo Amo Umo Gmo Amo Cmo Gmo Cm | 7 | 22 | 259 | 57 |

**Table 30: RNase H1 expression**

| Isis No. | Sequence | Start Site | Stop Site | Protein (% mock) | SEQ ID NO. |
|---|---|---|---|---|---|
| 761778 | Cmo Amo Cmo Cmo Gmo Gmo Cmo Gmo Cmo Gmo Gmo Gmo Amo Amo Gmo Am | 13 | 28 | 132 | 58 |
| 761779 | Gₘₒ Uₘₒ Cₘₒ Aₘₒ Cₘₒ Cₘₒ Gₘₒ Gₘₒ Cₘₒ Gₘₒ Cₘₒ Gₘₒ Gₘₒ Gₘₒ Aₘₒ Aₘ | 15 | 30 | 164 | 59 |
| 761780 | Cmo Cmo Gmo Umo Cmo Amo Cmo Cmo Gmo Gmo Cmo Gmo Cmo Gmo Gmo Gm | 17 | 32 | 182 | 60 |
| 761781 | Umo Umo Cmo Cmo Gmo Umo Cmo Amo Cmo Cmo Gmo Gmo Cmo Gmo Cmo Gm | 19 | 34 | 226 | 61 |
| 761782 | Amo Cmo Umo Umo Cmo Cmo Gmo Umo Cmo Amo Cmo Cmo Gmo Gmo Cmo Gm | 21 | 36 | 229 | 62 |
| 761783 | Gmo Cmo Amo Cmo Umo Umo Cmo Cmo Gmo Umo Cmo Amo Cmo Cmo Gmo Gm | 23 | 38 | 259 | 63 |
| 761784 | Cmo Cmo Gmo Cmo Amo Cmo Umo Umo Cmo Cmo Gmo Umo Cmo Amo Cmo Cm | 25 | 40 | 237 | 64 |

**Table 31: RNase H1 expression**

| Isis No. | Sequence | Start Site | Stop Site | Protein (% mock) | SEQ ID NO. |
|---|---|---|---|---|---|
| 761785 | Cmo Amo Cmo Cmo Gmo Cmo Amo Cmo Umo Umo Cmo Cmo Gmo Umo Cmo Am | 27 | 42 | 170 | 65 |
| 761786 | Amo Amo Cmo Amo Cmo Cmo Gmo Cmo Amo Cmo Umo Umo Cmo Cmo Gmo Um | 29 | 44 | 186 | 66 |
| 761787 | Uₘₒ Cₘₒ Aₘₒ Aₘₒ Cₘₒ Aₘₒ Cₘₒ Cₘₒ Gₘₒ Cₘₒ Aₘₒ Cₘₒ Uₘₒ Uₘₒ Cₘₒ Cₘ | 31 | 46 | 197 | 67 |
| 761788 | Gmo Cmo Umo Cmo Amo Amo Cmo Amo Cmo Cmo Gmo Cmo Amo Cmo Umo Um | 33 | 48 | 210 | 68 |
| 761789 | Gmo Cmo Gmo Cmo Umo Cmo Amo Amo Cmo Amo Cmo Cmo Gmo Cmo Amo Cm | 35 | 50 | 211 | 69 |
| 761790 | Cmo Gmo Gmo Cmo Gmo Cmo Umo Cmo Amo Amo Cmo Amo Cmo Cmo Gmo Cm | 37 | 52 | 202 | 70 |
| 761791 | Gmo Cmo Cmo Gmo Gmo Cmo Gmo Cmo Umo Cmo Amo Amo Cmo Amo Cmo Cm | 39 | 54 | 173 | 71 |
| 761792 | Cmo Cmo Gmo Cmo Cmo Gmo Gmo Cmo Gmo Cmo Umo Cmo Amo Amo Cmo Am | 41 | 56 | 120 | 72 |

**Table 32: RNase H1 expression**

| Isis No. | Sequence | Start Site | Stop Site | Protein (% mock) | SEQ ID NO. |
|---|---|---|---|---|---|
| 761776 | Gmo Gmo Cmo Gmo Cmo Gmo Gmo Gmo Amo Amo Gmo Amo Umo Gmo Amo Cm | 9 | 24 | 130 | 73 |
| 761794 | Cmo Gmo Amo Gmo Cmo Cmo Gmo Cmo Cmo Gmo Gmo Cmo Gmo Cmo Umo Cm | 45 | 60 | 92 | 74 |
| 761796 | Gmo Gmo Cmo Gmo Cmo Gmo Amo Gmo Cmo Cmo Gmo Cmo Cmo Gmo Gmo Cm | 49 | 64 | 94 | 75 |
| 761798 | Cmo Gmo Umo Gmo Gmo Gmo Cmo Gmo Cmo Gmo Amo Gmo Cmo Cmo Gmo Cm | 53 | 68 | 84 | 76 |
| 761800 | Cmo Cmo Amo Gmo Cmo Gmo Umo Gmo Gmo Gmo Cmo Gmo Cmo Gmo Amo Gm | 57 | 72 | 71 | 77 |

| | | | | | |
|---|---|---|---|---|---|
| Subscripts: "m" indicates a 2'-O-methyl modification, and "o" indicates a phosphodiester internucleoside linkage. | | | | | |

### Example 19: Effects of antisense oligonucleotides targeting the 5'-untranslated region of ACP1

Antisense oligonucleotides designed to target the 5'-untranslated region of cytoplasmic phosphotyrosine protein phosphatase (ACP1), which does not comprise a uORF, were tested for their effects on ACP1 protein expression *in vitro.* The antisense oligonucleotides target the human ACP1 mRNA (GENBANK accession number NM_004300.3, designated herein as SEQ ID NO: 78) and were designed to target at least one stem loop structure in the 5'-untranslated region. The start and stop sites listed in the table below indicate the positions on SEQ ID NO: 78 that the antisense oligonucleotides target. HEK 293 cells were transfected with a concentration of antisense oligonucleotide shown in the table below or were not transfected as a control. Ten hours after transfection, ACP1 protein expression was analyzed by western blot using an Abcam antibody to ACP1 (catalog # ab166896). The results are shown in the tables below as percent protein expression relative to untreated control cells following normalization to the p32 loading control. The results show that the antisense oligonucleotides targeting the 5'-untranslated region of human ACP1 induced translation of the target.

**Table 33: ACP1 expression**

| Isis No. | Sequence | Start Site | Stop Site | Concentration (nM) | Protein (% mock) | SEQ ID NO. |
|---|---|---|---|---|---|---|
| 812652 | Cmo Gmo Amo Cmo Gmo Cmo Gmo Gmo Cmo Gmo Cmo Amo Gmo Gmo Cmo Gm | 36 | 51 | 5 | 112 | 79 |
| | | | | 10 | 130 | |
| | | | | 20 | 116 | |
| | | | | 40 | 165 | |
| | | | | 80 | 172 | |
| 812658 | Gmo Cmo Gmo Cmo Amo Gmo Gmo Cmo Gmo Cmo Amo Cmo Umo Gmo Cmo Cmo Amo Cm | 27 | 44 | 5 | 121 | 80 |
| | | | | 10 | 161 | |
| | | | | 20 | 164 | |
| | | | | 40 | 119 | |
| | | | | 80 | 129 | |

| | | | | | | |
|---|---|---|---|---|---|---|
| Subscripts: "m" indicates a 2'-O-methyl modification, and "o" indicates a phosphodiester internucleoside linkage. | | | | | | |

### Example 20: Effects of antisense oligonucleotides targeting the 5'-untranslated region downstream of the CFTR uORF start codon on CFTR protein expression

Antisense oligonucleotides designed to target the 5'-untranslated region downstream of the uORF start codon of human cystic fibrosis transmembrane conductance regulator (CFTR) were tested for their effects on CFTR protein expression *in vitro.* These antisense oligonucleotides, described in the table below, were designed to target the CFTR mRNA (GENBANK accession number NM_000492.3, designated herein as SEQ ID NO: 81) and were uniformly modified in order to avoid inducing cleavage of the target RNA. The start and stop sites listed in the table below indicate the positions on SEQ ID NO: 81 that the antisense oligonucleotides target. One uORF begins at position 13 of SEQ ID NO: 81. Isis No. 786455 targets a portion of the uORF that is just downstream of the uORF start codon. Isis No. 786456 targets a stem loop structure further downstream.

HT-29 cells were transfected with a concentration of antisense oligonucleotide listed in the table below or were mock transfected as a control. Twelve or twenty-two hours after transfection, CFTR expression was analyzed by western blot using Anti-CFTR purchased from EMD Millipore (catalog # 05-583, clone M3A7). The results are shown in the table below as percent protein expression relative to mock transfected cells following normalization to the HSP90 loading control. The results show that the antisense oligonucleotides increased CFTR protein expression.

**Table 34: CFTR expression**

| Isis No. | Sequence | Start Site | Stop Site | Time point (h) | Concentration (nM) | Protein (% mock) | SEQ ID NO. |
|---|---|---|---|---|---|---|---|
| 786455 | Ums Ums Cms Ums Cms Ums Gms Ams Cms Cms Ums Gms Cms Ums Gms Ums Gms Am | 19 | 36 | 12 | 12.5 | 142 | 82 |
| | | | | | 25 | 151 | |
| | | | | | 50 | 150 | |
| | | | | | 100 | 86 | |
| | | | | 22 | 12.5 | 59 | |
| | | | | | 25 | 93 | |
| | | | | | 50 | 146 | |
| | | | | | 100 | 192 | |
| 786456 | Cms Cms Ams Ams Ams Gms Ams Cms Cms Ums Ams Cms Ums Ams Cms Ums Cms Um | 60 | 77 | 22 | 12.5 | 105 | 83 |
| | | | | | 25 | 140 | |
| | | | | | 50 | 186 | |
| | | | | | 75 | 186 | |
| | | | | | 100 | 390 | |
| | | | | | 150 | 181 | |
| | | | | | 200 | 262 | |

| | | | | | | | |
|---|---|---|---|---|---|---|---|
| Subscripts: "m" indicates a 2'-O-methyl modification, and "s" indicates a phosphorothioate internucleoside linkage. | | | | | | | |

### Example 21: Identification of a translation suppression element that does not comprise a uORF

In order to test the effect of portions of the RNase H1 5'-UTR sequence on RNase H1 expression, the wild type and mutant 5'-UTR sequences were cloned into a plasmid and fused to firefly luciferase. Renila luciferase was cloned into the same plasmids for normalization. The plasmids were transfected into HeLa cells and firefly and Renila luciferase activities were detected using standard methods. The mutants and their firefly luciferase activity relative to the wild type 5'-UTR reporters, normalized to Renila luciferase activity, are shown in the table below. The nucleotide positions reported in the table below refer to the positions of the 5'-UTR of SEQ ID NO: 1 that were mutated or deleted. The results are the averages of multiple experiments and show that the mutation and/or deletion of portions of the 5'-UTR sequence of RNase H1, including portions that do not comprise a uORF, resulted in increased expression in a reporter system, indicating that the mutated portions are part of a translation suppression element.

**Table 35: Firefly luciferase activity**

| Mutation | Deletion | Firefly luciferase activity (%) |
|---|---|---|
| AUG -> UUG (uORF start codon) | n/a | 770 |
| AUG -> UUG (uORF start codon) | Nucleotides 30-60 | 980 |
| GACGGAAGT-> CAGCCTTCA, nucleotides 28-36 | n/a | 1380 |
| CGGTG->GCCAC nucleotides 38-42 | n/a | 680 |
| CGGTG->GCCAC & CGCCG->GCGGC, nucleotides 38-42 & 48-52 | n/a | 600 |

### Example 22: Effects of antisense oligonucleotides targeting the uORF of mouse LRPPRC in vivo

Isis No. 806740 (see Example 11) was tested for its effect on LRPPRC protein expression *in vivo.* Groups of three, male, seven week old BALB/c mice were systemically administered Isis No. 806740, at a dose listed in the table below, or saline by subcutaneous injection. 48 hours later, the mice received a second dose listed in the table below. Animals were sacrificed 48 hours after the second dose. Liver homogenates were prepared, and LRPPRC protein expression was analyzed by western blot, as described in Example 6. The results are shown in the tables below as the percent expression relative to saline treated animals following normalization to the loading control (PSF). The results show that LRPPRC protein expression was increased *in vivo* following treatment with an antisense oligonucleotide targeting the LRPPRC uORF and comprising bicyclic nucleic acids.

**Table 36: LRPPRC expression in vivo**

| Isis No. 806740 dose (mg/kg) | Protein (% UTC) |
|---|---|
| 12.5 | 126 |
| 25 | 171 |
| 50 | 113 |
| 100 | 120 |

### Example 23: Effects of antisense oligonucleotides targeting the 5'-untranslated region of ACP1

Effects of antisense oligonucleotides designed to target the 5'-untranslated region of ACP1, which does not comprise a uORF, are shown above (see Example 19). Additional antisense oligonucleotides designed to target the 5'-untranslation region of ACP1 were tested for their effects on ACP1 protein expression *in vitro.* The antisense oligonucleotides target the human ACP1 mRNA (GENBANK accession number NM_004300.3, designated herein as SEQ ID NO: 78). The start and stop sites listed in the table below indicate the positions on SEQ ID NO: 78 that the antisense oligonucleotides target. HeLa cells were transfected with 25 nM of an antisense oligonucleotide shown in the table below or were not transfected as a control. Ten hours after transfection, ACP1 protein expression was analyzed by western blot using an Abcam antibody to ACP1 (catalog # ab166896). The results are shown in the tables below as percent protein expression relative to untreated control cells following normalization to the Annexin A2 loading control.

**Table 37: ACP1 expression**

| Isis No. | Sequence | Start Site | Stop Site | Protein (% mock) | SEQ ID NO. |
|---|---|---|---|---|---|
| 812658 | Gmo Cmo Gmo Cmo Amo Gmo Gmo Cmo Gmo Cmo Amo Cmo Umo Gmo Cmo Cmo Amo Cm | 27 | 44 | 127 | 80 |
| 812650 | A_mo G_mo G_mo C_mo G_mo C_mo A_mo C_mo U_mo G_mo C_mo C_mo A_mo C_m | 27 | 40 | 142 | 84 |
| (XL500) | Gmo Cmo Amo Gmo Gmo Cmo Gmo Cmo Amo Cmo Umo Gmo Cmo Cm | 29 | 42 | 147 | 85 |
| 812651 | Gmo Cmo Gmo Cmo Amo Gmo Gmo Cmo Gmo Cmo Amo Cmo Umo Gm | 31 | 44 | 107 | 86 |
| (XL502) | Cmo Gmo Gmo Cmo Gmo Cmo Amo Gmo Gmo Cmo Gmo Cmo Amo Cm | 33 | 46 | 100 | 87 |
| (XL503) | Cmo Gmo Cmo Gmo Gmo Cmo Gmo Cmo Amo Gmo Gmo Cmo Gmo Cm | 35 | 48 | 58 | 88 |
| (XL504) | Gₘₒ Aₘₒ Cₘₒ Gₘₒ Cₘₒ Gₘₒ Gₘₒ Cₘₒ Gₘₒ Cₘₒ Aₘₒ Gₘₒ Gₘₒ Cₘ | 37 | 50 | 107 | 89 |

| | | | | | |
|---|---|---|---|---|---|
| Subscripts: "m" indicates a 2'-O-methyl modification, and "o" indicates a phosphodiester internucleoside linkage. | | | | | |

### Example 24: Effects of antisense oligonucleotides targeting ACP1 comprising various modifications

Antisense oligonucleotides designed to target the same or similar site of the 5'-untranslated region of ACP1 as Isis No. 812658 (see Examples 19 and 23) were designed with various modifications and tested for their effects on ACP1 protein expression *in vitro.* The start and stop sites listed in the table below indicate the positions on SEQ ID NO: 78 that the antisense oligonucleotides target. HeLa cells were transfected with a concentration of an antisense oligonucleotide shown in the tables below or were not transfected as a control. Ten hours after transfection, ACP1 protein expression was analyzed by western blot using an Abcam antibody to ACP1 (catalog # ab166896). The results are shown in the tables below as percent protein expression relative to untreated control cells following normalization to the Annexin A2 or TMED9 loading control.

**Table 38: ACP1 expression**

| Isis No. | Sequence | Start Site | Stop Site | Concentration (nM) | Protein (% mock) | SEQ ID NO. |
|---|---|---|---|---|---|---|
| 812675 | Gms Cms Ams Gms Gms Cms Gms Cms Ams Cms Ums Gms Cms CmsAms Cm | 27 | 42 | 5 | 161 | 90 |
| | | | | 10 | 147 | |
| | | | | 20 | 227 | |
| | | | | 40 | 193 | |
| | | | | 80 | 98 | |
| 812653 | Gmo Cmo Amo Gmo Gmo Cmo Gmo Cmo Amo Cmo Umo Gmo Cmo Cmo Amo Cm | 27 | 42 | 5 | 87 | 90 |
| | | | | 10 | 167 | |
| | | | | 20 | 182 | |
| | | | | 40 | 139 | |
| | | | | 80 | 124 | |

**Table 39: ACP1 expression**

| Isis No. | Sequence | Start Site | Stop Site | Concentration (nM) | Protein (% mock) | SEQ ID NO. |
|---|---|---|---|---|---|---|
| 813860 | Gms Cms Gms Cms Ams Gms Gms Cms Gms Cms Ams Cms Ums Gms Cms mCks Aks mCk | 27 | 44 | 25 | 87 | 80 |
| 826061 | Gms Cms Gms Cms Ams Gms Gms Cms Gms Cms Ams Cms Ums Gms Cms mCko Aks mCk | 27 | 44 | 25 | 109 | 80 |
| 826062 | Gms Cms Gms Cms Ams Gms Gms Cms Gms Cms Ams Cmo Ums Gmo Cms mCko Aks mCk | 27 | 44 | 25 | 111 | 80 |
| 826063 | Gms Cmo Gms Cmo Ams Gms Gms Cms Gms Cms Ams Cms Ums Gms Cms mCks Aks mCk | 27 | 44 | 25 | 111 | 80 |
| 826064 | Gms Cmo Gms Cmo Ams Gmo Gms Cms Gms Cms Ams Cms Ums Gms Cms mCks Aks mCk | 27 | 44 | 25 | 151 | 80 |
| 826065 | Gms Cms Gms Cms Ams Gms Gmo Cms Gmo Cms Ams Cms Ums Gms Cms mCks Aks mCk | 27 | 44 | 25 | 158 | 80 |
| 826066 | Gms Cms Gms Cms Ams Gms Gms Cmo Gms Cmo Ams Cms Ums Gms Cms mCks Aks mCk | 27 | 44 | 25 | 140 | 80 |
| 826069 | Gms Cms Gms Cms Ams Gms Gms Cms Gms Cms Ams Cms Ums Gks Cds mCks Ads mCk | 27 | 44 | 25 | 196 | 80 |

| | | | | | | |
|---|---|---|---|---|---|---|
| Subscripts: "m" indicates a 2'-O-methyl modification, "o" indicates a phosphodiester internucleoside linkage, "s" indicates a phosphorothioate internucleoside linkage, "k" indicates a cEt bicyclic nucleoside, "d" indicates a 2'-deoxynucleoside. Superscript "m" preceding a "C" indicates a 5-methylcytosine. | | | | | | |

**Table 40: ACP1 expression**

| Isis No. | Start Site | Stop Site | Concentration (nM) | Protein (% mock) | SEQ ID NO. |
|---|---|---|---|---|---|
| 813860 | 27 | 44 | 5 | 130 | 80 |
| | | | 10 | 104 | |
| | | | 20 | 47 | |
| | | | 40 | 95 | |
| | | | 80 | 89 | |
| 826065 | 27 | 44 | 5 | 143 | 80 |
| | | | 10 | 137 | |
| | | | 20 | 182 | |
| | | | 40 | 153 | |
| | | | 80 | 127 | |
| 826069 | 27 | 44 | 5 | 127 | 80 |
| | | | 20 | 139 | |
| | | | 40 | 184 | |
| | | | 80 | 133 | |

### Example 25: Effects of antisense oligonucleotides targeting the 5'-UTR of mouse ACP1

Antisense oligonucleotides designed to target the 5'-untranslated region of mouse ACP1 were tested for their effects on ACP1 protein expression *in vitro.* The antisense oligonucleotides target the mouse ACP1 mRNA (GENBANK accession number NM_021330.4, designated herein as SEQ ID NO: 91) and were designed to target at least one stem loop structure in the 5'-UTR. The start and stop sites listed in the table below indicate the positions on SEQ ID NO: 91 that the antisense oligonucleotides target. MHT cells were transfected with a concentration of antisense oligonucleotide shown in the table below or were not transfected as a control. Ten hours after transfection, ACP1 protein expression was analyzed by western blot. The results are shown in the table below as percent protein expression relative to untreated control cells following normalization to the TCP-1β loading control. The results show that the antisense oligonucleotides targeting the 5'-untranslated region of mouse ACP1 induced translation of the target.

**Table 41: ACP1 expression**

| Isis No. | Sequence | Start Site | Stop Site | Concentration (nM) | Protein (% mock) | SEQ ID NO. |
|---|---|---|---|---|---|---|
| (XL546) | Gmo Cmo Amo Umo Gmo Cmo Gmo Cmo Amo Cmo Umo Gmo Cmo Cmo Am | 20 | 34 | 5 | 88 | 92 |
| | | | | 10 | 133 | |
| | | | | 20 | 153 | |
| | | | | 40 | 125 | |
| | | | | 80 | 126 | |
| (XL547) | Gms Cms Ams Ums Gms Cms Gms Cms Ams Cms Ums Gms Cms Cms Am | 20 | 34 | 5 | 161 | 92 |
| | | | | 10 | 198 | |
| | | | | 20 | 219 | |
| | | | | 40 | 227 | |
| | | | | 80 | 194 | |

| | | | | | | |
|---|---|---|---|---|---|---|
| See above Tables for subscripts legend. | | | | | | |

### Example 26: Effect of an antisense oligonucleotide targeting the 5'-UTR mouse ACP1 in vivo

Isis No. 827815 (see the table below) was tested for its effect on mouse ACP1 protein expression *in vivo.* Groups of three, male, seven week old BALB/c mice were systemically administered Isis No. 827815, at a dose listed in the table below, or saline by subcutaneous injection. Animals were sacrificed 72 hours later. Liver homogenates were prepared, and ACP1 protein expression was analyzed by western blot. The results are shown in the table below as the percent expression relative to saline treated animals following normalization to the TMED9 loading control. The results show that ACP1 protein expression was increased *in vivo* following treatment with an antisense oligonucleotide targeting the ACP1 5'-UTR.

**Table 42: ACP1 expression in vivo**

| Isis No. | Sequence | Start site | Stop site | Dose (mg/kg) | Protein (% UTC) | SEQ ID NO. |
|---|---|---|---|---|---|---|
| | Gms Cms Ams Ums Gms Cms Gms Cms Ams Cms Ums Gms Cms Cms Ams Gm | | | 12.5 | 145 | |
| 827815 | | 19 | 34 | 25 | 114 | 93 |
| | | | | 50 | 116 | |
| | | | | 100 | 94 | |

| | | | | | | |
|---|---|---|---|---|---|---|
| See above Tables for subscripts legend. | | | | | | |

### Example 27: Effect of antisense oligonucleotide targeting the 5'-UTR of mouse ARF1

An antisense oligonucleotide designed to target the 5'-untranslated region of mouse ADP-ribosylation factor 1 (ARF1) was tested for its effects on ARF1 protein expression *in vitro.* The antisense oligonucleotide targets the mouse ARF1 mRNA (GENBANK accession number NM_007476.3, designated herein as SEQ ID NO: 94), which does not comprise a uORF, and was designed to target at least one stem loop structure in the 5'-UTR. The start and stop sites listed in the table below indicate the positions on SEQ ID NO: 94 that the antisense oligonucleotide targets. MHT cells were transfected with a concentration of antisense oligonucleotide shown in the table below or were not transfected as a control. Ten hours after transfection, ARF1 protein expression was analyzed by western blot. The results are shown in the table below as percent protein expression relative to untreated control cells following normalization to the TCP-1β loading control. The results show that the antisense oligonucleotide targeting the 5'-untranslated region of mouse ARF1 induced translation of the target.

**Table 43: ARF1 expression**

| Isis No. | Sequence | Start Site | Stop Site | Concentration (nM) | Protein (% mock) | SEQ ID NO. |
|---|---|---|---|---|---|---|
| | Cmo Gmo Cmo Tmo Cmo Cmo Cmo Amo Cmo Amo Amo Gmo Amo Tmo Gmo Gmo Cm | | | 5 | 112 | |
| | | | | 10 | 179 | |
| 814929 | | 44 | 60 | 20 | 215 | 95 |
| | | | | 40 | 181 | |
| | | | | 80 | 122 | |

| | | | | | | |
|---|---|---|---|---|---|---|
| See above Tables for subscripts legend. | | | | | | |

### Example 28: Effect of antisense oligonucleotide targeting the 5'-UTR of mouse USP16

An antisense oligonucleotide designed to target the 5'-untranslated region of mouse ubiquitin processing protease (USP16) was tested for its effects on USP16 protein expression *in vitro.* The antisense oligonucleotide targets the mouse USP16 mRNA (GENBANK accession number NM_024258.2, designated herein as SEQ ID NO: 96), and was designed to target at least one stem loop structure in the 5'-UTR. The start and stop sites listed in the table below indicate the positions on SEQ ID NO: 96 that the antisense oligonucleotide targets. MHT cells were transfected with a concentration of antisense oligonucleotide shown in the table below or were not transfected as a control. Ten hours after transfection, USP16 protein expression was analyzed by western blot. The results are shown in the table below as percent protein expression relative to untreated control cells following normalization to the β-actin (ACTB) loading control. The results show that the antisense oligonucleotide targeting the 5'-untranslated region of mouse USP16 induced translation of the target.

**Table 44: USP16 expression**

| Isis No. | Sequence | Start Site | Stop Site | Concentration (nM) | Protein (% mock) | SEQ ID NO. |
|---|---|---|---|---|---|---|
| | | | | 5 | 124 | |
| | Gmo Amo Gmo Amo Gmo Cmo Gmo Amo Cmo Gmo Cmo Gmo Gmo Tmo Gmo Gmo Am | | | 10 | 133 | |
| 814928 | | 21 | 37 | 20 | 133 | 97 |
| | | | | 40 | 116 | |
| | | | | 80 | 116 | |

| | | | | | | |
|---|---|---|---|---|---|---|
| See above Tables for subscripts legend. | | | | | | |

### Example 29: Effects of antisense oligonucleotides targeting the 5'-UTR of human LDLr

Antisense oligonucleotides designed to target the 5'-untranslated region of human low density lipoprotein receptor (LDLr) were tested for their effects on LDLr protein expression and LDL uptake *in vitro.* The antisense oligonucleotides target the human LDLr mRNA (GENBANK accession number NM_000527.4, designated herein as SEQ ID NO: 98) and were designed to target at least one stem loop structure in the 5'-UTR. The start and stop sites listed in the table below indicate the positions on SEQ ID NO: 98 that the antisense oligonucleotides target. HEK293 or HeLa cells were transfected with a concentration of antisense oligonucleotide shown in the table below or were not transfected as a control. Ten hours after transfection, LDLr protein expression was analyzed by ELISA (R & D Systems, cat. # DLDLR0). The results are shown in the table below as percent protein expression relative to untreated control cells. The results show that the antisense oligonucleotides targeting the 5'-untranslated region of human LDLr induced translation of the target.

In order to test the effects of the antisense oligonucleotides in the tables below on LDL uptake, HEK 293 or HeLa cells were transfected with an antisense oligonucleotide at a concentration shown in the table below or were not transfected as a control. Approximately fourteen hours after transfection, the medium was changed to lipid-free medium, incubated at 37 °C for one hour, then 5 to 10 µg/mL of Bodipy labeled LDL was added at 4 °C for 30 to 60 minutes to all cells, including untransfected control cells. The cells were then washed with cold PBS, harvested, and lysed with RIPA buffer. The Bodipy fluorescence was measured, and the results in the table below show the percent Bodipy fluorescence relative to untransfected control cells.

**Table 45: LDLr expression**

| Isis No. | Sequence | Start Site | Stop Site | Cell type | Concentration (nM) | Protein (% UTC) | SEQ ID NO. |
|---|---|---|---|---|---|---|---|
| 814923 | Umo Gmo Cmo Amo Gmo Umo Gmo Gmo Gmo Gmo Umo Gmo Amo Umo Umo Um | 28 | 43 | HEK293 | 5 | 131 | 99 |
| | | | | | 10 | 146 | |
| | | | | | 20 | 169 | |
| | | | | | 40 | 188 | |
| | | | | | 80 | 238 | |
| 814923 | Umo Gmo Cmo Amo Gmo Umo Gmo Gmo Gmo Gmo Umo Gmo Amo Umo Umo Um | 28 | 43 | HeLa | 5 | 124 | 99 |
| | | | | | 10 | 154 | |
| | | | | | 20 | 113 | |
| | | | | | 40 | 128 | |
| | | | | | 80 | 106 | |
| 842196? (XL512) | Ums Gms Cms Ams Gms Ums Gms Gms Gms Gms Ums Gms Ams Ums Ums Um | 28 | 43 | HeLa | 5 | 112 | 99 |
| | | | | | 10 | 111 | |
| | | | | | 20 | 128 | |
| | | | | | 40 | 121 | |
| | | | | | 80 | 137 | |

| | | | | | | | |
|---|---|---|---|---|---|---|---|
| See above Tables for subscripts legend. | | | | | | | |

**Table 46: LDL uptake**

| Isis No. | Cell type | Bodipy-LDL incubation time (min) | Concentration (nM) | LDL uptake (% UTC) | SEQ ID NO. |
|---|---|---|---|---|---|
| 814923 | HEK293 | 60 | 40 | 152 | 99 |
| 842196 | HEK293 | 30 | 40 | 115 | 99 |
| | | 60 | 40 | 130 | |
| 842196 | HeLa | 30 | 12.5 | 108 | 99 |
| | | | 25 | 108 | |
| | | | 50 | 127 | |
| | | | 100 | 132 | |

### Example 30: Effects of antisense oligonucleotides targeting the 5'-untranslated region downstream of the CFTR uORF start codon on CFTR protein expression

Antisense oligonucleotides designed to target a uORF start codon of human CFTR were tested for their effects on CFTR protein expression *in vitro.* These antisense oligonucleotides, described in the table below, were designed to target the CFTR mRNA (GENBANK accession number NM_000492.3, designated herein as SEQ ID NO: 81) and were uniformly modified in order to avoid inducing cleavage of the target RNA. The start and stop sites listed in the table below indicate the positions on SEQ ID NO: 81 that the antisense oligonucleotides target.

HT-29 cells were transfected with a concentration of antisense oligonucleotide listed in the table below or were mock transfected as a control. Twenty-four hours after transfection, CFTR expression was analyzed by western blot, as described in Example 20. The results are shown in the table below as percent protein expression relative to mock transfected cells following normalization to a loading control. The results show that the antisense oligonucleotides increased CFTR protein expression.

**Table 47: CFTR expression**

| Isis No. | Sequence | Start Site | Stop Site | Concentration (nM) | Protein (% mock) | SEQ ID NO. |
|---|---|---|---|---|---|---|
| 812022 | Umo Gmo Cmo Umo Gmo Umo Gmo Amo Umo Gmo Umo Cmo Amo Umo Umo Umo Gmo Cm | 9 | 26 | 50 | 132 | 100 |
| | | | | 75 | 117 | |
| | | | | 100 | 125 | |
| | | | | 150 | 112 | |
| 812023 | Uₘₛ Gₘₛ Cₘₛ Uₘₛ Gₘₛ Uₘₛ Gₘₛ Aₘₛ Uₘₛ Gₘₛ Uₘₛ Cₘₛ Aₘₛ Uₘₛ Uₘₛ Uₘₛ Gₘₛ Cₘ | 9 | 26 | 50 | 130 | 100 |
| | | | | 75 | 126 | |
| | | | | 100 | 124 | |
| | | | | 150 | 128 | |

| | | | | | | |
|---|---|---|---|---|---|---|
| See above Tables for subscripts legend. | | | | | | |

### Example 31: Effects of antisense oligonucleotides targeting the 5'-UTR of human LDLr

Antisense oligonucleotides designed to target the 5'-untranslated region of human low density lipoprotein receptor (LDLr) were tested for their effects on LDLr protein expression *in vitro.* The antisense oligonucleotides target the human LDLr mRNA (SEQ ID NO: 98) and were designed to target at least one stem loop structure in the 5'-UTR. The start and stop sites listed in the table below indicate the positions on SEQ ID NO: 98 that the antisense oligonucleotides target. HEK293 cells were transfected with a concentration of antisense oligonucleotide shown in the table below or were not transfected as a control. Sixteen hours after transfection, LDLr protein expression was analyzed by ELISA (R & D Systems, cat. # DLDLR0). The results are shown in the table below as percent protein expression relative to untreated control cells. The results show that the antisense oligonucleotides targeting the 5'-untranslated region of human LDLr induced translation of the target.

**Table 48: LDLr expression**

| Isis No. | Sequence | Start Site | Stop Site | Concentration (nM) | Protein (% UTC) | SEQ ID NO. |
|---|---|---|---|---|---|---|
| 842196 | Ums Gms Cms Ams Gms Ums Gms Gms Gms Gms Ums Gms Ams Ums Ums Um | 28 | 43 | 20 | 125 | 99 |
| | | | | 40 | 195 | |
| | | | | 80 | 222 | |
| 842197 | Ums Gms Cms Ams Gms Ums Gms Gms Gms Gms Ums Gms Ams Tks Tks Tk | 28 | 43 | 20 | 176 | 101 |
| | | | | 40 | 266 | |
| | | | | 80 | 214 | |
| 842198 | Ums Gms Cms Ams Gmo Ums Gmo Gms Gmo Gms Ums Gms Ams Tks Tks Tk | 28 | 43 | 20 | 160 | 101 |
| | | | | 40 | 157 | |
| | | | | 80 | 194 | |
| 842200 | Ums Gms Cms Ams Gmo Ums Gmo Gms Gmo Gms Umo Gms Ams Ums Ums Um | 28 | 43 | 20 | 131 | 99 |
| | | | | 40 | 175 | |
| | | | | 80 | 170 | |
| 842202 | Ums Gms Cms Ams Gmo Ums Gmo Gms Gms Gms Ums Gms Ams Ums Ums Um | 28 | 43 | 20 | 125 | 99 |
| | | | | 40 | 132 | |
| | | | | 80 | 145 | |
| 842205 | Ums Gms Cms Ams Gms Ums Gms Gms Gms Gms Ums Gms Ams Ums Ums Ums Ums Cm | 26 | 43 | 20 | 123 | 102 |
| | | | | 40 | 136 | |
| | | | | 80 | 107 | |
| 842206 | Ums Gms Cms Ams Gms Ums Gms Gms Gms Gms Ums Gms Ams Ums Ums Tks Tks mCk | 26 | 43 | 20 | 155 | 103 |
| | | | | 40 | 173 | |
| | | | | 80 | 134 | |
| 842207 | Ums Gms Cms Ams Gmo Ums Gmo Gms Gmo Gms Ums Gms Ams Ums Ums Tks Tk mCk | 26 | 43 | 20 | 125 | 103 |
| | | | | 40 | 147 | |
| | | | | 80 | 129 | |
| 842211 | Ums Gms Cms Ams Gmo Ums Gmo Gms Gms Gms Ums Gms Ams Ums Ums Ums Ums Cm | 26 | 43 | 20 | 136 | 102 |
| | | | | 40 | 143 | |
| | | | | 80 | 142 | |
| 842212 | Ums Gms Cmo Ams Gmo Ums Gmo Gms Gmo Gms Umo Gms Amo Ums Umo Ums Ums Cm | 26 | 43 | 20 | 103 | 102 |
| | | | | 40 | 101 | |
| | | | | 80 | 120 | |
| 842213 | Uₘₛ Gₘₛ Cₘₛ Aₘₛ Gₘₛ Uₘₛ Gₘₛ Gₘₛ Gₘₛ Gₘₛ Uₘₛ Gₘₛ Aₘₛ Tₖₛ T_{ds} Tₖₛ T_{ds} ^{m}Cₖ | 26 | 43 | 20 | 128 | 104 |
| | | | | 40 | 135 | |
| | | | | 80 | 139 | |
| 842214 | Ums Gms Cms Ams Gms Ums Gms Gms Gms Gms Ums Gks Ads Tks Tds Tk | 28 | 43 | 20 | 134 | 101 |
| | | | | 40 | 152 | |
| | | | | 80 | 139 | |

| | | | | | | |
|---|---|---|---|---|---|---|
| See above Tables for subscripts legend. | | | | | | |

### Example 32: Time course of LDLr protein expression following treatment with an antisense oligonucleotide targeting the 5'-UTR

Hep3B cells were transfected with 30 nM Isis No. 842196 (see Table 45) and Lipofectamine 2000 (Life Technologies). At various time points following transfection, the cells were harvested and LDLr protein expression was analyzed by ELISA as described in Examples 30 and 31. The results are shown in Table 49 as the percent LDLr protein expression relative to cells that did not receive antisense oligonucleotide treatment (harvested at the 0 hour time point). The results show that LDLr expression was increased over 4-fold relative to baseline expression by Isis No. 842196, at the 48 hour time point.

**Table 49: LDLr expression**

| LDLr (% 0 h) following transfection with 30 nM Isis No. 842196 | | |
|---|---|---|
| 24 h | 36 h | 48 h |
| 212 | 230 | 457 |

## Claims

1. A method of increasing the amount of a target protein in an *in vitro* cell, comprising contacting the cell with an antisense compound comprising a modified oligonucleotide, wherein the target protein is encoded by a target transcript comprising at least one translation suppression element, wherein the translation suppression element comprises a uORF, wherein the uORF comprises an AUG codon, and thereby increasing expression of the target protein in the cell, wherein the modified oligonucleotide:
i) is complementary to the target transcript at the uORF start site region;
ii) has a nucleobase sequence that is 100% complementary to the target transcript;
iii) consists of 15 to 22 linked nucleosides; and
iv) comprises at least 15 modified nucleosides, each modified nucleoside independently comprising a modified sugar moiety, wherein the antisense compound does not recruit RNase H.

2. An antisense compound comprising a modified oligonucleotide for use in a method of treating a disease or condition associated with a deficiency of a target protein, wherein in said method the modified oligonucleotide increases the amount of the target protein in a human cell, wherein the target protein is encoded by a target transcript comprising at least one translation suppression element, wherein the translation suppression element comprises a uORF, wherein the uORF comprises an AUG codon, and thereby increasing expression of the target protein in the cell, wherein the modified oligonucleotide:
i) is complementary to the target transcript at the uORF start site region;
ii) has a nucleobase sequence 100% complementary to the target transcript;
iii) consists of 15 to 22 linked nucleosides; and
iv) comprises at least 15 modified nucleosides, each modified nucleoside independently comprising a modified sugar moiety, wherein the antisense compound does not recruit RNase H.

3. The method of claim 1, or the antisense compound for use according to claim 2, wherein the antisense compound is single-stranded.

4. The method of claim 1 or claim 3, or the antisense compound for use according to claim 2 or claim 3, wherein the translation suppression element comprises one and only one uORF, or consists of a uORF.

5. The method of any of claims 1, 3, and 4, or the antisense compound for use according to any of claims 2-4, wherein the translation suppression element arises from a mutation.

6. The method of any of claims 1 and 3-5, or the antisense compound for use according to any of claims 2-5, wherein the modified oligonucleotide consists of 16, 17, or 18 linked nucleosides.

7. The method of any of claims 1 and 3-6, or the antisense compound for use according to any of claims 2-6, wherein each nucleoside of the modified oligonucleotide is a modified nucleoside, wherein each modified nucleoside independently comprises a modified sugar moiety.

8. The method of any of claims 1 and 3-7, or the antisense compound for use according to any of claims 2-7, wherein at least one nucleoside of the modified oligonucleotide comprises a 2'-substituted sugar moiety, preferably wherein the at least one 2'-substituted sugar moiety is selected from among: 2'-OMe, 2'-F, and 2'-MOE.

9. The method of any of claims 1 and 3-8, or the antisense compound for use according to any of claims 2-8, wherein at least one nucleoside of the modified oligonucleotide comprises a bicyclic sugar moiety, optionally wherein the at least one bicyclic sugar moiety is LNA or cEt.

10. The method of any of claims 1 and 3-9, or the antisense compound for use according to any of claims 2-9, wherein the modified oligonucleotide comprises at least one modified internucleoside linkage, optionally wherein the at least one modified internucleoside linkage is a phosphorothioate internucleoside linkage.

11. The method of any of claims 1 and 3-10, or the antisense compound for use according to any of claims 2-10, wherein the antisense compound comprises at least one conjugate group, optionally wherein the conjugate group comprises GalNAc.

12. The method of any of claims 1 and 3-11, or the antisense compound for use according to any of claims 2-11, wherein the antisense compound consists of the modified oligonucleotide.

## Patentansprüche

1. Verfahren zum Erhöhen der Menge eines Zielproteins in einer in-vitro-Zelle, umfassend Inkontaktbringen der Zelle mit einer Antisense-Verbindung, die ein modifiziertes Oligonukleotid umfasst, wobei das Zielprotein durch ein Zieltranskript codiert wird, das mindestens ein Translationsunterdrückungselement umfasst, wobei das Translationsunterdrückungselement ein uORF umfasst, wobei das uORF ein AUG-Codon umfasst, und dadurch Erhöhen der Expression des Zielproteins in der Zelle, wobei das modifizierte Oligonukleotid:
i) komplementär zu dem Zieltranskript in der uORF-Startpunktregion ist;
ii) eine Nukleobasensequenz aufweist, die zu 100 % komplementär zu dem Zieltranskript ist;
iii) aus 15 bis 22 verknüpften Nukleosiden besteht; und
iv) mindestens 15 modifizierte Nukleoside umfasst, wobei jedes modifizierte Nukleosid unabhängig eine modifizierte Zuckereinheit umfasst, wobei die Antisense-Verbindung RNase H nicht rekrutiert

2. Antisense-Verbindung, umfassend ein modifiziertes Oligonukleotid, zur Verwendung in einem Verfahren zur Behandlung einer Erkrankung oder eines Zustands, die/der mit einem Mangel eines Zielproteins assoziiert ist, wobei in dem Verfahren das modifizierte Oligonukleotid die Menge des Zielproteins in einer menschlichen Zelle erhöht, wobei das Zielprotein durch ein Zieltranskript codiert wird, das mindestens ein Translationsunterdrückungselement umfasst, wobei das Translationsunterdrückungselement ein uORF umfasst, wobei das uORF ein AUG-Codon umfasst, und dadurch die Expression des Zielproteins in der Zelle erhöht, wobei das modifizierte Oligonukleotid:
i) komplementär zu dem Zieltranskript in der uORF-Startpunktregion ist;
ii) eine Nukleobasensequenz aufweist, die 100 % komplementär zu dem Zieltranskript ist;
iii) aus 15 bis 22 verknüpften Nukleosiden besteht; und
iv) mindestens 15 modifizierte Nukleoside umfasst, wobei jedes modifizierte Nukleosid unabhängig eine modifizierte Zuckereinheit umfasst, wobei die Antisense-Verbindung RNase H nicht rekrutiert

3. Verfahren nach Anspruch 1 oder Antisense-Verbindung zur Verwendung nach Anspruch 2, wobei die Antisense-Verbindung einzelsträngig ist.

4. Verfahren nach Anspruch 1 oder Anspruch 3 oder Antisense-Verbindung zur Verwendung nach Anspruch 2 oder Anspruch 3, wobei das Translationsunterdrückungselement ein und nur ein uORF umfasst oder aus einem uORF besteht.

5. Verfahren nach einem der Ansprüche 1, 3 und 4 oder Antisense-Verbindung zur Verwendung nach einem der Ansprüche 2-4, wobei das Translationsunterdrückungselement aus einer Mutation stammt.

6. Verfahren nach einem der Ansprüche 1 und 3-5 oder Antisense-Verbindung zur Verwendung nach einem der Ansprüche 2-5, wobei das modifizierte Oligonukleotid aus 16, 17 oder 18 verknüpften Nukleosiden besteht.

7. Verfahren nach einem der Ansprüche 1 und 3-6 oder Antisense-Verbindung zur Verwendung nach einem der Ansprüche 2-6, wobei jedes Nukleosid des modifizierten Oligonukleotids ein modifiziertes Nukleosid ist, wobei jedes modifizierte Nukleosid unabhängig eine modifizierte Zuckereinheit umfasst.

8. Verfahren nach einem der Ansprüche 1 und 3-7 oder Antisense-Verbindung zur Verwendung nach einem der Ansprüche 2-7, wobei mindestens ein Nukleosid des modifizierten Oligonukleotids eine 2'-substituierte Zuckereinheit umfasst, wobei die mindestens eine 2'-substituierte Zuckereinheit vorzugsweise ausgewählt ist aus: 2'-OMe, 2'-F und 2'-MOE.

9. Verfahren nach einem der Ansprüche 1 und 3-8 oder Antisense-Verbindung zur Verwendung nach einem der Ansprüche 2-8, wobei mindestens ein Nukleosid des modifizierten Oligonukleotids eine bicyclische Zuckereinheit umfasst, wobei die mindestens eine bicyclische Zuckereinheit gegebenenfalls LNA oder cEt ist.

10. Verfahren nach einem der Ansprüche 1 und 3-9 oder Antisense-Verbindung zur Verwendung nach einem der Ansprüche 2-9, wobei das modifizierte Oligonukleotid mindestens eine modifizierte Internukleosidverknüpfung umfasst, wobei die mindestens eine modifizierte Internukleosidverknüpfung gegebenenfalls eine Phosphorthioat-Internukleosidverknüpfung ist.

11. Verfahren nach einem der Ansprüche 1 und 3-10 oder Antisense-Verbindung zur Verwendung nach einem der Ansprüche 2-10, wobei die Antisense-Verbindung mindestens eine Konjugatgruppe umfasst, wobei gegebenenfalls die Konjugatgruppe GalNAc umfasst.

12. Verfahren nach einem der Ansprüche 1 und 3-11 oder Antisense-Verbindung zur Verwendung nach einem der Ansprüche 2-11, wobei die Antisense-Verbindung aus dem modifizierten Oligonukleotid besteht.

## Revendications

1. Procédé d'augmentation de la quantité d'une protéine cible dans une cellule *in vitro*, comprenant la mise en contact de la cellule avec un composé antisens comprenant un oligonucléotide modifié, dans lequel la protéine cible est codée par un produit de transcription cible comprenant au moins un élément de suppression de traduction, dans lequel l'élément de suppression de traduction comprend un uORF, dans lequel l'uORF comprend un codon AUG, et augmentant ainsi l'expression de la protéine cible dans la cellule, dans lequel l'oligonucléotide modifié :
i) est complémentaire du produit de transcription cible au niveau de la région de site de départ de l'uORF ;
ii) a une séquence de nucléobases qui est complémentaire à 100 % du produit de transcription cible ;
iii) est constitué de 15 à 22 nucléosides liés ; et
iv) comprend au moins 15 nucléosides modifiés, chaque nucléoside modifié comprenant indépendamment une fraction de sucre modifié, le composé antisens ne recrutant pas de RNase H.

2. Composé antisens comprenant un oligonucléotide modifié destiné à être utilisé dans un procédé de traitement d'une maladie ou d'une affection associée à une déficience en une protéine cible, dans lequel dans ledit procédé, l'oligonucléotide modifié augmente la quantité de la protéine cible dans une cellule humaine, dans lequel la protéine cible est codée par un produit de transcription cible comprenant au moins un élément de suppression de traduction, dans lequel l'élément de suppression de traduction comprend un uORF, dans lequel l'uORF comprend un codon AUG, et augmentant ainsi l'expression de la protéine cible dans la cellule, dans lequel l'oligonucléotide modifié :
i) est complémentaire du produit de transcription cible au niveau de la région de site de départ de l'uORF ;
ii) a une séquence de nucléobases complémentaire à 100 % du produit de transcription cible ;
iii) est constitué de 15 à 22 nucléosides liés ; et
iv) comprend au moins 15 nucléosides modifiés, chaque nucléoside modifié comprenant indépendamment une fraction de sucre modifié, le composé antisens ne recrutant pas de RNase H.

3. Procédé selon la revendication 1, ou composé antisens pour utilisation selon la revendication 2, dans lequel le composé antisens est simple brin.

4. Procédé selon la revendication 1 ou la revendication 3, ou composé antisens destiné à être utilisé selon la revendication 2 ou la revendication 3, dans lequel l'élément de suppression de traduction comprend un et un seul uORF, ou est constitué d'un uORF.

5. Procédé selon l'une quelconque des revendications 1,3 et 4, ou composé antisens destiné à être utilisé selon l'une quelconque des revendications 2 à 4, dans lequel l'élément de suppression de traduction provient d'une mutation.

6. Procédé selon l'une quelconque des revendications 1 et 3 à 5, ou composé antisens pour utilisation selon l'une quelconque des revendications 2 à 5, dans lequel l'oligonucléotide modifié est constitué de 16, 17 ou 18 nucléosides liés.

7. Procédé selon l'une quelconque des revendications 1 et 3 à 6, ou composé antisens pour utilisation selon l'une quelconque des revendications 2 à 6, dans lequel chaque nucléoside de l'oligonucléotide modifié est un nucléoside modifié, dans lequel chaque nucléoside modifié comprend indépendamment un fragment de sucre modifié.

8. Procédé selon l'une quelconque des revendications 1 et 3 à 7, ou composé antisens pour une utilisation selon l'une quelconque des revendications 2 à 7, dans lequel au moins un nucléoside de l'oligonucléotide modifié comprend un fragment de sucre substitué en 2', de préférence dans lequel l'au moins un fragment de sucre substitué en 2' est choisi parmi : 2'-OMe, 2'-F et 2'-MOE.

9. Procédé selon l'une quelconque des revendications 1 et 3 à 8, ou composé antisens pour utilisation selon l'une quelconque des revendications 2 à 8, dans lequel au moins un nucléoside de l'oligonucléotide modifié comprend un fragment de sucre bicyclique, éventuellement dans lequel l'au moins un fragment de sucre bicyclique est LNA ou cEt.

10. Procédé selon l'une quelconque des revendications 1 et 3 à 9, ou composé antisens pour utilisation selon l'une quelconque des revendications 2 à 9, dans lequel l'oligonucléotide modifié comprend au moins une liaison internucléosidique modifiée, éventuellement dans lequel l'au moins une liaison internucléosidique modifiée est une liaison internucléosidique phosphorothioate.

11. Procédé selon l'une quelconque des revendications 1 et 3 à 10, ou composé antisens pour utilisation selon l'une quelconque des revendications 2 à 10, dans lequel le composé antisens comprend au moins un groupe conjugué, facultativement dans lequel le groupe conjugué comprend GalNAc.

12. Procédé selon l'une quelconque des revendications 1 et 3-11, ou composé antisens pour utilisation selon l'une quelconque des revendications 2-11, dans lequel le composé antisens est constitué de l'oligonucléotide modifié.
